(19) Europäisches Patentamt
European Patent Office
Office européen des brevets

(11) EP 2 447 358 B1

(12) EUROPEAN PATENT SPECIFICATION

(45) Date of publication and mention of the grant of the patent:
**23.09.2015 Bulletin 2015/39**

(51) Int Cl.:
*C12N 9/04* (2006.01) *C12Q 1/00* (2006.01)

(21) Application number: **11186943.4**

(22) Date of filing: **27.10.2011**

(54) **Mutant glucose dehydrogenase**

Mutante Glucosedehydrogenase

Déshydrogénase de glucose mutant

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2010 JP 2010240426**

(43) Date of publication of application:
**02.05.2012 Bulletin 2012/18**

(73) Proprietors:
- **ARKRAY, Inc.**
**Minami-ku**
**Kyoto-shi**
**Kyoto 601-8045 (JP)**
- **BioEngineering Laboratories, LLC**
**Tokyo 160-0004 (JP)**

(72) Inventors:
- **Sode, Koji**
**Tokyo, 184-8588 (JP)**
- **Kojima, Katsuhiro**
**Tokyo, 184-8588 (JP)**

(74) Representative: **Jones, Elizabeth Louise**
**Dehns**
**St Bride's House**
**10 Salisbury Square**
**London**
**EC4Y 8JD (GB)**

(56) References cited:
**EP-A1- 1 739 174 EP-A1- 1 860 183**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 447 358 B1

SEQUENCE LISTING

<110> Arkray, Inc. BioEngineering Laboratories, LLC

<120> Mutant glucose dehydrogenase

<130> 42.13.110963

<150> JP2010-240426
<151> 2010-10-27

<160> 81

<170> PatentIn version 3.3

<210> 1
<211> 2467
<212> DNA
<213> Burkhorderia cepacia

<220>
<221> CDS
<222> (258)..(761)

<220>
<221> CDS
<222> (764)..(2380)

<220>
<221> CDS
<222> (2386)..(2466)

<400> 1

```
aagctttctg tttgattgca cgcgattcta accgagcgtc tgtgaggcgg aacgcgacat 60
gcttcgtgtc gcacacgtgt cgcgccgacg acacaaaaat gcagcgaaat ggctgatcgt 120
tacgaatggc tgacacattg aatggactat aaaaccattg tccgttccgg aatgtgcgcg 180
tacatttcag gtccgcgccg atttttgaga aatatcaagc gtggttttcc cgaatccggt 240
gttcgagaga aggaaac atg cac aac gac aac act ccc cac tcg cgt cgc    290
                  Met His Asn Asp Asn Thr Pro His Ser Arg Arg
                  1               5                   10
cac ggc gac gca gcc gca tca ggc atc acg cgg cgt caa tgg ttg caa   338
His Gly Asp Ala Ala Ala Ser Gly Ile Thr Arg Arg Gln Trp Leu Gln
            15                  20                  25
ggc gcg ctg gcg ctg acc gca gcg ggc ctc acg ggt tcg ctg aca ttg   386
Gly Ala Leu Ala Leu Thr Ala Ala Gly Leu Thr Gly Ser Leu Thr Leu
        30                  35                  40
cgg gcg ctt gca gac aac ccc ggc act gcg ccg ctc gat acg ttc atg   434
Arg Ala Leu Ala Asp Asn Pro Gly Thr Ala Pro Leu Asp Thr Phe Met
    45                  50                  55
acg ctt tcc gaa tcg ctg acc ggc aag aaa ggg ctc agc cgc gtg atc   482
Thr Leu Ser Glu Ser Leu Thr Gly Lys Lys Gly Leu Ser Arg Val Ile
60                  65                  70                  75
ggc gag cgc ctg ctg cag gcg ctg cag aag ggc tcg ttc aag acg gcc   530
Gly Glu Arg Leu Leu Gln Ala Leu Gln Lys Gly Ser Phe Lys Thr Ala
                80                  85                  90
gac agc ctg ccg cag ctc gcc ggc gcg ctc gcg tcc ggt tcg ctg acg   578
Asp Ser Leu Pro Gln Leu Ala Gly Ala Leu Ala Ser Gly Ser Leu Thr
            95                  100                 105
cct gaa cag gaa tcg ctc gca ctg acg atc ctc gag gcc tgg tat ctc   626
Pro Glu Gln Glu Ser Leu Ala Leu Thr Ile Leu Glu Ala Trp Tyr Leu
        110                 115                 120
ggc atc gtc gac aac gtc gtg att acg tac gag gaa gca tta atg ttc   674
Gly Ile Val Asp Asn Val Val Ile Thr Tyr Glu Glu Ala Leu Met Phe
    125                 130                 135
```

```
ggc gtc gtg tcc gat acg ctc gtg atc cgt tcg tat tgc ccc aac aaa    722
Gly Val Val Ser Asp Thr Leu Val Ile Arg Ser Tyr Cys Pro Asn Lys
140                 145                 150                 155
ccc ggc ttc tgg gcc gac aaa ccg atc gag agg caa gcc tg atg gcc     769
Pro Gly Phe Trp Ala Asp Lys Pro Ile Glu Arg Gln Ala    Met Ala
                160                 165                 170
gat acc gat acg caa aag gcc gac gtc gtc gtc gtt gga tcg ggt gtc    817
Asp Thr Asp Thr Gln Lys Ala Asp Val Val Val Val Gly Ser Gly Val
                175                 180                 185
gcg ggc gcg atc gtc gcg cat cag ctc gcg atg gcg ggc aag gcg gtg    865
Ala Gly Ala Ile Val Ala His Gln Leu Ala Met Ala Gly Lys Ala Val
            190                 195                 200
atc ctg ctc gaa gcg ggc ccg cgc atg ccg cgc tgg gaa atc gtc gag    913
Ile Leu Leu Glu Ala Gly Pro Arg Met Pro Arg Trp Glu Ile Val Glu
        205                 210                 215
cgc ttc cgc aat cag ccc gac aag atg gac ttc atg gcg ccg tac ccg    961
Arg Phe Arg Asn Gln Pro Asp Lys Met Asp Phe Met Ala Pro Tyr Pro
    220                 225                 230
tcg agc ccc tgg gcg ccg cat ccc gag tac ggc ccg ccg aac gac tac    1009
Ser Ser Pro Trp Ala Pro His Pro Glu Tyr Gly Pro Pro Asn Asp Tyr
235                 240                 245                 250
ctg atc ctg aag ggc gag cac aag ttc aac tcg cag tac atc cgc gcg    1057
Leu Ile Leu Lys Gly Glu His Lys Phe Asn Ser Gln Tyr Ile Arg Ala
                255                 260                 265
gtg ggc ggc acg acg tgg cac tgg gcc gcg tcg gcg tgg cgc ttc att    1105
Val Gly Gly Thr Thr Trp His Trp Ala Ala Ser Ala Trp Arg Phe Ile
            270                 275                 280
ccg aac gac ttc aag atg aag agc gtg tac ggc gtc ggc cgc gac tgg    1153
Pro Asn Asp Phe Lys Met Lys Ser Val Tyr Gly Val Gly Arg Asp Trp
        285                 290                 295
ccg atc cag tac gac gat ctc gag ccg tac tat cag cgc gcg gag gaa    1201
Pro Ile Gln Tyr Asp Asp Leu Glu Pro Tyr Tyr Gln Arg Ala Glu Glu
    300                 305                 310
gag ctc ggc gtg tgg ggc ccg ggc ccc gag gaa gat ctg tac tcg ccg    1249
Glu Leu Gly Val Trp Gly Pro Gly Pro Glu Glu Asp Leu Tyr Ser Pro
315                 320                 325                 330
cgc aag cag ccg tat ccg atg ccg ccg ctg ccg ttg tcg ttc aac gag    1297
Arg Lys Gln Pro Tyr Pro Met Pro Pro Leu Pro Leu Ser Phe Asn Glu
                335                 340                 345
cag acc atc aag acg gcg ctg aac aac tac gat ccg aag ttc cat gtc    1345
Gln Thr Ile Lys Thr Ala Leu Asn Asn Tyr Asp Pro Lys Phe His Val
            350                 355                 360
gtg acc gag ccg gtc gcg cgc aac agc cgc ccg tac gac ggc cgc ccg    1393
Val Thr Glu Pro Val Ala Arg Asn Ser Arg Pro Tyr Asp Gly Arg Pro
        365                 370                 375
act tgt tgc ggc aac aac aac tgc atg ccg atc tgc ccg atc ggc gcg    1441
Thr Cys Cys Gly Asn Asn Asn Cys Met Pro Ile Cys Pro Ile Gly Ala
    380                 385                 390
atg tac aac ggc atc gtg cac gtc gag aag gcc gaa cgc gcc ggc gcg    1489
Met Tyr Asn Gly Ile Val His Val Glu Lys Ala Glu Arg Ala Gly Ala
395                 400                 405                 410
aag ctg atc gag aac gcg gtc gtc tac aag ctc gag acg ggc ccg gac    1537
Lys Leu Ile Glu Asn Ala Val Val Tyr Lys Leu Glu Thr Gly Pro Asp
                415                 420                 425
aag cgc atc gtc gcg gcg ctc tac aag gac aag acg ggc gcc gag cat    1585
Lys Arg Ile Val Ala Ala Leu Tyr Lys Asp Lys Thr Gly Ala Glu His
            430                 435                 440
cgc gtc gaa ggc aag tat ttc gtg ctc gcc gcg aac ggc atc gag acg    1633
Arg Val Glu Gly Lys Tyr Phe Val Leu Ala Ala Asn Gly Ile Glu Thr
        445                 450                 455
ccg aag atc ctg ctg atg tcc gcg aac cgc gat ttc ccg aac ggt gtc    1681
Pro Lys Ile Leu Leu Met Ser Ala Asn Arg Asp Phe Pro Asn Gly Val
    460                 465                 470
gcg aac agc tcg gac atg gtc ggc cgc aac ctg atg gac cat ccg ggc    1729
Ala Asn Ser Ser Asp Met Val Gly Arg Asn Leu Met Asp His Pro Gly
```

```
475                 480                 485                 490
acc ggc gtg tcg ttc tat gcg agc gag aag ctg tgg ccg ggc cgc ggc    1777
Thr Gly Val Ser Phe Tyr Ala Ser Glu Lys Leu Trp Pro Gly Arg Gly
                495                 500                 505
ccg cag gag atg acg tcg ctg atc ggt ttc cgc gac ggt ccg ttc cgc    1825
Pro Gln Glu Met Thr Ser Leu Ile Gly Phe Arg Asp Gly Pro Phe Arg
            510                 515                 520
gcg acc gaa gcg gcg aag aag atc cac ctg tcg aac ctg tcg cgc atc    1873
Ala Thr Glu Ala Ala Lys Lys Ile His Leu Ser Asn Leu Ser Arg Ile
        525                 530                 535
gac cag gag acg cag aag atc ttc aag gcc ggc aag ctg atg aag ccc    1921
Asp Gln Glu Thr Gln Lys Ile Phe Lys Ala Gly Lys Leu Met Lys Pro
    540                 545                 550
gac gag ctc gac gcg cag atc cgc gac cgt tcc gca cgc tac gtg cag    1969
Asp Glu Leu Asp Ala Gln Ile Arg Asp Arg Ser Ala Arg Tyr Val Gln
555                 560                 565                 570
ttc gac tgc ttc cac gaa atc ctg ccg caa ccc gag aac cgc atc gtg    2017
Phe Asp Cys Phe His Glu Ile Leu Pro Gln Pro Glu Asn Arg Ile Val
                575                 580                 585
ccg agc aag acg gcg acc gat gcg atc ggc att ccg cgc ccc gag atc    2065
Pro Ser Lys Thr Ala Thr Asp Ala Ile Gly Ile Pro Arg Pro Glu Ile
            590                 595                 600
acg tat gcg atc gac gac tac gtg aag cgc ggc gcc gcg cat acg cgc    2113
Thr Tyr Ala Ile Asp Asp Tyr Val Lys Arg Gly Ala Ala His Thr Arg
        605                 610                 615
gag gtc tac gcg acc gcc gcg aag gtg ctc ggc ggc acg gac gtc gtg    2161
Glu Val Tyr Ala Thr Ala Ala Lys Val Leu Gly Gly Thr Asp Val Val
    620                 625                 630
ttc aac gac gaa ttc gcg ccg aac aat cac atc acg ggc tcg acg atc    2209
Phe Asn Asp Glu Phe Ala Pro Asn Asn His Ile Thr Gly Ser Thr Ile
635                 640                 645                 650
atg ggc gcc gat gcg cgc gac tcc gtc gtc gac aag gac tgc cgc acg    2257
Met Gly Ala Asp Ala Arg Asp Ser Val Val Asp Lys Asp Cys Arg Thr
                655                 660                 665
ttc gac cat ccg aac ctg ttc att tcg agc agc gcg acg atg ccg acc    2305
Phe Asp His Pro Asn Leu Phe Ile Ser Ser Ser Ala Thr Met Pro Thr
            670                 675                 680
gtc ggt acc gta aac gtg acg ctg acg atc gcc gcg ctc gcg ctg cgg    2353
Val Gly Thr Val Asn Val Thr Leu Thr Ile Ala Ala Leu Ala Leu Arg
        685                 690                 695
atg tcg gac acg ctg aag aag gaa gtc tgacc gtg cgg aaa tct act ctc 2403
Met Ser Asp Thr Leu Lys Lys Glu Val       Val Arg Lys Ser Thr Leu
    700                 705                       710
act ttc ctc atc gcc ggc tgc ctc gcg ttg ccg ggc ttc gcg cgc gcg    2451
Thr Phe Leu Ile Ala Gly Cys Leu Ala Leu Pro Gly Phe Ala Arg Ala
    715                 720                 725
gcc gat gcg gcc gat c                                              2467
Ala Asp Ala Ala Asp
730
```

<210> 2
<211> 168
<212> PRT
<213> Burkhorderia cepacia

<400> 2

Met His Asn Asp Asn Thr Pro His Ser Arg Arg His Gly Asp Ala Ala
1 5 10 15
Ala Ser Gly Ile Thr Arg Arg Gln Trp Leu Gln Gly Ala Leu Ala Leu
20 25 30
Thr Ala Ala Gly Leu Thr Gly Ser Leu Thr Leu Arg Ala Leu Ala Asp
35 40 45
Asn Pro Gly Thr Ala Pro Leu Asp Thr Phe Met Thr Leu Ser Glu Ser
50 55 60
Leu Thr Gly Lys Lys Gly Leu Ser Arg Val Ile Gly Glu Arg Leu Leu

65 70 75 80
Gln Ala Leu Gln Lys Gly Ser Phe Lys Thr Ala Asp Ser Leu Pro Gln
85 90 95
Leu Ala Gly Ala Leu Ala Ser Gly Ser Leu Thr Pro Glu Gln Glu Ser
100 105 110
Leu Ala Leu Thr Ile Leu Glu Ala Trp Tyr Leu Gly Ile Val Asp Asn
115 120 125
Val Val Ile Thr Tyr Glu Glu Ala Leu Met Phe Gly Val Val Ser Asp
130 135 140
Thr Leu Val Ile Arg Ser Tyr Cys Pro Asn Lys Pro Gly Phe Trp Ala
145 150 155 160
Asp Lys Pro Ile Glu Arg Gln Ala
165

<210> 3
<211> 539
<212> PRT
<213> Burkhorderia cepacia

<400> 3

Met Ala Asp Thr Asp Thr Gln Lys Ala Asp Val Val Val Val Gly Ser
1               5                   10                  15
Gly Val Ala Gly Ala Ile Val Ala His Gln Leu Ala Met Ala Gly Lys
            20                  25                  30
Ala Val Ile Leu Leu Glu Ala Gly Pro Arg Met Pro Arg Trp Glu Ile
        35                  40                  45
Val Glu Arg Phe Arg Asn Gln Pro Asp Lys Met Asp Phe Met Ala Pro
    50                  55                  60
Tyr Pro Ser Ser Pro Trp Ala Pro His Pro Glu Tyr Gly Pro Pro Asn
65                  70                  75                  80
Asp Tyr Leu Ile Leu Lys Gly Glu His Lys Phe Asn Ser Gln Tyr Ile
                85                  90                  95
Arg Ala Val Gly Gly Thr Thr Trp His Trp Ala Ala Ser Ala Trp Arg
            100                 105                 110
Phe Ile Pro Asn Asp Phe Lys Met Lys Ser Val Tyr Gly Val Gly Arg
        115                 120                 125
Asp Trp Pro Ile Gln Tyr Asp Asp Leu Glu Pro Tyr Tyr Gln Arg Ala
    130                 135                 140
Glu Glu Glu Leu Gly Val Trp Gly Pro Gly Pro Glu Glu Asp Leu Tyr
145                 150                 155                 160
Ser Pro Arg Lys Gln Pro Tyr Pro Met Pro Pro Leu Pro Leu Ser Phe
                165                 170                 175
Asn Glu Gln Thr Ile Lys Thr Ala Leu Asn Asn Tyr Asp Pro Lys Phe
            180                 185                 190
His Val Val Thr Glu Pro Val Ala Arg Asn Ser Arg Pro Tyr Asp Gly
        195                 200                 205
Arg Pro Thr Cys Cys Gly Asn Asn Asn Cys Met Pro Ile Cys Pro Ile
    210                 215                 220
Gly Ala Met Tyr Asn Gly Ile Val His Val Glu Lys Ala Glu Arg Ala
225                 230                 235                 240
Gly Ala Lys Leu Ile Glu Asn Ala Val Val Tyr Lys Leu Glu Thr Gly
                245                 250                 255
Pro Asp Lys Arg Ile Val Ala Ala Leu Tyr Lys Asp Lys Thr Gly Ala
            260                 265                 270
Glu His Arg Val Glu Gly Lys Tyr Phe Val Leu Ala Ala Asn Gly Ile
        275                 280                 285
Glu Thr Pro Lys Ile Leu Leu Met Ser Ala Asn Arg Asp Phe Pro Asn
    290                 295                 300
Gly Val Ala Asn Ser Ser Asp Met Val Gly Arg Asn Leu Met Asp His
305                 310                 315                 320
Pro Gly Thr Gly Val Ser Phe Tyr Ala Ser Glu Lys Leu Trp Pro Gly
                325                 330                 335
Arg Gly Pro Gln Glu Met Thr Ser Leu Ile Gly Phe Arg Asp Gly Pro
            340                 345                 350
Phe Arg Ala Thr Glu Ala Ala Lys Lys Ile His Leu Ser Asn Leu Ser

```
                355                 360                 365
Arg Ile Asp Gln Glu Thr Gln Lys Ile Phe Lys Ala Gly Lys Leu Met
    370                 375                 380
Lys Pro Asp Glu Leu Asp Ala Gln Ile Arg Asp Arg Ser Ala Arg Tyr
385                 390                 395                 400
Val Gln Phe Asp Cys Phe His Glu Ile Leu Pro Gln Pro Glu Asn Arg
                405                 410                 415
Ile Val Pro Ser Lys Thr Ala Thr Asp Ala Ile Gly Ile Pro Arg Pro
            420                 425                 430
Glu Ile Thr Tyr Ala Ile Asp Asp Tyr Val Lys Arg Gly Ala Ala His
        435                 440                 445
Thr Arg Glu Val Tyr Ala Thr Ala Ala Lys Val Leu Gly Gly Thr Asp
    450                 455                 460
Val Val Phe Asn Asp Glu Phe Ala Pro Asn Asn His Ile Thr Gly Ser
465                 470                 475                 480
Thr Ile Met Gly Ala Asp Ala Arg Asp Ser Val Val Asp Lys Asp Cys
                485                 490                 495
Arg Thr Phe Asp His Pro Asn Leu Phe Ile Ser Ser Ser Ala Thr Met
            500                 505                 510
Pro Thr Val Gly Thr Val Asn Val Thr Leu Thr Ile Ala Ala Leu Ala
        515                 520                 525
Leu Arg Met Ser Asp Thr Leu Lys Lys Glu Val
    530                 535
```

<210> 4
<211> 27
<212> PRT
<213> Burkhorderia cepacia

<400> 4

```
Val Arg Lys Ser Thr Leu Thr Phe Leu Ile Ala Gly Cys Leu Ala Leu
 1              5                   10                  15
Pro Gly Phe Ala Arg Ala Ala Asp Ala Ala Asp
            20                  25
```

<210> 5
<211> 1441
<212> DNA
<213> Burkholderia cepacia

<220>
<221> CDS
<222> (121)..(1398)

<400> 5

```
tccgaacctg ttcatttcga gcagcgcgac gatgccgacc gtcggtaccg taaacgtgac 60
gctgacgatc gccgcgctcg cgctgcggat gtcggacacg ctgaagaagg aagtctgacc 120
gtg cgg aaa tct act ctc act ttc ctc atc gcc ggc tgc ctc gcg ttg   168
Val Arg Lys Ser Thr Leu Thr Phe Leu Ile Ala Gly Cys Leu Ala Leu
 1               5                  10                  15
ccg ggc ttc gcg cgc gcg gcc gat gcg gcc gat ccg gcg ctg gtc aag   216
Pro Gly Phe Ala Arg Ala Ala Asp Ala Ala Asp Pro Ala Leu Val Lys
            20                  25                  30
cgc ggc gaa tac ctc gcg acc gcc ggc gac tgc atg gcc tgc cac acc   264
Arg Gly Glu Tyr Leu Ala Thr Ala Gly Asp Cys Met Ala Cys His Thr
        35                  40                  45
gtg aag ggc ggc aag ccg tac gcg ggc ggc ctt ggc atg ccg gta ccg   312
Val Lys Gly Gly Lys Pro Tyr Ala Gly Gly Leu Gly Met Pro Val Pro
    50                  55                  60
atg ctc ggc aag atc tac acg agc aac atc acg ccc gat ccc gat acg   360
Met Leu Gly Lys Ile Tyr Thr Ser Asn Ile Thr Pro Asp Pro Asp Thr
65                  70                  75                  80
ggc atc ggc aaa tgg acg ttc gag gac ttc gag cgc gcg gtg cgg cac   408
Gly Ile Gly Lys Trp Thr Phe Glu Asp Phe Glu Arg Ala Val Arg His
                85                  90                  95
```

```
ggc gtg tcg aag aac ggc gac aac ctg tat ccg gcg atg ccg tac gtg    456
Gly Val Ser Lys Asn Gly Asp Asn Leu Tyr Pro Ala Met Pro Tyr Val
            100                 105                 110
tcg tac gcg aag atc acg gac gac gac gta cgc gcg ctg tac gcc tac    504
Ser Tyr Ala Lys Ile Thr Asp Asp Asp Val Arg Ala Leu Tyr Ala Tyr
        115                 120                 125
ttc atg cac ggc gtc gag ccg gtc aag cag gcg ccg ccg aag aac gag    552
Phe Met His Gly Val Glu Pro Val Lys Gln Ala Pro Pro Lys Asn Glu
    130                 135                 140
att ccc gcg ctg ctc agc atg cgc tgg ccg ctg aag atc tgg aac tgg    600
Ile Pro Ala Leu Leu Ser Met Arg Trp Pro Leu Lys Ile Trp Asn Trp
145                 150                 155                 160
ctg ttc ctg aag gac ggc ccg tac cag ccg aag ccg tcg cag agc gcc    648
Leu Phe Leu Lys Asp Gly Pro Tyr Gln Pro Lys Pro Ser Gln Ser Ala
                165                 170                 175
gaa tgg aat cgc ggc gcg tat ctg gtg cag ggt ctc gcg cac tgc agc    696
Glu Trp Asn Arg Gly Ala Tyr Leu Val Gln Gly Leu Ala His Cys Ser
            180                 185                 190
acg tgc cac acg ccg cgc ggc atc gcg atg cag gag aag tcg ctc gac    744
Thr Cys His Thr Pro Arg Gly Ile Ala Met Gln Glu Lys Ser Leu Asp
        195                 200                 205
gaa acc ggc ggc agc ttc ctc gcg ggg tcg gtg ctc gcc ggc tgg gac    792
Glu Thr Gly Gly Ser Phe Leu Ala Gly Ser Val Leu Ala Gly Trp Asp
    210                 215                 220
ggc tac aac atc acg tcg gac ccg aat gcg ggg atc ggc agc tgg acg    840
Gly Tyr Asn Ile Thr Ser Asp Pro Asn Ala Gly Ile Gly Ser Trp Thr
225                 230                 235                 240
cag cag cag ctc gtg cag tat ttg cgc acc ggc agc gtg ccg ggc gtc    888
Gln Gln Gln Leu Val Gln Tyr Leu Arg Thr Gly Ser Val Pro Gly Val
                245                 250                 255
gcg cag gcg gcc ggg ccg atg gcc gag gcg gtc gag cac agc ttc tcg    936
Ala Gln Ala Ala Gly Pro Met Ala Glu Ala Val Glu His Ser Phe Ser
            260                 265                 270
aag atg acc gaa gcg gac atc ggt gcg atc gcc acg tac gtc cgc acg    984
Lys Met Thr Glu Ala Asp Ile Gly Ala Ile Ala Thr Tyr Val Arg Thr
        275                 280                 285
gtg ccg gcc gtt gcc gac agc aac gcg aag cag ccg cgg tcg tcg tgg    1032
Val Pro Ala Val Ala Asp Ser Asn Ala Lys Gln Pro Arg Ser Ser Trp
    290                 295                 300
ggc aag ccg gcc gag gac ggg ctg aag ctg cgc ggt gtc gcg ctc gcg    1080
Gly Lys Pro Ala Glu Asp Gly Leu Lys Leu Arg Gly Val Ala Leu Ala
305                 310                 315                 320
tcg tcg ggc atc gat ccg gcg cgg ctg tat ctc ggc aac tgc gcg acg    1128
Ser Ser Gly Ile Asp Pro Ala Arg Leu Tyr Leu Gly Asn Cys Ala Thr
                325                 330                 335
tgc cac cag atg cag ggc aag ggc acg ccg gac ggc tat tac ccg tcg    1176
Cys His Gln Met Gln Gly Lys Gly Thr Pro Asp Gly Tyr Tyr Pro Ser
            340                 345                 350
ctg ttc cac aac tcc acc gtc ggc gcg tcg aat ccg tcg aac ctc gtg    1224
Leu Phe His Asn Ser Thr Val Gly Ala Ser Asn Pro Ser Asn Leu Val
        355                 360                 365
cag gtg atc ctg aac ggc gtg cag cgc aag atc ggc agc gag gat atc    1272
Gln Val Ile Leu Asn Gly Val Gln Arg Lys Ile Gly Ser Glu Asp Ile
    370                 375                 380
ggg atg ccc gct ttc cgc tac gat ctg aac gac gcg cag atc gcc gcg    1320
Gly Met Pro Ala Phe Arg Tyr Asp Leu Asn Asp Ala Gln Ile Ala Ala
385                 390                 395                 400
ctg acg aac tac gtg acc gcg cag ttc ggc aat ccg gcg gcg aag gtg    1368
Leu Thr Asn Tyr Val Thr Ala Gln Phe Gly Asn Pro Ala Ala Lys Val
                405                 410                 415
acg gag cag gac gtc gcg aag ctg cgc tga catagtcggg cgcgccgaca      1418
Thr Glu Gln Asp Val Ala Lys Leu Arg
            420                 425
cggcgcaacc gataggacag gag                                          1441
```

<210> 6
<211> 425
<212> PRT
<213> Burkholderia cepacia

<400> 6

```
Val Arg Lys Ser Thr Leu Thr Phe Leu Ile Ala Gly Cys Leu Ala Leu
1               5                   10                  15
Pro Gly Phe Ala Arg Ala Ala Asp Ala Ala Asp Pro Ala Leu Val Lys
            20                  25                  30
Arg Gly Glu Tyr Leu Ala Thr Ala Gly Asp Cys Met Ala Cys His Thr
        35                  40                  45
Val Lys Gly Gly Lys Pro Tyr Ala Gly Gly Leu Gly Met Pro Val Pro
    50                  55                  60
Met Leu Gly Lys Ile Tyr Thr Ser Asn Ile Thr Pro Asp Pro Asp Thr
65                  70                  75                  80
Gly Ile Gly Lys Trp Thr Phe Glu Asp Phe Glu Arg Ala Val Arg His
                85                  90                  95
Gly Val Ser Lys Asn Gly Asp Asn Leu Tyr Pro Ala Met Pro Tyr Val
            100                 105                 110
Ser Tyr Ala Lys Ile Thr Asp Asp Asp Val Arg Ala Leu Tyr Ala Tyr
        115                 120                 125
Phe Met His Gly Val Glu Pro Val Lys Gln Ala Pro Pro Lys Asn Glu
    130                 135                 140
Ile Pro Ala Leu Leu Ser Met Arg Trp Pro Leu Lys Ile Trp Asn Trp
145                 150                 155                 160
Leu Phe Leu Lys Asp Gly Pro Tyr Gln Pro Lys Pro Ser Gln Ser Ala
                165                 170                 175
Glu Trp Asn Arg Gly Ala Tyr Leu Val Gln Gly Leu Ala His Cys Ser
            180                 185                 190
Thr Cys His Thr Pro Arg Gly Ile Ala Met Gln Glu Lys Ser Leu Asp
        195                 200                 205
Glu Thr Gly Gly Ser Phe Leu Ala Gly Ser Val Leu Ala Gly Trp Asp
    210                 215                 220
Gly Tyr Asn Ile Thr Ser Asp Pro Asn Ala Gly Ile Gly Ser Trp Thr
225                 230                 235                 240
Gln Gln Gln Leu Val Gln Tyr Leu Arg Thr Gly Ser Val Pro Gly Val
                245                 250                 255
Ala Gln Ala Ala Gly Pro Met Ala Glu Ala Val Glu His Ser Phe Ser
            260                 265                 270
Lys Met Thr Glu Ala Asp Ile Gly Ala Ile Ala Thr Tyr Val Arg Thr
        275                 280                 285
Val Pro Ala Val Ala Asp Ser Asn Ala Lys Gln Pro Arg Ser Ser Trp
    290                 295                 300
Gly Lys Pro Ala Glu Asp Gly Leu Lys Leu Arg Gly Val Ala Leu Ala
305                 310                 315                 320
Ser Ser Gly Ile Asp Pro Ala Arg Leu Tyr Leu Gly Asn Cys Ala Thr
                325                 330                 335
Cys His Gln Met Gln Gly Lys Gly Thr Pro Asp Gly Tyr Tyr Pro Ser
            340                 345                 350
Leu Phe His Asn Ser Thr Val Gly Ala Ser Asn Pro Ser Asn Leu Val
        355                 360                 365
Gln Val Ile Leu Asn Gly Val Gln Arg Lys Ile Gly Ser Glu Asp Ile
    370                 375                 380
Gly Met Pro Ala Phe Arg Tyr Asp Leu Asn Asp Ala Gln Ile Ala Ala
385                 390                 395                 400
Leu Thr Asn Tyr Val Thr Ala Gln Phe Gly Asn Pro Ala Ala Lys Val
                405                 410                 415
Thr Glu Gln Asp Val Ala Lys Leu Arg
            420                 425
```

<210> 7
<211> 539
<212> PRT
<213> Burkholderia cenocepacia

<400> 7

Met Ala Asp Thr Asp Thr Gln Lys Ala Asp Val Val Val Val Gly Ser
1 5 10 15

Gly Val Ala Gly Ala Ile Val Ala His Gln Leu Ala Met Ala Gly Lys
20 25 30

Ser Val Ile Leu Leu Glu Ala Gly Pro Arg Met Pro Arg Trp Glu Ile
35 40 45

Val Glu Arg Phe Arg Asn Gln Pro Asp Lys Thr Asp Phe Met Ala Pro
50 55 60

Tyr Pro Ser Ser Pro Trp Ala Pro His Pro Glu Tyr Gly Pro Pro Asn
65 70 75 80

Asp Tyr Leu Ile Leu Lys Gly Glu His Lys Phe Asn Ser Gln Tyr Ile
85 90 95

Arg Ala Val Gly Gly Thr Thr Trp His Trp Ala Ala Ser Ala Trp Arg
100 105 110

Phe Ile Pro Asn Asp Phe Lys Met Lys Thr Val Tyr Gly Val Ala Arg
115 120 125

Asp Trp Pro Ile Gln Tyr Asp Asp Leu Glu His Trp Tyr Gln Arg Ala
130 135 140

Glu Glu Glu Leu Gly Val Trp Gly Pro Gly Pro Glu Glu Asp Leu Tyr
145 150 155 160

Ser Pro Arg Lys Gln Ala Tyr Pro Met Pro Pro Leu Pro Leu Ser Phe
165 170 175

Asn Glu Gln Thr Ile Lys Ser Ala Leu Asn Gly Tyr Asp Pro Lys Phe
180 185 190

His Val Val Thr Glu Pro Val Ala Arg Asn Ser Arg Pro Tyr Asp Gly
195 200 205

Arg Pro Thr Cys Cys Gly Asn Asn Asn Cys Met Pro Ile Cys Pro Ile
210 215 220

Gly Ala Met Tyr Asn Gly Ile Val His Val Glu Lys Ala Glu Gln Ala
225 230 235 240

Gly Ala Lys Leu Ile Asp Ser Ala Val Val Tyr Lys Leu Glu Thr Gly

245 250 255

Pro Asp Lys Arg Ile Val Ala Ala Ile Tyr Lys Asp Lys Thr Gly Ala
260 265 270

Asp His Arg Val Glu Gly Lys Tyr Phe Val Leu Ala Ala Asn Gly Ile
275 280 285

Glu Thr Pro Lys Ile Leu Leu Met Ser Ala Asn Arg Asp Phe Pro Asn
290 295 300

Gly Val Ala Asn Ser Ser Asp Met Val Gly Arg Asn Leu Met Asp His
305 310 315 320

Pro Gly Thr Gly Val Ser Phe Tyr Ala Asn Glu Lys Leu Trp Pro Gly
325 330 335

Arg Gly Pro Gln Glu Met Thr Ser Leu Ile Gly Phe Arg Asp Gly Pro
340 345 350

Phe Arg Ala Thr Glu Ala Ala Lys Lys Ile His Leu Ser Asn Met Ser
355 360 365

Arg Ile Asn Gln Glu Thr Gln Lys Ile Phe Lys Ala Gly Lys Leu Met
370 375 380

Lys His Glu Glu Leu Asp Ala Gln Ile Arg Asp Arg Ser Ala Arg Tyr
385 390 395 400

Val Gln Phe Asp Cys Phe His Glu Ile Leu Pro Gln Pro Glu Asn Arg
405 410 415

Ile Val Pro Ser Lys Thr Ala Thr Asp Ala Ile Gly Ile Pro Arg Pro
420 425 430

Glu Ile Thr Tyr Ala Ile Asp Asp Tyr Val Lys Arg Gly Ala Val His
435 440 445

Thr Arg Glu Val Tyr Ala Thr Ala Ala Lys Val Leu Gly Gly Thr Asp
450 455 460

Val Val Phe Asn Asp Glu Phe Ala Pro Asn Asn His Ile Thr Gly Ala
465 470 475 480

Thr Ile Met Gly Ala Asp Ala Arg Asp Ser Val Val Asp Lys Asp Cys
485 490 495

Arg Thr Phe Asp His Pro Asn Leu Phe Ile Ser Ser Ser Ser Thr Met
500 505 510

Pro Thr Val Gly Thr Val Asn Val Thr Leu Thr Ile Ala Ala Leu Ala
515 520 525

Leu Arg Met Ser Asp Thr Leu Lys Lys Glu Val
530 535

<210> 8
<211> 537
<212> PRT
<213> Burkholderia thailandensis

<400> 8

Met Ala Glu Thr Gln Gln Ala Asp Val Val Val Val Gly Ser Gly Val
1 5 10 15

Ala Gly Ala Ile Val Ala His Gln Leu Ala Met Ala Gly Lys Ser Val
20 25 30

Ile Leu Leu Glu Ala Gly Pro Arg Met Pro Arg Trp Glu Ile Val Glu
35 40 45

Arg Phe Arg Asn Gln Pro Asp Lys Met Asp Phe Met Ala Pro Tyr Pro
50 55 60

Ser Ser Ala Trp Ala Pro His Pro Glu Tyr Ala Pro Pro Asn Asp Tyr
65 70 75 80

Leu Val Leu Lys Gly Glu His Lys Phe Asn Ser Gln Tyr Ile Arg Ala
85 90 95

Val Gly Gly Thr Thr Trp His Trp Ala Ala Ser Ala Trp Arg Phe Ile
100 105 110

Pro Asn Asp Phe Lys Met Lys Thr Val Tyr Gly Val Gly Arg Asp Trp
115 120 125

Pro Ile Gln Tyr Asp Asp Leu Glu His Phe Tyr Gln Arg Ala Glu Glu
130 135 140

Glu Leu Gly Val Trp Gly Pro Gly Ala Glu Glu Asp Leu Leu Ser Pro
145 150 155 160

Arg Lys Ala Pro Tyr Pro Met Pro Pro Leu Pro Leu Ser Tyr Asn Glu
165 170 175

Arg Thr Ile Lys Thr Ala Leu Asn Asn His Asp Pro Lys Tyr His Val
180 185 190

Val Thr Glu Pro Val Ala Arg Asn Ser Arg Pro Tyr Asp Gly Arg Pro
195 200 205

Thr Cys Cys Gly Asn Asn Asn Cys Met Pro Ile Cys Pro Ile Gly Ala
210 215 220

Met Tyr Asn Gly Ile Val His Val Glu Lys Ala Glu Gln Ala Gly Ala
225 230 235 240

Lys Leu Ile Glu Asn Ala Val Val His Lys Leu Glu Val Gly Pro Gln
245 250 255

Lys Lys Ile Val Ala Ala Leu Tyr Lys Asp Pro Lys Gly Ala Glu His
260 265 270

Arg Val Glu Gly Lys Tyr Phe Val Leu Ala Ala Asn Gly Ile Glu Thr
275 280 285

Pro Lys Leu Met Leu Met Ser Thr Ser His Asp Phe Pro Asn Gly Val
290 295 300

Gly Asn Ser Ser Asp Met Val Gly Arg Asn Leu Met Asp His Pro Gly
305 310 315 320

Thr Gly Val Ser Phe Tyr Ala Ser Glu Lys Leu Trp Pro Gly Arg Gly
325 330 335

Pro Gln Glu Met Thr Ser Leu Ile Gly Phe Arg Asp Gly Pro Phe Arg
340 345 350

Ala Thr Glu Ala Ala Lys Lys Ile His Leu Ser Asn Leu Ser Arg Ile
355 360 365

Asp Gln Glu Thr Gln Lys Ile Phe Lys Ala Gly Lys Leu Leu Lys Pro
370 375 380

Ala Glu Leu Asp Ala Gln Ile Arg Asp Arg Ser Ala Arg Tyr Val Gln
385 390 395 400

Phe Asp Cys Phe His Glu Ile Leu Pro Gln Pro Glu Asn Arg Ile Val
405 410 415

Pro Ser Lys Thr Ala Thr Asp Ala Ile Gly Ile Pro Arg Pro Glu Ile
420 425 430

Thr Tyr Ala Ile Asp Asp Tyr Val Lys Arg Gly Ala Ala His Thr Arg
435 440 445

Glu Val Tyr Ala Ser Ala Ala Gln Val Leu Gly Gly Thr Asp Val Val

450 455 460

Phe Asn Asp Glu Phe Ala Pro Asn Asn His Ile Thr Gly Ala Thr Ile
465 470 475 480

Met Gly Ala Asp Pro Arg Asp Ser Val Val Asp Lys Asp Cys Arg Thr
485 490 495

Phe Asp His Pro Asn Leu Phe Ile Ser Ser Ser Ala Thr Met Pro Thr
500 505 510

Val Gly Thr Val Asn Val Thr Leu Thr Ile Ala Ala Leu Ala Leu Arg
515 520 525

Ile Ser Asp Gln Leu Lys Lys Glu Ile
530 535

<210> 9
<211> 540
<212> PRT
<213> Ralstonia pickettii

<400> 9

Met Ala Gln Ser Glu Gln Thr Arg Gln Gln Ala Asp Ile Val Val Val
1 5 10 15

Gly Ser Gly Val Ala Gly Ala Leu Val Ala Tyr Glu Leu Ala Arg Ala
20 25 30

Gly Lys Ser Val Leu Met Leu Glu Ala Gly Pro Arg Leu Pro Arg Trp
35 40 45

Glu Ile Val Glu Arg Phe Arg Asn Gln Ala Asp Lys Met Asp Phe Met
50 55 60

Ala Pro Tyr Pro Ser Thr Ala Trp Ala Pro His Pro Glu Tyr Gly Pro
65 70 75 80

Pro Asn Asn Tyr Leu Val Leu Lys Gly Glu His Gln Phe Asn Ser Gln
85 90 95

Tyr Ile Arg Ala Val Gly Gly Thr Thr Trp His Trp Ala Ala Ser Thr
100 105 110

Trp Arg Phe Leu Pro Asn Asp Phe Lys Leu Arg Ser Val Tyr Gly Ile
115 120 125

Ala Arg Asp Trp Pro Ile Gln Tyr Gln Asp Leu Glu Arg Tyr Tyr Gly
130 135 140

Leu Ala Glu Glu Ala Leu Gly Val Trp Gly Pro Asn Asp Glu Asp Leu
145 150 155 160

Gly Ser Pro Arg Ser Gln Pro Tyr Pro Met Thr Pro Leu Pro Leu Ser
165 170 175

Phe Asn Glu Arg Thr Ile Lys Glu Ala Leu Asn Ala His Asp Ala Ser
180 185 190

Phe His Val Val Thr Glu Pro Val Ala Arg Asn Ser Arg Pro Tyr Asp
195 200 205

Gly Arg Pro Thr Cys Cys Gly Asn Asn Asn Cys Met Pro Ile Cys Pro
210 215 220

Ile Gly Ala Met Tyr Asn Gly Ile Val His Val Glu Lys Ala Glu Gln
225 230 235 240

Ala Gly Ala Arg Leu Ile Glu Asn Ala Val Val Phe Lys Leu Glu Val
245 250 255

Gly Pro Asn Lys Arg Ile Val Ala Ala Arg Tyr Lys Asp Ser Lys Gly
260 265 270

Ala Glu His Arg Val Glu Gly Lys Trp Phe Val Leu Ala Ala Asn Gly
275 280 285

Ile Glu Thr Pro Lys Leu Met Leu Met Ser Thr Ser Gln Asp Phe Pro
290 295 300

Lys Gly Val Gly Asn Ser Ser Asp Met Val Gly Arg Asn Leu Met Asp
305 310 315 320

His Pro Gly Thr Gly Val Ser Phe Tyr Ala Asp Arg Lys Leu Trp Pro
325 330 335

Gly Arg Gly Pro Gln Glu Met Thr Ser Leu Ile Gly Phe Arg Asp Gly
340 345 350

Pro Phe Arg Ala Thr Gln Ala Gly Lys Lys Leu His Leu Ser Asn Ile
355 360 365

Ser Arg Ile Glu Gln Glu Thr Gln Arg Ile Phe Lys Glu Gly Lys Leu
370 375 380

Ile Lys Pro Ala Asp Leu Asp Ala Arg Ile Arg Asp Gln Ala Ala Arg
385 390 395 400

Tyr Val Gln Phe Asp Ser Phe His Glu Ile Leu Pro Leu Pro Glu Asn
405 410 415

Arg Ile Val Pro Ser Ala Thr Glu Val Asp Ala Ile Gly Ile Pro Arg
420 425 430

Pro Glu Ile Thr Tyr His Ile Asp Asp Tyr Val Lys Arg Ser Ala Val
435 440 445

His Thr Arg Glu Val Tyr Ala Thr Ala Ala Gln Val Met Gly Gly Thr
450 455 460

Asn Val Glu Phe His Asp Asp Phe Ala Pro Asn Asn His Ile Thr Gly
465 470 475 480

Ala Thr Ile Met Gly Ala Asp Pro Lys Asp Ser Val Val Asp Lys Asp
485 490 495

Cys Arg Thr Phe Asp His Pro Asn Leu Phe Ile Ser Ser Ser Ser Thr
500 505 510

Met Pro Thr Val Gly Thr Val Asn Val Thr Leu Thr Ile Ala Ala Leu
515 520 525

Ala Leu Arg Ile Ala Asp Gln Leu Lys Gln Glu Ala
530 535 540

<210> 10
<211> 540
<212> PRT
<213> Ralstonia solanacearum

<400> 10

Met Ala Asp Thr Arg Arg Ala Asp Gln Ala Asp Ile Val Val Val Gly
1               5                   10                  15

Ser Gly Val Ala Gly Ala Leu Val Ala Tyr Glu Leu Ala Arg Ala Gly
            20                  25                  30

Lys Ser Val Leu Met Leu Glu Ala Gly Pro Arg Leu Pro Arg Trp Glu
        35                  40                  45

Ile Val Glu Arg Phe Arg Asn Gln Ala Asp Lys Met Asp Phe Met Ala
    50                  55                  60

Pro Tyr Pro Ser Thr Pro Trp Ala Pro His Pro Glu Tyr Gly Pro Ser
65                  70                  75                  80

Pro Asn Asp Tyr Leu Val Leu Lys Gly Glu His Lys Phe Asp Ser Gln
                85                  90                  95

Tyr Ile Arg Ala Val Gly Gly Thr Thr Trp His Trp Ala Ala Ser Thr
100 105 110

Trp Arg Phe Leu Pro Asn Asp Phe Lys Leu Arg Ser Val Tyr Gly Ile
115 120 125

Ala Arg Asp Trp Pro Leu Gln Tyr Asp Asp Leu Glu Arg Asp Tyr Gly
130 135 140

Arg Ala Glu Ala Ala Leu Gly Val Trp Gly Pro Asn Asp Glu Asp Leu
145 150 155 160

Gly Ser Pro Arg Ser Gln Pro Tyr Pro Met Ala Pro Leu Pro Leu Ser
165 170 175

Phe Asn Glu Arg Thr Ile Lys Glu Ala Leu Asn Ala His Asp Pro Ala
180 185 190

Phe His Val Val Thr Glu Pro Val Ala Arg Asn Ser Arg Pro Tyr Asp
195 200 205

Gly Arg Pro Thr Cys Cys Gly Asn Asn Asn Cys Met Pro Ile Cys Pro
210 215 220

Ile Gly Ala Met Tyr Asn Gly Ile Val His Val Glu Lys Ala Glu Gln
225 230 235 240

Ala Gly Ala Arg Leu Ile Glu Asn Ala Val Val Tyr Lys Leu Glu Val
245 250 255

Gly Ala Gly Arg Arg Ile Val Ala Ala His Tyr Lys Asp Pro Lys Gly
260 265 270

Val Asp His Arg Val Glu Gly Lys Trp Phe Val Leu Ala Ala Asn Gly
275 280 285

Ile Glu Thr Pro Lys Leu Met Leu Met Ser Thr Ser Glu Ala Phe Pro
290 295 300

Arg Gly Val Gly Asn Ser Ser Asp Met Val Gly Arg Asn Leu Met Asp
305 310 315 320

His Pro Gly Thr Gly Val Ser Phe Tyr Ala Asp Arg Lys Leu Trp Pro
325 330 335

Gly Arg Gly Pro Gln Glu Met Thr Ser Leu Ile Gly Phe Arg Asp Gly
340 345 350

Pro Phe Arg Ala Met Gln Ala Gly Lys Lys Leu His Leu Ser Asn Ile
355 360 365

Ser Arg Ile Glu Gln Glu Thr Ala Arg Ile Phe Lys Ala Gly Lys Leu
370 375 380

Leu Lys Pro Ala Glu Leu Asp Ala Arg Ile Arg Asp Gln Ala Ala Arg
385 390 395 400

Tyr Val Gln Phe Asp Ser Phe His Glu Ile Leu Pro Leu Pro Glu Asn
405 410 415

Arg Ile Val Pro Ser Ala Thr Glu Thr Asp Ala Leu Gly Ile Pro Arg
420 425 430

Pro Glu Ile Thr Tyr Arg Ile Asp Asp Tyr Val Lys Arg Ser Ala Val
435 440 445

His Thr Arg Glu Val Tyr Ala Thr Ala Ala Lys Val Leu Gly Ala Thr
450 455 460

Asp Val Gln Phe His Asp Asp Phe Ala Pro Asn Asn His Ile Thr Gly
465 470 475 480

Ala Thr Ser Met Gly Ala Asp Pro Lys Asp Ser Val Val Asp Lys Asp
485 490 495

Cys Arg Thr Phe Asp His Pro Asn Leu Phe Ile Ser Ser Ser Ala Thr
500 505 510

Met Pro Thr Val Gly Thr Val Asn Val Thr Leu Thr Ile Ala Ala Leu
515 520 525

Ala Leu Arg Ile Ala Asp Arg Leu Lys Lys Glu Ala
530 535 540

<210> 11
<211> 533
<212> PRT
<213> Burkholderia phytofirmans

<400> 11

```
Met Ala Asn Lys Asn Ser Ala Asp Ile Val Val Val Gly Ser Gly Val
1               5                   10                  15

Ala Gly Gly Leu Val Ala His Gln Met Ala Leu Ala Gly Ala Ser Val
            20                  25                  30

Ile Leu Leu Glu Ala Gly Pro Arg Ile Pro Arg Trp Gln Ile Val Glu
```

35 40 45

Asn Phe Arg Asn Ser Pro Val Lys Ser Asp Phe Ala Thr Pro Tyr Pro
50 55 60

Ser Thr Pro Tyr Ala Pro His Pro Glu Tyr Ala Pro Ala Asn Asn Tyr
65 70 75 80

Leu Ile Gln Lys Gly Asp Tyr Pro Tyr Ser Ser Gln Tyr Leu Arg Leu
85 90 95

Val Gly Gly Thr Thr Trp His Trp Ala Ala Ala Ala Trp Arg Leu Leu
100 105 110

Pro Ser Asp Phe Gln Leu His Lys Leu Tyr Gly Val Gly Arg Asp Trp
115 120 125

Pro Tyr Pro Tyr Glu Thr Leu Glu Pro Trp Tyr Ser Ala Ala Glu Val
130 135 140

Gln Leu Gly Val Ser Gly Pro Gly Asn Ser Ile Asp Leu Gly Ser Pro
145 150 155 160

Arg Ser Lys Pro Tyr Pro Met Asn Pro Leu Pro Leu Ser Tyr Met Asp
165 170 175

Gln Arg Phe Ser Asp Val Leu Asn Ala Gln Gly Phe Lys Val Val Pro
180 185 190

Glu Pro Val Ala Arg Asn Ser Arg Pro Tyr Asp Ala Arg Pro Thr Cys
195 200 205

Cys Gly Asn Asn Asn Cys Met Pro Ile Cys Pro Ile Ala Ala Met Tyr
210 215 220

Asn Gly Val Val His Ala Glu Lys Ala Glu Gln Ala Gly Ala Lys Leu
225 230 235 240

Ile Pro Glu Ala Val Val Tyr Arg Val Glu Ala Asp Asn Lys Gly Leu
245 250 255

Ile Thr Ala Val His Tyr Lys Asp Pro Asn Gly Asn Ser Thr Arg Val
260 265 270

Thr Gly Lys Leu Phe Val Leu Ala Ala Asn Gly Ile Glu Thr Pro Lys
275 280 285

Leu Met Leu Met Ser Thr Ser Asp Lys Phe Pro His Gly Val Gly Asn
290 295 300

Ser Ser Asp Gln Val Gly Arg Asn Leu Met Asp His Pro Gly Thr Gly
305 310 315 320

Val Thr Phe Leu Ala Asn Glu Ala Leu Trp Pro Gly Arg Gly Pro Met
325 330 335

Glu Met Thr Ser Ile Val Asn Phe Arg Asp Gly Ala Phe Arg Ser Asp
340 345 350

Tyr Ala Ala Lys Lys Leu His Leu Ser Asn Gly Val Pro Thr Met Ser
355 360 365

Val Thr Ala Asp Leu Leu Lys Lys Gly Leu Thr Gly Ala Glu Leu Asp
370 375 380

Arg Gln Ile Arg Asp Arg Ala Ala Arg Thr Leu Asn Ile Asn Ser Phe
385 390 395 400

His Glu His Leu Ala Glu Pro Gln Asn Arg Val Val Pro Ser Ala Asp
405 410 415

His Lys Asp Ser Leu Gly Ile Pro Gln Pro Glu Ile Tyr Tyr Ser Ile
420 425 430

Asn Asp Tyr Val Lys Lys Ser Ala Ala Asn Thr His Glu Leu Tyr Ala
435 440 445

Gln Ile Ala Ala Leu Phe Gly Gly Ala Glu Val Thr Phe Asp Asp Thr
450 455 460

Phe Ala Pro Asn Asn His Ile Met Gly Thr Thr Ile Met Gly Ser Asp
465 470 475 480

Pro Ala Asp Ser Val Val Asp Ala Asp Cys Arg Thr His Asp His Ser
485 490 495

Asn Leu Phe Ile Ala Ser Ser Gly Val Met Pro Thr Ala Ala Ser Val
500 505 510

Asn Cys Thr Leu Thr Ile Ala Ala Leu Ser Leu Lys Leu Ala Asp Lys
515 520 525

Leu Lys Arg Glu Ile
530

<210> 12
<211> 539
<212> PRT

<213> Burkholderia cepacia

<400> 12

```
Met Ala Asp Thr Asp Thr Gln Lys Ala Asp Val Val Val Val Gly Ser
1               5                   10                  15

Gly Val Ala Gly Ala Ile Val Ala His Gln Leu Ala Met Ala Gly Lys
            20                  25                  30

Ser Val Ile Leu Leu Glu Ala Gly Pro Arg Met Pro Arg Trp Glu Ile
        35                  40                  45

Val Glu Arg Phe Arg Asn Gln Val Asp Lys Thr Asp Phe Met Ala Pro
    50                  55                  60

Tyr Pro Ser Ser Ala Trp Ala Pro His Pro Glu Tyr Gly Pro Pro Asn
65                  70                  75                  80

Asp Tyr Leu Ile Leu Lys Gly Glu His Lys Phe Asn Ser Gln Tyr Ile
                85                  90                  95

Arg Ala Val Gly Gly Thr Thr Trp His Trp Ala Ala Ser Ala Trp Arg
            100                 105                 110

Phe Ile Pro Asn Asp Phe Lys Met Lys Thr Val Tyr Gly Val Gly Arg
        115                 120                 125

Asp Trp Pro Ile Gln Tyr Asp Asp Ile Glu His Tyr Tyr Gln Arg Ala
    130                 135                 140

Glu Glu Glu Leu Gly Val Trp Gly Pro Gly Pro Glu Glu Asp Leu Tyr
145                 150                 155                 160

Ser Pro Arg Lys Glu Pro Tyr Pro Met Pro Pro Leu Pro Leu Ser Phe
                165                 170                 175

Asn Glu Gln Thr Ile Lys Ser Ala Leu Asn Gly Tyr Asp Pro Lys Phe
            180                 185                 190

His Val Val Thr Glu Pro Val Ala Arg Asn Ser Arg Pro Tyr Asp Gly
        195                 200                 205

Arg Pro Thr Cys Cys Gly Asn Asn Asn Cys Met Pro Ile Cys Pro Ile
    210                 215                 220

Gly Ala Met Tyr Asn Gly Ile Val His Val Glu Lys Ala Glu Gln Ala
225                 230                 235                 240

Gly Ala Lys Leu Ile Asp Ser Ala Val Val Tyr Lys Leu Glu Thr Gly
```

245 250 255

Pro Asp Lys Arg Ile Thr Ala Ala Val Tyr Lys Asp Lys Thr Gly Ala
260 265 270

Asp His Arg Val Glu Gly Lys Tyr Phe Val Ile Ala Ala Asn Gly Ile
275 280 285

Glu Thr Pro Lys Ile Leu Leu Met Ser Ala Asn Arg Asp Phe Pro Asn
290 295 300

Gly Val Ala Asn Ser Ser Asp Met Val Gly Arg Asn Leu Met Asp His
305 310 315 320

Pro Gly Thr Gly Val Ser Phe Tyr Ala Asn Glu Lys Leu Trp Pro Gly
325 330 335

Arg Gly Pro Gln Glu Met Thr Ser Leu Ile Gly Phe Arg Asp Gly Pro
340 345 350

Phe Arg Ala Thr Glu Ala Ala Lys Lys Ile His Leu Ser Asn Met Ser
355 360 365

Arg Ile Asn Gln Glu Thr Gln Lys Ile Phe Lys Ala Gly Lys Leu Met
370 375 380

Lys Pro Glu Glu Leu Asp Ala Gln Ile Arg Asp Arg Ser Ala Arg Tyr
385 390 395 400

Val Gln Phe Asp Cys Phe His Glu Ile Leu Pro Gln Pro Glu Asn Arg
405 410 415

Ile Val Pro Ser Lys Thr Ala Thr Asp Ala Ile Gly Ile Pro Arg Pro
420 425 430

Glu Ile Thr Tyr Ala Ile Asp Asp Tyr Val Lys Arg Gly Ala Val His
435 440 445

Thr Arg Glu Val Tyr Ala Thr Ala Ala Lys Val Leu Gly Gly Thr Glu
450 455 460

Val Val Phe Asn Asp Glu Phe Ala Pro Asn Asn His Ile Thr Gly Ala
465 470 475 480

Thr Ile Met Gly Ala Asp Ala Arg Asp Ser Val Val Asp Lys Asp Cys
485 490 495

Arg Thr Phe Asp His Pro Asn Leu Phe Ile Ser Ser Ser Ser Thr Met
500 505 510

Pro Thr Val Gly Thr Val Asn Val Thr Leu Thr Ile Ala Ala Leu Ala
515 520 525

Leu Arg Met Ser Asp Thr Leu Lys Lys Glu Val
530 535

<210> 13
<211> 539
<212> PRT
<213> Burkholderia cepacia

<400> 13

Met Ala Asp Thr Asp Thr Gln Lys Ala Asp Val Val Val Val Gly Ser
1 5 10 15

Gly Val Ala Gly Ala Ile Val Ala His Gln Leu Ala Met Ala Gly Lys
20 25 30

Ser Val Ile Leu Leu Glu Ala Gly Pro Arg Met Pro Arg Trp Glu Ile
35 40 45

Val Glu Arg Phe Arg Asn Gln Val Asp Lys Thr Asp Phe Met Ala Pro
50 55 60

Tyr Pro Ser Ser Ala Trp Ala Pro His Pro Glu Tyr Gly Pro Pro Asn
65 70 75 80

Asp Tyr Leu Ile Leu Lys Gly Glu His Lys Phe Asn Ser Gln Tyr Ile
85 90 95

Arg Ala Val Gly Gly Thr Thr Trp His Trp Ala Ala Ser Ala Trp Arg
100 105 110

Phe Ile Pro Asn Asp Phe Lys Met Lys Thr Val Tyr Gly Val Gly Arg
115 120 125

Asp Trp Pro Ile Gln Tyr Asp Asp Ile Glu His Tyr Tyr Gln Arg Ala
130 135 140

Glu Glu Glu Leu Gly Val Trp Gly Pro Gly Pro Glu Glu Asp Leu Tyr
145 150 155 160

Ser Pro Arg Lys Glu Pro Tyr Pro Met Pro Pro Leu Pro Leu Ser Phe
165 170 175

Asn Glu Gln Thr Ile Lys Ser Ala Leu Asn Gly Tyr Asp Pro Lys Phe
180 185 190

His Val Val Thr Glu Pro Val Ala Arg Asn Ser Arg Pro Tyr Asp Gly
195 200 205

Arg Pro Thr Cys Cys Gly Asn Asn Asn Cys Met Pro Ile Cys Pro Ile
210 215 220

Gly Ala Met Tyr Asn Gly Ile Val His Val Glu Lys Ala Glu Gln Ala
225 230 235 240

Gly Ala Lys Leu Ile Asp Ser Ala Val Val Tyr Lys Leu Glu Thr Gly
245 250 255

Pro Asp Lys Arg Ile Thr Ala Ala Val Tyr Lys Asp Lys Thr Gly Ala
260 265 270

Asp His Arg Val Glu Gly Lys Tyr Phe Val Ile Ala Ala Asn Gly Ile
275 280 285

Glu Thr Pro Lys Ile Leu Leu Met Ser Ala Asn Arg Asp Phe Pro Asn
290 295 300

Gly Val Ala Asn Ser Ser Asp Met Val Gly Arg Asn Leu Met Asp His
305 310 315 320

Pro Gly Thr Gly Val Ser Phe Tyr Ala Asn Glu Lys Leu Trp Pro Gly
325 330 335

Arg Gly Pro Gln Glu Met Thr Ser Leu Ile Gly Phe Arg Asp Gly Pro
340 345 350

Phe Arg Ala Asn Glu Ala Ala Lys Lys Ile His Leu Ser Asn Met Ser
355 360 365

Arg Ile Asn Gln Glu Thr Gln Lys Ile Phe Lys Gly Gly Lys Leu Met
370 375 380

Lys Pro Glu Glu Leu Asp Ala Gln Ile Arg Asp Arg Ser Ala Arg Phe
385 390 395 400

Val Gln Phe Asp Cys Phe His Glu Ile Leu Pro Gln Pro Glu Asn Arg
405 410 415

Ile Val Pro Ser Lys Thr Ala Thr Asp Ala Val Gly Ile Pro Arg Pro
420 425 430

Glu Ile Thr Tyr Ala Ile Asp Asp Tyr Val Lys Arg Gly Ala Val His
435 440 445

Thr Arg Glu Val Tyr Ala Thr Ala Ala Lys Val Leu Gly Gly Thr Glu

450 455 460

Val Val Phe Asn Asp Glu Phe Ala Pro Asn Asn His Ile Thr Gly Ala
465 470 475 480

Thr Ile Met Gly Ala Asp Ala Arg Asp Ser Val Val Asp Lys Asp Cys
485 490 495

Arg Thr Phe Asp His Pro Asn Leu Phe Ile Ser Ser Ser Ser Thr Met
500 505 510

Pro Thr Val Gly Thr Val Asn Val Thr Leu Thr Ile Ala Ala Leu Ala
515 520 525

Leu Arg Met Ser Asp Thr Leu Lys Lys Glu Val
530 535

<210> 14
<211> 539
<212> PRT
<213> Burkholderia cepacia

<400> 14

```
Met Ala Asp Thr Asp Thr Gln Lys Ala Asp Val Val Val Val Gly Ser
1               5                   10                  15

Gly Val Ala Gly Ala Ile Val Ala His Gln Leu Ala Met Ala Gly Lys
            20                  25                  30

Ser Val Ile Leu Leu Glu Ala Gly Pro Arg Met Pro Arg Trp Glu Ile
        35                  40                  45

Val Glu Arg Phe Arg Asn Gln Thr Asp Lys Thr Asp Phe Met Ala Pro
    50                  55                  60

Tyr Pro Ser Ser Pro Trp Ala Pro His Pro Glu Tyr Gly Pro Pro Asn
65                  70                  75                  80

Asp Tyr Leu Val Leu Lys Gly Glu His Lys Phe Asn Ser Gln Tyr Ile
                85                  90                  95

Arg Ala Val Gly Gly Thr Thr Trp His Trp Ala Ala Ser Ala Trp Arg
            100                 105                 110

Phe Ile Pro Asn Asp Phe Lys Met Lys Thr Val Tyr Gly Val Gly Arg
        115                 120                 125

Asp Trp Pro Ile Gln Tyr Asp Asp Leu Glu His Tyr Tyr Gln Arg Ala
    130                 135                 140
```

Glu Glu Glu Leu Gly Val Trp Gly Pro Gly Pro Glu Glu Asp Leu Tyr
145 150 155 160

Ser Pro Arg Arg Gln Pro Tyr Pro Met Pro Pro Leu Pro Leu Ser Phe
165 170 175

Asn Glu Gln Thr Ile Lys Ser Ala Leu Asn Gly Tyr Asp Pro Lys Phe
180 185 190

His Val Val Thr Glu Pro Val Ala Arg Asn Ser Arg Pro Tyr Asp Gly
195 200 205

Arg Pro Thr Cys Cys Gly Asn Asn Asn Cys Met Pro Ile Cys Pro Ile
210 215 220

Gly Ala Met Tyr Asn Gly Ile Val His Val Glu Lys Ala Glu Gln Ala
225 230 235 240

Gly Ala Lys Leu Ile Glu Asn Ala Val Val Tyr Lys Leu Glu Thr Gly
245 250 255

Pro Asn Lys Arg Ile Val Ala Ala Ile Tyr Lys Asp Lys Ser Gly Ala
260 265 270

Asp His Arg Val Glu Gly Lys Tyr Phe Val Val Ala Ala Asn Gly Ile
275 280 285

Glu Thr Pro Lys Ile Leu Leu Met Ser Ala Asn Arg Asp Phe Pro Asn
290 295 300

Gly Val Ala Asn Ser Ser Asp Met Val Gly Arg Asn Leu Met Asp His
305 310 315 320

Pro Gly Thr Gly Val Ser Phe Tyr Ala Asn Glu Lys Leu Trp Pro Gly
325 330 335

Arg Gly Pro Gln Glu Met Thr Ser Leu Ile Gly Phe Arg Asp Gly Pro
340 345 350

Phe Arg Ala Thr Glu Ala Ala Lys Lys Ile His Leu Ser Asn Met Ser
355 360 365

Arg Ile Asn Gln Glu Thr Gln Lys Ile Phe Lys Gly Gly Lys Leu Met
370 375 380

Lys Pro Glu Glu Leu Asp Ala Gln Ile Arg Asp Arg Ser Ala Arg Tyr
385 390 395 400

Val Gln Phe Asp Cys Phe His Glu Ile Leu Pro Gln Pro Glu Asn Arg
405 410 415

Ile Val Pro Ser Lys Thr Ala Thr Asp Ala Ile Gly Ile Pro Arg Pro
420 425 430

Glu Ile Thr Tyr Ala Ile Asp Asp Tyr Val Lys Arg Gly Ala Val His
435 440 445

Thr Cys Glu Val Tyr Ala Thr Ala Ala Lys Val Leu Gly Gly Thr Glu
450 455 460

Val Val Phe Asn Asp Glu Phe Ala Pro Asn Asn His Ile Thr Gly Ala
465 470 475 480

Thr Ile Met Gly Ala Asp Ala Arg Asp Ser Val Val Asp Lys Asp Cys
485 490 495

Arg Thr Phe Asp His Pro Asn Leu Phe Ile Ser Ser Ser Ser Thr Met
500 505 510

Pro Thr Val Gly Thr Val Asn Val Thr Leu Thr Ile Ala Ala Leu Ala
515 520 525

Leu Arg Met Ser Asp Thr Leu Lys Lys Glu Val
530 535

<210> 15
<211> 539
<212> PRT
<213> Burkholderia cepacia

<400> 15

Met Ala Asp Thr Asp Thr Gln Lys Ala Asp Ile Val Val Val Gly Ser
1 5 10 15

Gly Val Ala Gly Ala Ile Val Ala His Gln Leu Ala Met Ala Gly Lys
20 25 30

Ser Val Ile Leu Leu Glu Ala Gly Pro Arg Met Pro Arg Trp Glu Ile
35 40 45

Val Glu Arg Phe Arg Asn Gln Pro Asp Lys Thr Asp Phe Met Ala Pro
50 55 60

Tyr Pro Ser Ser Pro Trp Ala Pro His Pro Glu Tyr Gly Pro Pro Asn
65 70 75 80

Asp Tyr Leu Ile Leu Lys Gly Glu His Lys Phe Asn Ser Gln Tyr Ile
85 90 95

Arg Ala Val Gly Gly Thr Thr Trp His Trp Ala Ala Ser Ala Trp Arg
100 105 110

Phe Ile Pro Asn Asp Phe Lys Met Lys Thr Val Tyr Gly Val Ala Arg
115 120 125

Asp Trp Pro Ile Gln Tyr Asp Asp Leu Glu His Trp Tyr Gln Arg Ala
130 135 140

Glu Glu Glu Leu Gly Val Trp Gly Pro Gly Pro Glu Glu Asp Leu Tyr
145 150 155 160

Ser Pro Arg Lys Gln Ala Tyr Pro Met Pro Pro Leu Pro Leu Ser Phe
165 170 175

Asn Glu Gln Thr Ile Lys Ser Ala Leu Asn Gly Tyr Asp Pro Lys Phe
180 185 190

His Val Val Thr Glu Pro Val Ala Arg Asn Ser Arg Pro Tyr Asp Gly
195 200 205

Arg Pro Thr Cys Cys Gly Asn Asn Asn Cys Met Pro Ile Cys Pro Ile
210 215 220

Gly Ala Met Tyr Asn Gly Ile Val His Val Glu Lys Ala Glu Gln Ala
225 230 235 240

Gly Ala Lys Leu Ile Asp Ser Ala Val Val Tyr Lys Leu Glu Thr Gly
245 250 255

Pro Asp Lys Arg Ile Val Ala Ala Ile Tyr Lys Asp Lys Thr Gly Ala
260 265 270

Asp His Arg Val Glu Gly Lys Tyr Phe Val Leu Ala Ala Asn Gly Ile
275 280 285

Glu Thr Pro Lys Ile Leu Leu Met Ser Ala Asn Arg Asp Phe Pro Asn
290 295 300

Gly Val Ala Asn Ser Ser Asp Met Val Gly Arg Asn Leu Met Asp His
305 310 315 320

Pro Gly Thr Gly Val Ser Phe Tyr Ala Asn Glu Lys Leu Trp Pro Gly
325 330 335

Arg Gly Pro Gln Glu Met Thr Ser Leu Ile Gly Phe Arg Asp Gly Pro
340 345 350

Phe Arg Ala Thr Glu Ala Ala Lys Lys Ile His Leu Ser Asn Met Ser
355 360 365

Arg Ile Asn Gln Glu Thr Gln Lys Ile Phe Lys Ala Gly Lys Leu Met
370 375 380

Lys His Glu Glu Leu Asp Ala Gln Ile Arg Asp Arg Ser Ala Arg Tyr
385 390 395 400

Val Gln Phe Asp Cys Phe His Glu Ile Leu Pro Gln Pro Glu Asn Arg
405 410 415

Ile Val Pro Ser Lys Thr Ala Thr Asp Ala Ile Gly Ile Pro Arg Pro
420 425 430

Glu Ile Thr Tyr Ala Ile Asp Asp Tyr Val Lys Arg Gly Ala Val His
435 440 445

Thr Arg Glu Val Tyr Ala Thr Ala Ala Lys Val Leu Gly Gly Thr Asp
450 455 460

Val Val Phe Asn Asp Glu Phe Ala Pro Asn Asn His Ile Thr Gly Ala
465 470 475 480

Thr Ile Met Gly Ala Asp Ala Arg Asp Ser Val Val Asp Lys Asp Cys
485 490 495

Arg Thr Phe Asp His Pro Asn Leu Phe Ile Ser Ser Ser Ser Thr Met
500 505 510

Pro Thr Val Gly Thr Val Asn Val Thr Leu Thr Ile Ala Ala Leu Ala
515 520 525

Leu Arg Met Ser Asp Thr Leu Lys Lys Glu Val
530 535

<210> 16
<211> 539
<212> PRT
<213> Burkholderia cepacia

<400> 16

Met Ala Asp Thr Asp Thr Gln Lys Ala Asp Ile Val Val Val Gly Ser
1               5                   10                  15

Gly Val Ala Gly Ala Ile Val Ala His Gln Leu Ala Met Ala Gly Lys
            20                  25                  30

Ser Val Ile Leu Leu Glu Ala Gly Pro Arg Met Pro Arg Trp Glu Ile

35 40 45

Val Glu Arg Phe Arg Asn Gln Pro Asp Lys Thr Asp Phe Met Ala Pro
50 55 60

Tyr Pro Ser Ser Pro Trp Ala Pro His Pro Glu Tyr Gly Pro Pro Asn
65 70 75 80

Asp Tyr Leu Ile Leu Lys Gly Glu His Lys Phe Asn Ser Gln Tyr Ile
85 90 95

Arg Ala Val Gly Gly Thr Thr Trp His Trp Ala Ala Ser Ala Trp Arg
100 105 110

Phe Ile Pro Asn Asp Phe Lys Met Lys Thr Val Tyr Gly Val Ala Arg
115 120 125

Asp Trp Pro Ile Gln Tyr Asp Asp Leu Glu His Trp Tyr Gln Arg Ala
130 135 140

Glu Glu Glu Leu Gly Val Trp Gly Pro Gly Pro Glu Glu Asp Leu Tyr
145 150 155 160

Ser Pro Arg Lys Gln Ala Tyr Pro Met Pro Pro Leu Pro Leu Ser Phe
165 170 175

Asn Glu Gln Thr Ile Lys Ser Ala Leu Asn Gly Tyr Asp Pro Lys Phe
180 185 190

His Val Val Thr Glu Pro Val Ala Arg Asn Ser Arg Pro Tyr Asp Gly
195 200 205

Arg Pro Thr Cys Cys Gly Asn Asn Asn Cys Met Pro Ile Cys Pro Ile
210 215 220

Gly Ala Met Tyr Asn Gly Ile Val His Val Glu Lys Ala Glu Gln Ala
225 230 235 240

Gly Ala Lys Leu Ile Asp Ser Ala Val Val Tyr Lys Leu Glu Thr Gly
245 250 255

Pro Asp Lys Arg Ile Val Ala Ala Ile Tyr Lys Asp Lys Thr Gly Ala
260 265 270

Asp His Arg Val Glu Gly Lys Tyr Phe Val Leu Ala Ala Asn Gly Ile
275 280 285

Glu Thr Pro Lys Ile Leu Leu Met Ser Ala Asn Arg Asp Phe Pro Asn
290 295 300

Gly Val Ala Asn Ser Ser Asp Met Val Gly Arg Asn Leu Met Asp His
305 310 315 320

Pro Gly Thr Gly Val Ser Phe Tyr Ala Asn Glu Lys Leu Trp Pro Gly
325 330 335

Arg Gly Pro Gln Glu Met Thr Ser Leu Ile Gly Phe Arg Asp Gly Pro
340 345 350

Phe Arg Ala Thr Glu Ala Ala Lys Lys Ile His Leu Ser Asn Met Ser
355 360 365

Arg Ile Asn Gln Glu Thr Gln Lys Ile Phe Lys Ala Gly Lys Leu Met
370 375 380

Lys His Glu Glu Leu Asp Ala Gln Ile Arg Asp Arg Ser Ala Arg Tyr
385 390 395 400

Val Gln Phe Asp Cys Phe His Glu Ile Leu Pro Gln Pro Glu Asn Arg
405 410 415

Ile Val Pro Ser Lys Thr Ala Thr Asp Ala Ile Gly Ile Pro Arg Pro
420 425 430

Glu Ile Thr Tyr Ala Ile Asp Asp Tyr Val Lys Arg Gly Ala Val His
435 440 445

Thr Arg Glu Val Tyr Ala Thr Ala Ala Lys Val Leu Gly Gly Thr Asp
450 455 460

Val Val Phe Asn Asp Glu Phe Ala Pro Asn Asn His Ile Thr Gly Ala
465 470 475 480

Thr Ile Met Gly Ala Asp Ala Arg Asp Ser Val Val Asp Lys Asp Cys
485 490 495

Arg Thr Phe Asp His Pro Asn Leu Phe Ile Ser Ser Ser Ser Thr Met
500 505 510

Pro Thr Val Gly Thr Val Asn Val Thr Leu Thr Ile Ala Ala Leu Ala
515 520 525

Leu Arg Met Ser Asp Thr Leu Lys Lys Glu Val
530 535

<210> 17
<211> 40
<212> DNA

<213> artificial sequence

<220>
<223> primer

<400> 17
accaccactg ataaggaggt ctgaccgtgc ggaaatctac 40

<210> 18
<211> 40
<212> DNA
<213> artificial sequence

<220>
<223> primer

<400> 18
agcctgtgcg acttcttcct tcagcgatcg gtggtggtgg 40

<210> 19
<211> 26
<212> DNA
<213> artificial sequence

<220>
<223> primer

<400> 19
catgccatgg cacacaacga caacac 26

<210> 20
<211> 30
<212> DNA
<213> artificial sequence

<220>
<223> primer

<400> 20
gtcgacgatc ttcttccagc cgaacatcac 30

<210> 21
<211> 30
<212> DNA
<213> artificial sequence

<220>
<223> primer

<400> 21
gaattctatg cgagcgagaa gctgtggccg 30

<210> 22
<211> 30
<212> DNA
<213> artificial sequence

<220>

<223> primer

<400> 22
ttcttctatg cgagcgagaa gctgtggccg 30

<210> 23
<211> 30
<212> DNA
<213> artificial sequence

<220>
<223> primer

<400> 23
atcttctatg cgagcgagaa gctgtggccg 30

<210> 24
<211> 30
<212> DNA
<213> artificial sequence

<220>
<223> primer

<400> 24
aaattctatg cgagcgagaa gctgtggccg 30

<210> 25
<211> 30
<212> DNA
<213> artificial sequence

<220>
<223> primer

<400> 25
ctgttctatg cgagcgagaa gctgtggccg 30

<210> 26
<211> 30
<212> DNA
<213> artificial sequence

<220>
<223> primer

<400> 26
aacttctatg cgagcgagaa gctgtggccg 30

<210> 27
<211> 30
<212> DNA
<213> artificial sequence

<220>
<223> primer

<400> 27

cagttctatg cgagcgagaa gctgtggccg 30

<210> 28
<211> 30
<212> DNA
<213> artificial sequence

<220>
<223> primer

<400> 28
cgcttctatg cgagcgagaa gctgtggccg 30

<210> 29
<211> 30
<212> DNA
<213> artificial sequence

<220>
<223> primer

<400> 29
accttctatg cgagcgagaa gctgtggccg 30

<210> 30
<211> 30
<212> DNA
<213> artificial sequence

<220>
<223> primer

<400> 30
gttttctatg cgagcgagaa gctgtggccg 30

<210> 31
<211> 30
<212> DNA
<213> artificial sequence

<220>
<223> primer

<400> 31
tggttctatg cgagcgagaa gctgtggccg 30

<210> 32
<211> 30
<212> DNA
<213> artificial sequence

<220>
<223> primer

<400> 32
tacttctatg cgagcgagaa gctgtggccg 30

<210> 33

<211> 30
<212> DNA
<213> artificial sequence

<220>
<223> primer

<400> 33
cacgccggtc ccgggatggt ccatcaggtt 30

<210> 34
<211> 30
<212> DNA
<213> artificial sequence

<220>
<223> primer

<400> 34
cacgccggtg cccggatggt ccatcaggtt 30

<210> 35
<211> 30
<212> DNA
<213> artificial sequence

<220>
<223> primer

<400> 35
gacaacctgt cgcgcatcga ccaggagacg 30

<210> 36
<211> 30
<212> DNA
<213> artificial sequence

<220>
<223> primer

<400> 36
ttcaacctgt cgcgcatcga ccaggagacg 30

<210> 37
<211> 30
<212> DNA
<213> artificial sequence

<220>
<223> primer

<400> 37
atcaacctgt cgcgcatcga ccaggagacg 30

<210> 38
<211> 30
<212> DNA
<213> artificial sequence

<220>
<223> primer

<400> 38
aaaaacctgt cgcgcatcga ccaggagacg 30

<210> 39
<211> 30
<212> DNA
<213> artificial sequence

<220>
<223> primer

<400> 39
cagaacctgt cgcgcatcga ccaggagacg 30

<210> 40
<211> 30
<212> DNA
<213> artificial sequence

<220>
<223> primer

<400> 40
cgtaacctgt cgcgcatcga ccaggagacg 30

<210> 41
<211> 30
<212> DNA
<213> artificial sequence

<220>
<223> primer

<400> 41
accaacctgt cgcgcatcga ccaggagacg 30

<210> 42
<211> 30
<212> DNA
<213> artificial sequence

<220>
<223> primer

<400> 42
tacaacctgt cgcgcatcga ccaggagacg 30

<210> 43
<211> 30
<212> DNA
<213> artificial sequence

<220>
<223> primer

<400> 43
caggtggatc ttcttcgccg cttcggtcgc 30

<210> 44
<211> 30
<212> DNA
<213> artificial sequence

<220>
<223> primer

<400> 44
gcgccgaaca atcacatcac gggctcgacg 30

<210> 45
<211> 30
<212> DNA
<213> artificial sequence

<220>
<223> primer

<400> 45
gacccgaaca atcacatcac gggctcgacg 30

<210> 46
<211> 30
<212> DNA
<213> artificial sequence

<220>
<223> primer

<400> 46
gaaccgaaca atcacatcac gggctcgacg 30

<210> 47
<211> 30
<212> DNA
<213> artificial sequence

<220>
<223> primer

<400> 47
ttcccgaaca atcacatcac gggctcgacg 30

<210> 48
<211> 30
<212> DNA
<213> artificial sequence

<220>
<223> primer

<400> 48
catccgaaca atcacatcac gggctcgacg 30

<210> 49
<211> 30
<212> DNA
<213> artificial sequence

<220>
<223> primer

<400> 49
atcccgaaca atcacatcac gggctcgacg 30

<210> 50
<211> 30
<212> DNA
<213> artificial sequence

<220>
<223> primer

<400> 50
aaaccgaaca atcacatcac gggctcgacg 30

<210> 51
<211> 30
<212> DNA
<213> artificial sequence

<220>
<223> primer

<400> 51
aacccgaaca atcacatcac gggctcgacg 30

<210> 52
<211> 30
<212> DNA
<213> artificial sequence

<220>
<223> primer

<400> 52
cgtccgaaca atcacatcac gggctcgacg 30

<210> 53
<211> 30
<212> DNA
<213> artificial sequence

<220>
<223> primer

<400> 53
agtccgaaca atcacatcac gggctcgacg 30

<210> 54
<211> 30
<212> DNA

<213> artificial sequence

<220>
<223> primer

<400> 54
actccgaaca atcacatcac gggctcgacg 30

<210> 55
<211> 30 <212> DNA
<213> artificial sequence

<220>
<223> primer

<400> 55
tacccgaaca atcacatcac gggctcgacg 30

<210> 56
<211> 30
<212> DNA
<213> artificial sequence

<220>
<223> primer

<400> 56
gaattcgtcg ttgaacacga cgtccgtgcc 30

<210> 57
<211> 30
<212> DNA
<213> artificial sequence

<220>
<223> primer

<400> 57
gaactcgtcg ttgaacacga cgtccgtgcc 30

<210> 58
<211> 31
<212> DNA
<213> Artificial Sequence

<220>
<223> primer

<400> 58
gggcaccggc gtgcagttct atgcgaacga g 31

<210> 59
<211> 37
<212> DNA
<213> Artificial Sequence

<220>
<223> primer

<400> 59
gaagaagatc cacctgtaca acatgtcgcg catcaac 37

<210> 60
<211> 44
<212> DNA
<213> Artificial Sequence

<220>
<223> primer

<400> 60
gtcgtgttca acgacgaatt ctacccgaac aatcacatca cggg 44

<210> 61
<211> 31
<212> DNA
<213> Artificial Sequence

<220>
<223> primer

<400> 61
ctcgttcgca tagaactgca cgccggtgcc c 31

<210> 62
<211> 37
<212> DNA
<213> Artificial Sequence

<220>
<223> primer

<400> 62
gttgatgcgc gacatgttgt acaggtggat cttcttc 37

<210> 63
<211> 44
<212> DNA
<213> Artificial Sequence

<220>
<223> primer

<400> 63
cccgtgatgt gattgttcgg gtagaattcg tcgttgaaca cgac 44

<210> 64
<211> 31
<212> DNA
<213> Artificial Sequence

<220>
<223> primer

<400> 64
gggcaccggc gtgcagttct atgcgagcga g 31

<210> 65
<211> 37
<212> DNA
<213> Artificial Sequence

<220>
<223> primer

<400> 65
gaagaagatc cacctgtaca acctgtcgcg catcgac 37

<210> 66
<211> 31
<212> DNA
<213> Artificial Sequence

<220>
<223> primer

<400> 66
ctcgctcgca tagaactgca cgccggtgcc c 31

<210> 67
<211> 37
<212> DNA
<213> Artificial Sequence

<220>
<223> primer

<400> 67
gtcgatgcgc gacaggttgt acaggtggat cttcttc 37

<210> 68
<211> 31
<212> DNA
<213> Artificial Sequence

<220>
<223> primer

<400> 68
gggcaccggc gtgcagttct atgcggaccg c 31

<210> 69
<211> 37
<212> DNA
<213> Artificial Sequence

<220>
<223> primer

<400> 69
caagaagctg cacctgtaca acatctcgcg catcgag 37

<210> 70
<211> 44
<212> DNA

<213> Artificial Sequence

<220>
<223> primer

<400> 70
gtcgagttcc acgacgactt ctacccgaac aatcacatca cggg 44

<210> 71
<211> 31
<212> DNA
<213> Artificial Sequence

<220>
<223> primer

<400> 71
gcggtccgca tagaactgca cgccggtgcc c 31

<210> 72
<211> 37
<212> DNA
<213> Artificial Sequence

<220>
<223> primer

<400> 72
ctcgatgcgc gagatgttgt acaggtgcag cttcttg 37

<210> 73
<211> 44
<212> DNA
<213> Artificial Sequence

<220>
<223> primer

<400> 73
cccgtgatgt gattgttcgg gtagaagtcg tcgtggaact cgac 44

<210> 74
<211> 44
<212> DNA
<213> Artificial Sequence

<220>
<223> primer

<400> 74
gtccagttcc acgacgactt ctacccgaac aatcacatca cggg 44

<210> 75
<211> 44
<212> DNA
<213> Artificial Sequence

<220>

<223> primer

<400> 75
cccgtgatgt gattgttcgg gtagaagtcg tcgtggaact ggac 44

<210> 76
<211> 31
<212> DNA
<213> Artificial Sequence

<220>
<223> primer

<400> 76
gggcaccggc gtgcagttcc tggcgaacga g 31

<210> 77
<211> 37
<212> DNA
<213> Artificial Sequence

<220>
<223> primer

<400> 77
gaagaagctg cacctgtaca acggcgtccc gacgatg 37

<210> 78
<211> 44
<212> DNA
<213> Artificial Sequence

<220>
<223> primer

<400> 78
gtcacgttcg acgacacgtt ctacccgaac aatcacatca tggg 44

<210> 79
<211> 31
<212> DNA
<213> Artificial Sequence

<220>
<223> primer

<400> 79
ctcgttcgcc aggaactgca cgccggtgcc c 31

<210> 80
<211> 37
<212> DNA
<213> Artificial Sequence

<220>
<223> primer

<400> 80

catcgtcggg acgccgttgt acaggtgcag cttcttc 37

<210> 81
<211> 44
<212> DNA
<213> Artificial Sequence

<220>
<223> primer

<400> 81
cccatgatgt gattgttcgg gtagaacgtg tcgtcgaacg tgac 44

**Description**

TECHNICAL FIELD

The present invention relates to a mutant glucose dehydrogenase showing an improved substrate specificity. Specifically, the present invention relates to a glucose dehydrogenase comprising a mutant-type α-subunit which is produced by introducing mutations into amino acid residues of its α-subunit that constitutes a cytochrome C-containing glucose dehydrogenase (hereinafter also referred to as CyGDH), and relates to a gene thereof. The glucose dehydrogenases of the present invention can be suitably used for glucose sensors, glucose assay kits and so forth, and are useful in the fields of biochemistry, clinical medicine and so forth.

BACKGROUND ART

At present, a wild-type CyGDH, a PQQGDH using pyrroloquinoline quinone as a coenzyme, or the like is used for self-monitoring blood glucose sensors. The wild-type CyGDH and PQQGDH have a drawback in that they are incapable of accurately measuring the blood sugar level in cases where the blood maltose level of patients is high, because they react not only with glucose but also with maltose. Especially in Japan and the United Kingdom, maltose is used as an energy material in infusion solutions, and, in fact, there have been some accidents caused by the wrong measurement results wherein patients who received administration of such infusion solution by peritoneal dialysis or the like and whose blood sugar level was low were mistaken for high blood sugar level due to a sensor that uses PQQGDH for measuring the blood sugar level.

In wild-type CyGDHs, the reactivities to maltose are high compared to those to glucose, and therefore, even if the glucose concentration is 50 mg/dL, the measurement results of the blood sugar level in the case where the maltose concentration is 100 mg/dL will indicate a value which is higher by 170%.

In view of these circumstances, a mutant enzyme of CyGDH (a mutant glucose dehydrogenase that is obtained by introducing mutations into 326th and 365th amino acid residues of its α-subunit so that the 326th and 365th amino acid residues are replaced with a glutamine (Q) and a tyrosine (Y), respectively) (hereinafter referred to as CyGDH (QY) or also referred to as simply QY or QYA) has been devised (WO2006/137283). According to this invention, the apparent increase of the blood sugar level can be suppressed so that, when the glucose concentration is 50 mg/dL, the measurement results for the blood sugar level in the case where the maltose concentration is 100 mg/dL will indicate a value which is higher by up to 36%. However, the effect to avoid the influence of maltose was not perfect.

Further mutants of CyGDHs (including mutant glucose dehydrogenases that are obtained by introducing mutations into the 472$^{nd}$ amino acid residue) have also been disclosed (WO2005/103248).

SUMMARY OF THE INVENTION

An object of the present invention is to provide a CyGDH showing improved substrate specificity to glucose as compared to the CyGDH (QY).

The present inventors intensively studied the above-described object and discovered that, compared to the CyGDH (QY), the substrate specificities are further improved by replacing the sites corresponding to positions 326 and 365 of amino acid residues of its α-subunit constituting the CyGDH with glutamine and tyrosine, respectively, and further replacing the site corresponding to position 472 with tyrosine, and that, also in GDH homologues, a similar effect can be obtained by replacing the sites corresponding to positions 326, 365 and 472 with glutamine, tyrosine and tyrosine, respectively, thereby completing the present invention.

That is to say, the present invention is as follows.

(1) A mutant glucose dehydrogenase having an amino acid sequence at least 80% identical to SEQ ID NO:3 and having glucose dehydrogenase activity,
wherein amino acid residues in said mutant glucose dehydrogenase corresponding to positions 326, 365 and 472 of SEQ ID NO:3 are replaced with glutamine, tyrosine and tyrosine, respectively, and
wherein said mutant glucose dehydrogenase shows an improved substrate specificity to glucose and a reduced reactivity to maltose relative to the glucose dehydrogenase with an amino acid sequence as set forth in SEQ ID NO:3, wherein said substrate specificity to glucose is improved by at least 20% and reactivity to maltose is 1% or less of the reactivity to glucose.

(2) The mutant glucose dehydrogenase according to (1), which has an amino acid sequence at least 90% identical to SEQ ID NO:3.

(3) The mutant glucose dehydrogenase according to (1) or (2), which has the amino acid sequence of SEQ ID NO:3 except for the positions corresponding to positions 326, 365 and 472.

(4) The mutant glucose dehydrogenase according to (1) or (2), which has the amino acid sequence of SEQ ID NO:7 except for the positions corresponding to positions 326, 365 and 472 of SEQ ID NO:3.

(5) The mutant glucose dehydrogenase according to (1) or (2), which has the amino acid sequence of SEQ ID NO:8 except for the positions corresponding to positions 326, 365 and 472 of SEQ ID NO:3.

(6) The mutant glucose dehydrogenase according to (1) or (2), which has the amino acid sequence of SEQ ID NO:9 except for the positions corresponding to positions 326, 365 and 472 of SEQ ID NO:3.

(7) The mutant glucose dehydrogenase according to (1) or (2), which has the amino acid sequence of SEQ ID NO: 10 except for the positions corresponding to positions 326, 365 and 472 of SEQ ID NO:3.

(8) The mutant glucose dehydrogenase according to (1) to (7), wherein said mutant glucose dehydrogenase shows substrate specificity to glucose improved by at least 20% and a reduced reactivity to maltose as compared to a glucose dehydrogenase with an amino acid sequence as set forth in SEQ ID NO:3 except that the amino acid residues in said glucose dehydrogenase corresponding to positions 326 and 365 of SEQ ID NO:3 are replaced with glutamine and tyrosine respectively.

(9) A mutant glucose dehydrogenase complex comprising at least the mutant glucose dehydrogenase according to (1) to (8) and an electron transfer subunit.

(10) The glucose dehydrogenase complex according to (9), wherein the electron transfer subunit is cytochrome C.

(11) A DNA molecule coding for the mutant glucose dehydrogenase according to (1) to (8).

(12) A microorganism comprising the DNA molecule according to (11) and producing the mutant glucose dehydrogenase according to (1) to (8) or the mutant glucose dehydrogenase complex according to (9) or (10).

(13) A glucose assay kit comprising the mutant glucose dehydrogenase according to (1) to (8), the mutant glucose dehydrogenase complex according to (9) or (10), or the microorganism according to (12).

(14) A glucose sensor comprising the mutant glucose dehydrogenase according to (1) to (8), the mutant glucose dehydrogenase complex according to (9) or (10), or the microorganism according to (12).

BRIEF DESCRIPTION OF THE DRAWINGS

Fig. 1 shows the structure of a glucose sensor.

Fig. 2 shows the reagent parts of a glucose sensor.

Fig. 3 shows the influence of the maltose concentrations (100 mg/dL, 200 mg/dL and 300 mg/dL) on the blood sugar level. The glucose concentration was 50 mg/dL and blood sugar levels were measured using a colorimetric sensor.

Fig. 4 shows the influence of the maltose concentrations (100 mg/dL, 200 mg/dL and 300 mg/dL) on the blood sugar level. The glucose concentration was 50 mg/dL and blood sugar levels were measured using an electrode sensor.

Fig. 5 shows the structure of an electrode glucose sensor.

MODE FOR CARRYING OUT THE INVENTION

Hereinafter, the present invention will be described in detail.

The mutant GDHs of the present invention can be produced by introducing specific mutations into an α-subunit of a wild-type GDH. Examples of the wild-type GDH include a GDH produced by *Burkholderia cepacia.* Examples of the GDH of *Burkholderia cepacia* include GDHs produced by *Burkholderia cepacia* KS1, JCM2800 and JCM2801 strain. The KS1 strain was deposited in the International Patent Organism Depositary, National Institute of Advanced Industrial Science and Technology (Tsukuba Central 6, 1-1-1 Higashi, Tsukuba, Ibaraki 305-8566, Japan) on September 25, 2000, under Accession No. FERM BP-7306.

The nucleotide sequence of a chromosomal DNA fragment that contains a GDH α-subunit gene, and a part of a GDH β-subunit gene of the KS1 strain is shown in SEQ ID NO:1 (U.S. Published Patent Application No. 2004/0023330). There are three open reading frames (ORFs) in this nucleotide sequence, and the second and third ORFs from the 5'-terminal of the sequence encode an α-subunit (SEQ ID NO:3) and a β-subunit (SEQ ID NO:4), respectively. The first ORF from the 5-terminal of the sequence is presumed to code for a γ-subunit (SEQ ID NO:2). Further, the nucleotide sequence of a fragment that contains a full-length β-subunit gene is shown in SEQ ID NO:5. Furthermore, the amino acid sequence of the β-subunit is shown in SEQ ID NO:6 (EP1498484A). The sequence of amino acids 1 to 22 in SEQ ID NO:6 is presumed to be a signal peptide.

Additionally, besides these, each α-subunit of a putative oxidoreductase of *Burkholderia cenocepacia* J2315 strain (SEQ ID NO:7), a hypothetical protein BthaT_07876 of *Burkholderia thailandensis* TXDOH strain (SEQ ID NO:8), a FAD dependent oxidoreductase of *Ralstonia pickettii* 12D strain (SEQ ID NO:9), a transmembrane dehydrogenase of *Ralstonia solanacearum* IPO1609 strain (SEQ ID NO:10) and a glucose-methanol-choline oxidoreductase of *Burkholderia phytofirmans* PsJN strain (SEQ ID NO:11), which are homologues of the GDH of *Burkholderia cepacia* KS1 strain, can be also used in the same manner as the GDH of *Burkholderia cepacia* KS1 strain.

All of the amino acid sequences as shown in SEQ ID NOs:7 to 11 have been registered in the NCBI (National Center for Biotechnology Information, the U.S.) database. The sequence of SEQ ID NO:7 has been registered under Accession No. YP_002234347; the sequence of SEQ ID NO:8 has been registered under Accession No. ZP_02370914; the sequence of SEQ ID NO:9 has been registered under Accession No. YP_002980762; the sequence of SEQ ID NO:10 has been registered under Accession No. YP_002260434; and the sequence of SEQ ID NO:1 has been registered under Accession No. YP_001890482.

The *Burkholderia cenocepacia* J2315 strain has been deposited as LMG 16656, ATCC BAA-245, CCM 4899, CCUG 48434 and NCTC 13227. The *Burkholderia phytofirmans* PsJN strain has been deposited as LMG 22487 and CCUG 49060.

In addition, each GDH α-subunit derived from other *Burkholderia cepacia* strains whose genus is the same as the *Burkholderia cepacia* KS1 strain, such as JCM2800, JCM2801, JCM5506, JCM5507 and IFO14595 (SEQ ID NOs:12 to 16), can be also used in the same manner as the GDH of *Burkholderia cepacia* KS1 strain. The JCM2800, JCM2801, JCM5506 and JCM5507 have been preserved in Japan Collection of Microorganisms (JCM), RIKEN. The IF014595 has been preserved in Institute for Fermentation, Osaka (IFO).

The mutant GDHs of the present invention may be an α-subunit alone, a complex of an α-subunit and a β-subunit, a complex of an α-subunit and a γ-subunit, or a complex consisting of an α-subunit, a β-subunit and a γ-subunit. In the present specification, a GDH complex containing a β-subunit is referred to as a CyGDH, and a GDH complex not containing any β-subunit is referred to as a GDH. All of the mutant GDHs of the present invention are mutants wherein specific mutations (mutations at positions 326, 365 and 472 of SEQ ID NO:3 or mutations at positions corresponding to these) are introduced into their α-subunits. However, the mutant GDHs of the present invention may have a conservative mutation(s) in addition to such specific mutations. In addition, other subunits may be wild-type and/or may have a conservative mutation(s). The term "a conservative mutation(s)" means a mutation(s) that does(do) not substantially affect GDH activity.

The mutant-type α-subunits of the present invention preferably have an amino acid sequence as shown in any one of SEQ ID NOs:3 and 7 to 11 except for the above-mentioned specific mutations. In addition, the mutant-type α-subunits may have a conservative mutation(s) as described above, as long as they have GDH activity. That is to say, they may be proteins which have an amino acid sequence including a substitution(s), a deletion(s), an insertion(s) and/or an addition(s) of one or more amino acid residues in the amino acid sequences of SEQ ID NOs:3 and 7 to 11 in addition to the above-described specific mutations. Although an amino acid sequence that can be coded for by the nucleotide sequence of SEQ ID NO: 1 is shown in SEQ ID NO:3, the methionine residue of the N-terminus maybe eliminated after translation. The term "one or more" as described above means preferably 1 to 10, more preferably 1 to 5, especially preferably 1 to 3. Further, the mutant-type α-subunits of the present invention have an amino acid identity of at least 80%, preferably 85%, more preferably 90%, to the amino acid sequence shown in SEQ ID NO:3.

In addition, the β-subunits typically have the amino acid sequence of SEQ ID NO:6. However, as long as they can function as a β-subunit of a CyGDH, they may be proteins which have an amino acid sequence including a substitution(s), a deletion(s), an insertion(s) and/or an addition(s) of one or more amino acid residues in the amino acid sequence consisting of amino acids 23 to 425 of SEQ ID NO:6. In addition, as long as they can function as a β-subunit of a CyGDH, they may be β-subunits of strains other than KS1 strain, and may be proteins which have an amino acid sequence including a substitution(s), a deletion(s), an insertion(s) and/or an addition(s) of one or more amino acid residues in the amino acid sequences of the said β-subunits of strains other than KS1 strain. The term "one or more" as described above means preferably 1 to 20, more preferably 1 to 10, especially preferably 1 to 5.

And, the wording "function as a β-subunit of a CyGDH" means that, when the β-subunit forms a complex together with an α-subunit, the β-subunit functions as an electron transfer subunit, namely, cytochrome C, without adversely affecting GDH activity of the said complex.

Specific examples of the wild-type α-subunit gene include a DNA which contains a nucleotide sequence consisting of nucleotides 764 to 2380 of SEQ ID NO:1. Additionally, the α-subunit gene may be a DNA which has a nucleotide sequence consisting of nucleotides 764 to 2380 of the nucleotide sequence of SEQ ID NO:1, or may be a DNA which hybridizes under stringent conditions with a probe that can be prepared from said nucleotide sequence consisting of nucleotides 764 to 2380 of the nucleotide sequence of SEQ ID NO:1, and which codes for a protein that has GDH activity.

Specific examples of the β-subunit gene include a DNA which contains a nucleotide sequence consisting of nucleotides 187 to 1398 of SEQ ID NO:5. Additionally, the β-subunit gene may be a DNA which has a nucleotide sequence consisting of nucleotides 187 to 1398 of SEQ ID NO:5, or may be a DNA which hybridizes under stringent conditions with a probe that can be prepared from said nucleotide sequence consisting of nucleotides 187 to 1398 of SEQ ID NO:5, and which codes for a protein that can function as a β-subunit.

Examples of the stringent conditions as mentioned above include conditions wherein DNAs having a homology (e.g. identity) of preferably 80% or more, more preferably 90% or more, especially preferably 95% or more, hybridize with each other, and more specifically, hybridize under conditions of 0.1 x SSC, 0.1% SDS and 60°C.

The α- and β-subunit genes can be obtained by PCR using chromosomal DNA of *Burkholderia cepacia* KS1 strain as a template, for example. Primers for the PCR can be prepared by chemically synthesizing on the basis of the above-described nucleotide sequence. Alternatively, they can also be obtained from the chromosomal DNA of *Burkholderia cepacia* KS1 strain by hybridization wherein oligonucleotides made on the basis of the above-described sequence are used as probes. Also, *Burkholderia cenocepacia* J2315 strain, *Burkholderia thailandensis* TXDOH strain, *Ralstonia pickettii* 12D strain, *Ralstonia solanacearum* IPO1609 strain and *Burkholderia phytofirmans* PsJN strain other than KS1 strain may be used.

The mutant GDHs of the present invention are mutants obtained by introduction of the specific mutations into the wild-type GDHs or the GDHs having a conservative mutation(s) as described above, and, as a result of this, they show improved substrate specificities to glucose. The wording "show an improved substrate specificity to glucose" includes showing a reduced reactivity to other sugars such as monosaccharides, disaccharides, oligosaccharide or the like, for example, maltose, galactose, xylose or the like, while substantially maintaining the reactivity to glucose, or showing an improved reactivity to glucose as compared to the reactivity to other sugars. For example, even if the reactivity to glucose is reduced, the substrate specificity to glucose is improved when the reactivity to other sugars is reduced more than that. And, even if the reactivity to other sugars is increased, a substrate specificity to glucose is improved when the substrate specificity to glucose is increased more than that. Specifically, for example, if the improvement of the substrate specificity of a mutant-type enzyme relative to a wild-type enzyme (a ratio of a specific activity to glucose and a specific activity to other sugars such as maltose) (this improvement is represented by the following formula) is 10% or more, preferably 20% or more, more preferably 40% or more, then the substrate specificity to glucose is improved. For example, if the substrate specificity in a wild-type enzyme is 60% and the substrate specificity in a mutant-type GDH is 40%, then the substrate specificity to glucose is improved by 33%. In mutant GDHs of the invention, the substrate specificity to glucose is improved by at least 20% relative to the GDHs with an amino acid sequence as set forth in SEQ ID NO:3.

$$\text{Substrate Specificity} = (\text{specific activity to sugars other than glucose} / \text{specific activity to glucose}) \times 100$$

$$\text{Improvement of Substrate Specificity} = (A - B) \times 100 / A$$

A: Substrate specificity of wild-type enzyme
B: Substrate specificity of mutant-type enzyme

In a mutant-type GDH, the reactivity to maltose (specific activity) is 1% or less, preferably 0.5% or less, of the reactivity to glucose (specific activity).

The mutant-type GDHs of the present invention preferably show improved substrate specificities to glucose and show reduced reactivities to disaccharides as compared to those of a glucose dehydrogenase whose amino acid residues corresponding to positions 326 and 365 of the amino acid sequence of SEQ ID NO:3 are replaced with glutamine and tyrosine, respectively, but whose amino acid residue corresponding to position 472 is not replaced. Thus a preferred aspect of the invention provides a mutant glucose dehydrogenase with substrate specificity to glucose improved by at least 20% and a reduced reactivity to maltose as compared to the glucose dehydrogenase with an amino acid sequence as set forth in SEQ ID NO:3 except that thet amino acid residues in said glucose dehydrogenase corresponding to positions 326 and 365 of SEQ ID NO:3 are replaced with glutamine and tyrosine respectively.

The "mutations" in the present invention are specifically as follows.

(1) Substitution of serine residue corresponding to position 326 of the amino acid sequence of SEQ ID NO:3 with glutamine.
(2) Substitution of serine residue corresponding to position 365 of the amino acid sequence of SEQ ID NO:3 with tyrosine.
(3) Substitution of alanine residue corresponding to position 472 of the amino acid sequence of SEQ ID NO:3 with tyrosine.

The positions of the amino acid substitution mutations as described above are positions in SEQ ID NO:3, i.e., the amino acid sequence of a wild-type GDH α-subunit of *Burkholderia cepacia* KS1 strain. However, in the case of homologues or variants of the GDH α-subunit which have an amino acid sequence including a substitution(s), a deletion(s), an insertion(s) and/or an addition(s) of one or more amino acid residues in the amino acid sequence of SEQ ID NO:3 in addition to the above-described specific mutations, the positions as described above mean positions corresponding to the above-described amino acid substitution positions in the amino acid sequence alignment of the homologue or variant with SEQ ID NO:3. For example, in the case of a conservative variant of the GDH α-subunit which has a deletion of one amino acid residue in the region from position 1 to position 364, the position 365 as described above means position 364 of this variant.

In SEQ ID NO:7, residues corresponding to positions 326, 365 and 472 of SEQ ID NO:3 are residues of the same positions 326, 365 and 472, respectively.

In SEQ ID NO:8, residues corresponding to positions 326, 365 and 472 of SEQ ID NO:3 are residues of positions 324, 363 and 470, respectively.

In SEQ ID NO:9, residues corresponding to positions 326, 365 and 472 of SEQ ID NO:3 are residues of positions 327, 366 and 473, respectively.

In SEQ ID NO:10, residues corresponding to positions 326, 365 and 472 of SEQ ID NO:3 are residues of positions 327, 366 and 473, respectively.

In SEQ ID NO:11, residues corresponding to positions 326, 365 and 472 of SEQ ID NO:3 are residues of positions 322, 361 and 466, respectively.

In addition, amino acid sequence identities of the amino acid sequence shown in SEQ ID NO:3 to SEQ ID NOs:7 to 11 are 96%, 93%, 82%, 82%, 63%, respectively.

Amino acid sequence alignment of SEQ ID NOs:3 and 7 to 11 is shown in Table 1 below.

[Table 1-1]

| | | | |
|---|---|---|---|
| SEQ ID NO:3 | 1 | MADTDT--QKADVVVVGSGVAGAIVAHQLAMAGKAVILLEAGPRMPRWEI | 48 |
| SEQ ID NO:7 | 1 | MADTDT--QKADVVWGSGVAGAIVAHQLAMAGKSVILLEAGPRMPRWEI | 48 |
| SEQ ID NO:8 | 1 | MAET----QQADVVVVGSGVAGAIVAHQLAMAGKSVILLEAGPRMPRWEI | 46 |
| SEQ ID NO:9 | 1 | MAQSEQTRQQADIVVVGSGVAGALVAYELARAGKSVLMLEAGPRLPRWEI | 50 |
| SEQ ID NO:10 | 1 | MADTRR-ADQADI VVVGSGVAGALVAYELARAGKSVLMLEAGPRLPRWEI | 49 |
| SEQ ID NO:11 | 1 | MANKNS----ADIVVVGSGVAGGLVAHQMALAGASVILLEAGPRIPRWQI | 46 |
| | | | |
| SEQ ID NO:3 | 49 | VERFRNQPDKMDFMAPYPSSPWAPHPEYGP-PNOYLILKGEHKFNSQYUR | 97 |
| SEQ ID NO:7 | 49 | VERFRNQPDKTDFMAPYPSSPWAPHPEYGP-PNDYLILKGEHKFNSQYIR | 97 |
| SEQ ID NO:8 | 47 | VERFRNQPDKMDFMAPYPSSAWAPHPEYAP-PNDYLVLKGEHKFNSQYIR | 95 |
| SEQ ID NO:9 | 51 | VERFRNQADKMDFMAPYPSTAWAPHPEYGP-PNNYLVLKGEHQFNSQYIR | 99 |
| SEQ ID NO:10 | 50 | VERFRNQADKMDFMAPYPSTPTWAPHPEYGPSPNDYLVLKGEHKFDSQYIR | 99 |
| SEQ ID NO:11 | 47 | VENFRNSPVKSDFATPYPSTPYAPHPEYAP-ANNYLIQKGDYPYSSQYLR | 95 |
| | | | |
| SEQ ID NO:3 | 98 | AVGGTTWHWAASAWRFIPNDFKMKSVYGVGRDWPIQYDDLEPYYQRAEEE | 147 |
| SEQ ID NO:7 | 98 | AVGGTTWHWAASAWRFIPNDFKMKTVYGVARDWPIQYDDLEHWYQRAEEE | 147 |
| SEQ ID NO:8 | 96 | AVGGTTWHWAASAWRFIPNDFKMKTVYGVGRDWPIQYDDLEHFYQRAEEE | 145 |
| SEQ ID NO:9 | 100 | AVGGTTWHWAASTWRFLPNDFKLRSVYGIARDWPIQYQDLERYYGLAEEA | 149 |
| SEQ ID NO:10 | 100 | AVGGTTWHWAASTWRFLPKDFKLRSVYGIARWPLQYDDLERDYGRAEAA | 149 |
| SEQ ID NO:11 | 96 | LYGGTTWHWAAAAWRLLPSDFQLHKLYGVGRDWPYPYETLEPWYSAAVQ | 145 |
| | | | |
| SEQ ID NO:3 | 148 | LGVWGPGPEEDLYSPRKQPYPMPPLPLSFNEQTIKTALNNYDPKFHVVTE | 197 |
| SEQ ID NO:7 | 148 | LGVWGPGPEEDLYSPRKQAYPMPPLPLSFNEQT IKSALNGYDPKFHVVTE | 197 |
| SEQ ID NO:8 | 146 | LGVWGPGAEEDLLSPRKAPYPMPPLPLSYNERTIKTALNNHDPKYHVVTE | 195 |
| SEQ ID NO:9 | 150 | LGVWGPN-DEDLGSPRSQPYPMTPLPLSFNERTIKEALNAHDASFHVVTE | 198 |
| SEQ ID NO:10 | 150 | LGVWGPN-DEDLGSPRSQPYPMAPLPLSFNERTI KEALNAHDPAFHVVTE | 198 |
| SEQ ID NO:11 | 146 | LGVSGPGNSIDLGSPRSKPYPMNPLPLSYMDQRFSDVLNAQG-FKVVPE | 193 |
| | | | |
| SEQ ID NO:3 | 198 | PVARNSRPYDGRPTCCGNNNCMPICPIGAMYNGIVHVEKAERAGAKLIEN | 247 |
| SEQ ID NO:7 | 198 | PVARNSRPYDGRPTCCGNNNCMPICPIGAMYNGIVHVEKAEQAGAKLIDS | 247 |
| SEQ ID NO:8 | 196 | PVARNSRPYDGRPTCCGNNNCMPICPIGAMYNGIVHVEKAEQAGAKLIEN | 245 |
| SEQ ID NO:9 | 199 | PVARNSRPYDGRPTCCGNNNCMPICPIEGAMYNGIVHVEKAEQAGARLIEN | 248 |
| SEQ ID NO:10 | 199 | PVARNSRPYDGRPTCCGNNNCMPICPIGAMYNGIVHVEKAEQAGARLIEN | 248 |

(continued)

| | | | |
|---|---|---|---|
| SEQ ID NO:11 | 194 | PVARNSRPYDARPTCCGNNNCMPICPIAAMYNGVVHAEKAEQAGAKLIPE | 243 |
| SEQ ID NO:3 | 248 | AVVYKLETGPDKRIVAALYKDKTGAEHRVEGKYFVLAANGIETPKILLMS | 297 |
| SEQ ID NO:7 | 248 | AVVYKLETGPDKRIVAAIYKDKTGADHRVEGKYFVLAANGIETPKILLMS | 297 |
| SEQ ID NO:8 | 246 | AVVHKLEVGPQKK IVAALYKDPKGAEHRVEGKYFVLAANGIETPKLMLMS | 296 |
| SEQ ID NO:9 | 249 | AVVFKLEVGPNKR IVAARYKDSKGAEHRVEGKWFVLAANG IETPKLMLMS | 298 |
| SEQ ID NO:10 | 249 | AVVYKLEVGAGRRIVAAHYKDPKGVDHRVEGKWFVLAANGIETPKLMLMS | 298 |
| SEQ ID NO:11 | 242 | AVVYRVEADNKGLITAVHYKDPNGNSTRVTGKLFVLAANGIETPKLMLMS | 293 |

[Table 1-2]

| | | | |
|---|---|---|---|
| SEQ ID NO:3 | 298 | ANRDFPNGVANSSDMVGRNLMDHPGTGVSFYASEKLWPGRGPGEMTSLIH | 347 |
| SEQ ID NO:7 | 298 | ANRDFPNGVANSSDMVGRNLMDHPGTGVSFYANEKLWPGRGPQEMTSLIG | 347 |
| SEQ ID NO:8 | 296 | TSHDFPNGVGNSSDMVGRNLMDHPGTGVSFYASEKLWPGRGPQEMTSLIG | 345 |
| SEQ ID NO:9 | 299 | TSQDFPKGVGNSSDMVGRNLMDHPGTGVSFYADRKLWPGRGPGEMTSLIG | 348 |
| SEQ ID NO:10 | 299 | TSEAFPRGVGNSSDMVGRNLMDHPGTGVSFYADRKLWPGRGPQEMTSLIG | 348 |
| SEQ ID NO:11 | 294 | TSDKFPHGVGSSDQVGRNLMDHPGTGVTFLANEALWPGRGPMEMTSIVN | 343 |
| SEQ ID NO:3 | 348 | FRDGPFRATEAAKKIHLSNLSR IDQETQK IFKAGKLMKPDELDAQ I RDRS | 397 |
| SEQ ID NO:7 | 348 | FRDGPFRATEAAKKIHLSNMSRINQETQKIFKAGKLMKHEELDAQI RDRS | 397 |
| SEQ ID NO:8 | 346 | FRDGPFRATEAAKKIHLSNLSRIDQETQKIFKAGKLLKPAELDAQIRDRS | 395 |
| SEQ ID NO:9 | 349 | FRDGPFRATQAGKKLHLSNISRIEQETQRIFKEQKLIKPADLDARIRDQA | 398 |
| SEQ ID NO:10 | 379 | FRDGPFRAMQAGKKLHLSNISR I EQETARI FKAGKLLKPAELDARIRDQA | 398 |
| SEQ ID NO:19 | 344 | FRDGAFRSDYAAKKLHLSNGVPTMSVTADLLKKG-LTGAELDRQIRDRA | 391 |
| SEQ ID NO:3 | 398 | ARYVQFDCFHEILPQPENRIVPSKTATDAIGIPRPEITYAIDDYVKRGAA | 447 |
| SEQ ID NO:7 | 398 | ARYVQFDCFHEILPQPENRIVPSKTATDAIGIPRPEITYAIDDYVKRGAV | 447 |
| SEQ ID NO:8 | 396 | ARYVQFDGFHEILPQPENRIVPSKTATDAIGIPRPEI TYAIDDYYKRGAA | 445 |
| SEQ ID NO:9 | 399 | ARYVQFDSFHEILPLPENRIVPSATEVDAIGIPRPEITYHIDDYVKRSAV | 448 |
| SEQ ID NO:10 | 399 | ARYVQFDSFHE ILPLPENRIVPSATETDALGIPRPEITYR IDDYVKRSAV | 448 |
| SEQ ID NO:11 | | ARTLNI NSFHEHLAEPQNRVVPSADHKDSLGI PQPEI YYS INDYVKKSAA | 441 |
| SEQ ID NO:3 | 448 | HTREVYATAAKVLGGTDVVFNDEFAPNNHITGSTIMGADARDSVVDKDCR | 497 |
| SEQ ID NO:7 | 448 | HTREVYATAAKVLGGTDVVFNDEFAPNNHITGATIMGADARDSWDKDCR | 497 |
| SEQ ID NO:8 | 446 | HTREVYASAAQVLGGTDVVFNDEFAPNNHITGATIMGADPRDSVVDKDCR | 495 |
| SEQ ID NO:9 | 449 | HTREVYATAAQVMGGTNVEFHDDFAPNNHITGATIMGADPKDSVVDKDCR | 498 |
| SEQ ID NO:10 | 449 | HTREVYATAAKVLGATDVQFHDDFAPNNHITGATSMGADPKDSVVDKDCR | 498 |
| SEQ ID NO:11 | 442 | NTHELYAQIAALFGGAEVTFDDTFAPNNHIMGTTIMGSDPADSWDADCR | 491 |
| SEQ ID NO:3 | 498 | TFDHPNLFISSSATMPTVGTVNVTLTIAALALRMSDTLKKEV | 539 |
| SEQ ID NO:7 | 498 | TFDHPNLFISSSSTMPTVGTVNVTLTI AALALRMSDTLKKEV | 539 |
| SEQ ID NO:8 | 496 | TFDHPNLFISSSATMPTVGTVNVTLTIAALALRISDQLKKEI | 537 |
| SEQ ID NO:9 | 499 | TFDHPNLFISSSSTMPTVGTVNVTLTIAALALRIADQLKQEA | 540 |
| SEQ ID NO:10 | 499 | TFDHPNLFISSSATMPTVGTVNVTLTIAALALRIADRLKKEA | 540 |
| SEQ ID NO:11 | 492 | THDHSNLFIASSGVMPTAASVNCTLTIAALSLKLADKLKREI | 533 |

The present inventors studied the most suitable combination of mutations at positions 326, 365 and 472 by using a genetic algorithm so as to increase the substrate specificity to glucose as compared to the mutant glucose dehydrogenase (CyGDH (QY)). As a result, the present inventors discovered a combination of mutations that can provide an improved substrate specificity.

A preferred mode of mutations of the mutant-type GDHs of the present invention is shown below.

(The numerals represent positions in the amino acid sequences; the amino acid residues represent amino acid residues after substitution at those positions; and, the symbol "+" means that two amino acids are simultaneously substituted)
326Gln + 365Tyr + 472Tyr

The GDH α-subunits having the desired mutations can be obtained by introducing nucleotide mutations corresponding to the desired amino acids into a DNA coding for a GDH α-subunit (α-subunit gene) by site-specific mutagenesis, and expressing the obtained mutant DNA using an appropriate expression system. In addition, the mutant CyGDH complexes can be obtained by expressing a DNA coding for a mutant GDH α-subunit together with a DNA coding for a β-subunit (β-subunit gene) or together with a β-subunit gene and a DNA coding for a γ-subunit (γ-subunit gene). Alternatively, for the introduction of mutations into a DNA coding for a GDH α-subunit, a polycistronic DNA fragment coding for a γ-subunit, an α-subunit and a β-subunit in the order mentioned may be used.

The substrate specificity of the GDH α-subunits or CyGDH complexes with the mutations introduced can be decided by examining their reactivities to various sugars with the methods as described in the Examples below and comparing those with the reactivities of wild-type GDH α-subunits or wild-type CyGDH complexes.

The polycistronic DNA fragments coding for a γ-subunit, an α-subunit and a β-subunit in the order mentioned can be obtained, for example, by PCR wherein a chromosomal DNA of *Burkholderia cepacia* KS1 strain is used as a template and oligonucleotides having the nucleotide sequences of SEQ ID NOs:19 and 20 are used as primers (see the Examples as described below).

Examples of vectors used for obtaining these GDH subunit genes, introducing these mutations, and/or expressing these genes, etc., include, for example, a vector that functions in a bacterium belonging to genus *Escherichia,* more specifically pTrc99A, pBR322, pUC18, pUC118, pUC19, pUC119, pACYC184, pBBR122 and the like. Examples of promoters used for expressing these genes include, for example, lac, trp, tac, trc, $P_L$, tet, PhoA and the like. In addition, an α-subunit gene or other subunit gene can be inserted into an appropriate site of a promoter-containing expression vector, thereby accomplishing both the insertion of the gene into the vector and the ligation with the promoter in one step. Examples of such expression vectors include pTrc99A, pBluescript, pKK223-3 and the like.

Alternatively, the α-subunit gene or other subunit gene may be incorporated into a chromosomal DNA of a host microorganism in an expressible form.

Examples of methods for transforming microorganisms with recombinant vectors include, for example, a competent cell method by calcium treatment, a protoplast method, an electroporation method and the like.

Examples of the host, microorganisms include bacteria belonging to genus *Bacillus* such as *Bacillus subtilis,* yeasts such as *Saccharomyces cerevisiae,* filamentous fungi such as *Aspergillus niger.* However, the host microorganisms which can be used are not limited to these examples, and other host microorganisms can be used as long as they are suitable for producing a foreign protein(s).

The mutant α-subunits or the mutant CyGDH complexes, or the microorganisms expressing those, of the present invention, can be used for an enzyme electrode of a glucose sensor or as a component of a glucose assay kit. A glucose sensor and a glucose assay kit which use a wild-type GDH of *Burkholderia cepacia* are described in U.S. Patent Publication No. 2004/0023330A1. The mutant GDHs of the present invention can be used in the same manner.

## EXAMPLES

The present invention will now be further described by way of Examples thereof. However, the present invention is not limited to these Examples in any way.

### [Example 1] Plasmids Expressing GDH or CyGDH *of Burkholderia cepacia*

As a plasmid expressing a GDH of *Burkholderia cepacia,* a plasmid expressing an α-subunit and a γ-subunit of the GDH was prepared; and, as a plasmid expressing a CyGDH, a plasmid expressing an α-subunit, a β-subunit and a γ-subunit was prepared.

### <1> Plasmid Expressing α-Subunit and γ-Subunit of GDH

As a plasmid expressing an α-subunit and a γ-subunit, a plasmid pTrc99A/γ+α which is described in WO02/036779 (corresponding to EP1331272A1, US2004023330A and CN1484703A) was used. This plasmid is a plasmid wherein a DNA fragment contiguously containing a GDH γ-subunit structural gene and a GDH α-subunit structural gene, which was isolated from chromosomal DNA of *Burkholderia cepacia* KS1 strain (FERM BP-7306), was inserted into the NcoI/HindIII site which is a cloning site of the vector pTrc99A. The GDHγα gene in this plasmid is regulated by the trc promoter. The pTrc99A/γ+α has an ampicillin resistance gene.

The whole plasmid, including the DNA fragment which contains a sequence coding for six histidine residues added to the C-terminus of the GDH α-subunit, was amplified by PCR, wherein the above-mentioned plasmid pTrc99A/γ+α was used as a template and oligonucleotides having the following sequences were used as primers.

[Forward Primer]

5'-ACCACCACTGATAAGGAGGTCTGACCGTGCGGAAATCTAC-3' (SEQ ID NO:17)

[Reverse Primer]

5'-AGCCTGTGCGACTTCTTCCTTCAGCGATCGGTGGTGGTGG-3' (SEQ ID NO:18)

Both termini of the amplified fragments were blunted. Thereafter, the 5'-terminal was phosphorylated, and the resulting fragments were circularized by ligation. *Escherichia coli* DH5α was transformed with the obtained recombinant vector, and colonies that were formed on LB agar medium containing 50 μg/mL of ampicillin were collected. The obtained transformants were cultured in liquid LB medium and the plasmid therein was extracted. The inserted DNA fragment was analyzed, and, as a result, about 2.1 kb of inserted fragment was confirmed. The GDH structural genes in this plasmid are regulated by the trc promoter. This plasmid has an ampicillin resistance gene.

<2> Plasmid Expressing α-Subunit, β-Subunit and γ-Subunit of CyGDH

A plasmid expressing an α-subunit, a β-subunit and a γ-subunit of a CyGDH was prepared as described below.

(1) Preparing Chromosomal DNA from *Burkholderia cepacia* KS1 Strain

Chromosomal genes were prepared from *Burkholderia cepacia* KS1 strain according to a conventional method. In other words, cells of the strain were shaken in TL liquid medium (polypeptone 10 g, yeast extract 1 g, NaCl 5 g, $KH_2PO_4$ 2 g, glucose 5 g; 1L, pH 7.2) at 34°C overnight. The proliferated bacterial cells were collected by centrifugation. These bacterial cells were suspended in a solution containing 10 mM NaCl, 20 mM Tris-HCl (pH 8.0), 1 mM EDTA, 0.5% SDS, and 100 μg/ml of proteinase K and treated at 50°C for 6 hours. An equal amount of phenol-chloroform was added to this solution and the resultant mixture was stirred at room temperature for 10 minutes. Thereafter, the supernatant was collected by centrifugation. Sodium acetate was added thereto so as to attain a final concentration of 0.3 M, and double the amount of ethanol was overlaid thereon to precipitate the chromosomal DNA in the middle layer. The precipitated DNA was spooled out using a glass rod, washed with 70% ethanol, and then dissolved in an appropriate amount of TE buffer to obtain a chromosomal DNA solution.

(2) Preparing DNA Fragment Coding for γ-Subunit, α-Subunit and β-Subunit of CyGDH

A DNA fragment coding for a γ-subunit, an α-subunit and a β-subunit of a CyGDH was amplified by PCR, wherein the above-mentioned chromosomal DNA was used as a template and oligonucleotides having the following sequences were used as primers.

[Forward Primer]

5'-CATGCCATGGCACACAACGACAACAC-3' (SEQ ID NO:19)

[Reverse Primer]

5'-GTCGACGATCTTCTTCCAGCCGAACATCAC-3' (SEQ ID NO:20)

The C-termini of the amplified fragments were blunted, and thereafter the N-termini were digested with NcoI. The resulting fragments were ligated to the pTrc99A treated in the same manner. *Escherichia coli* DH5α was transformed with the obtained recombinant vector, and colonies that were formed on LB agar medium containing 50 μg/mL of ampicillin were collected. The obtained transformants were cultured in liquid LB medium and the plasmid therein was extracted. The inserted DNA fragment was analyzed, and, as a result, about 3.8 kb of inserted fragments was confirmed. This plasmid was named pTrc99Aγαβ. The CyGDH structural genes in this plasmid are regulated by the trc promoter. The pTrc99Aγαβ has an ampicillin resistance gene and a kanamycin resistance gene.

[Example 2] Search for Substrate Interaction Sites by Introducing Mutations into CyGDH α-Subunit Gene

(1) Introducing Mutations into Positions 326,365 and 472

Mutations were introduced into the GDH α-subunit gene contained in the pTrc99Aγαβ obtained in the Example 1 so that the serine residue at position 326, serine residue at position 365 and alanine residue at position 472 of the α-subunit that was coded for by that gene were replaced with other amino acid residues.

Specifically, the codon corresponding to the serine at position 326 (TCG), the codon corresponding to the serine at position 365 (TCG) and the codon corresponding to the alanine at position 472 (GCG) of the GDH α-subunit genes contained in the plasmids pTrc99A/γ+α and pTrc99Aγαβ as described in Example 1 were replaced with codons corresponding to other amino acids using a commercially available site-specific mutagenesis kit (Stratagene, QuikChangeII Site-Directed Mutagenesis Kit).

The sequences of forward primers and reverse primers used in the above-mentioned amino acid residue substitutions are shown in Table 2 below. Reverse primers used for introducing 3 mutations are shown in Table 3.

In the notations that represent the mutations, the numerals represent positions in the amino acid sequences, the letters before the numerals represent amino acid residues before the amino acid substitutions, and the letters after the numerals represent amino acid residues after the amino acid substitutions. For example, R53F indicates that arginine at position 53 is replaced with phenylalanine.

PCR was carried out with the following reaction composition by performing a reaction of 95°C for 30 seconds; then repeating 15 times a cycle of 95°C for 30 seconds, 55°C for 1 minute and 68°C for 8 minutes; performing a reaction of 68°C for 30 minutes; and then maintaining at 4°C.

[Composition of the Reaction Solution]

| | |
|---|---|
| Template DNA (5 ng/μl) | 2 μl |
| (pTrc99A/γ+α or pTrc99Aγαβ with 3 mutations introduced) | |
| 10 x Reaction Buffer Solution | 5 μl |
| Forward Primer (100 ng/μl) | 1.25 μl |
| Reverse Primer (100 ng/μl) | 1.25 μl |
| dNTPs | 1 μl |
| Distilled Water | 38.5 μl |
| DNA Polymerase | 1 μl |
| Total | 50 μl |

After the PCR, 0.5 μL of DpnI was added to the reaction solution, and the resultant mixture was incubated at 37°C for 1 hour to degrade the template plasmid.

Competent cells of *Escherichia coli* DH5α (supE44, ΔlacU169 (φ80lacZΔM15), hsdR17, recAi, endAI, gyrA96, thi-1, relAI) were transformed using the obtained reaction solution. Each plasmid DNA was prepared from several colonies that had been grown on LB agar medium (bacto tryptone 1%, yeast extract 0.5%, sodium chloride 1% and agar 1.5%) containing ampicillin (50 μg/ml) and kanamycin (30 μg/ml), and the sequence thereof was analyzed to confirm that mutations of interest were introduced into the GDH α-subunit gene.

[Table 2]

Forward Primers for S326

| Amino acid substitution | Primer name | Sequence | SEQ ID NOs. |
|---|---|---|---|
| S326E | CGDH326ED | 5'GAATTCTATGCGAGCGAGAAGCTGTGGCCG3' | SEQ ID NO:21 |
| S326F | CGDH326FD | 5'TTCTTCTATGCGAGCGAGAAGCTGTGGCCG3' | SEQ ID NO:22 |
| S326I | CGDH326ID | 5'ATCTTCTATGCGAGCGAGAAGCTGTGGCCG3' | SEQ ID NO:23 |
| S326K | CGDH326KD | 5'AAATTCTATGCGAGCGAGAAGCTGTGGCCG3' | SEQ ID NO:24 |
| S326L | CGDH326LD | 5'CTGTTCTATGCGAGCGAGAAGCTGTGGCCG3' | SEQ ID NO:25 |
| S326N | CGDH326ND | 5'AACTTCTATGCGAGCGAGAAGCTGTGGCCG3' | SEQ ID NO:26 |
| S326Q | CGDH326QD | 5'CAG'TTCTATGCGAGCGAGAAGCTGTGGCCG3' | SEQ ID NO:27 |
| S326R | CGDH326RD | 5'CGCTTCTATGCGAGCGAGAAGCTGTGGCCG3' | SEQ ID NO:28 |

(continued)

Forward Primers for S326

| Amino acid substitution | Primer name | Sequence | SEQ ID NOs. |
|---|---|---|---|
| S326T | CGDH326TD | 5'ACCTTCTATGCGAGCGAGAAGCTGTGGCCG3' | SEQ ID NO:29 |
| S326V | CGDH326VD | 5'GTTTTCTATGCGAGCGAGAAGCTGTGGCCG3' | SEQ ID NO:30 |
| S326W | CGDH326WD | 5'TGGTTCTATGCGAGCGAGAAGCTGTGGCCG3' | SEQ ID NO:31 |
| S326Y | CGDH326YD | 5'TACTTCTATGCGAGCGAGAAGCTGTGGCCG3' | SEQ ID NO:32 |

Reverse Primers for S326

| Primer name | Sequence | SEQ ID NOs. |
|---|---|---|
| CGDH326RSma | 5'CACGCCGGTCCCGGGATGGTCCATCAGGTT3' | SEQ ID NO:33 |
| CGDH326U | 5'CACGCCGGTGCCCGGATGGTCCATCAGGTT3' | SEQ ID NO:34 |

Forward Primers for S365

| Amino acid substitution | Primer name | Sequence | SEQ ID NOs. |
|---|---|---|---|
| S365D | CGDH365DD | 5'GACAACCTGTCGCGCATCGACCAGGAGACG3' | SEQ ID NO:35 |
| S365F | CGDH365FD | 5'TTCAACCTGTCGCGCATCGACCAGGAGACG3' | SEQ ID NO:36 |
| S365I | CGDH365ID | 5'ATCAACCTGTCGCGCATCGACCAGGAGACG3' | SEQ ID NO:37 |
| S365K | CGDH365KD | 5'AAAAACCTGTCGCGCATCGACCAGGAGACG3' | SEQ ID NO:38 |
| S365Q | CGDH365QD | 5'CAGAACCTGTCGCGCATCGACCAGGAGACG3' | SEQ ID NO:39 |
| S365R | CGDH365RD | 5'CGTAACCTGTCGCGCATCGACCAGGAGACG3' | SEQ ID NO:40 |
| S365T | CGDH365TD | 5'ACCAACCTGTCGCGCATCGACCAGGAGACG3' | SEQ ID NO:41 |
| S365Y | CGDH365YD | 5'TACAACCTGTCGCGCATCGACCAGGAGACG3' | SEQ ID NO:42 |

Reverse Primers for S365

| Primer name | Sequence | SEQ ID NOs. |
|---|---|---|
| CGDH365U 5' | CAGGTGGATCTTCTTCGCCGCTTCGGTCGC3' | SEQ ID NO:43 |

Forward P rimers for A472

| Amino acid substitution | Primer name | Sequence | SEQ ID NOs. |
|---|---|---|---|
| A472A | CGDH472AD | 5'GCGCCGAACAATCACATCACGGGCTCGACG3' | SEQ ID NO:44 |
| A472D | CGDH472DD | 5'GACCCGAACAATCACATCACGGGCTCGACG3' | SEQ ID NO:45 |
| A472E | CGDH472ED | 5'GAACCGAACAATCACATCACGGGCTCGACG3' | SEQ ID NO:46 |
| A472F | CGDH472FD | 5'TTCCCGAACAATCACATCACGGGCTCGACG3' | SEQ ID NO:47 |
| A472H | CGDH472HD | 5'CATCCGAACAATCACATCACGGGCTCGACG3' | SEQ ID NO:48 |
| A472I | CGDH472ID | 5'ATCCCGAACAATCACATCACGGGCTCGACG3' | SEQ ID NO:49 |
| A472K | CGDH472KD | 5'AAACCGAACAATCACATCACGGGCTCGACG3' | SEQ ID NO:50 |
| A472N | CGDH472ND | 5'AACCCGAACAATCACATCACGGGCTCGACG3' | SEQ ID NO:51 |
| A472R | CGDH472RD | 5'CGTCCGAACAATCACATCACGGGCTCGACG3' | SEQ ID NO:52 |
| A472S | CGDH472SD | 5'AGTCCGAACAATCACATCACGGGCTCGACG3' | SEQ ID NO:53 |
| A472T | CGDH472TD | 5'ACTCCGAACAATCACATCACGGGCTCGACG3' | SEQ ID NO:54 |
| A472Y | CGDH472YD | 5'TACCCGAACAATCACATCACGGGCTCGACG3' | SEQ ID NO:55 |

Reverse Primers for A472

| Primer name | Sequence | SEQ ID NOs. |
|---|---|---|
| CGDH472U | 5'GAATTCGTCGTTGAACACGACGTCCGTGCC3' | SEQ ID NO:56 |
| CGDH472UdE1 | 5'GAACTCGTCGTTGAACACGACGTCCGTGCC3' | SEQ ID NO:57 |

[Table 3]

List of Three Mutations and Reverse Primer Used

| | Reverse primer for S326 | Reverse primer for S365 | Reverse primer for A472 |
|---|---|---|---|
| TTS | CGDH326RSma | CGDH365U | CGDH472UdE1 |
| TDF | CGDH326U | CGDH365U | CGDH472UdE1 |

(continued)

List of Three Mutations and Reverse Primer Used

| | Reverse primer for S326 | Reverse primer for S365 | Reverse primer for A472 |
|---|---|---|---|
| YKY | CGDH326U | CGDH365U | CGDH472UdE1 |
| YQE | CGDH326U | CGDH365U | CGDH472U |
| QII | CGDH326RSma | CGDH365U | CGDH472U |
| QDN | CGDH326RSma | CGDH365U | CGDH472U |
| ERY | CGDH326RSma | CGDH365U | CGDH472UdE1 |
| EFE | CGDH326RSma | CGDH365U | CGDH472UdE1 |
| IKD | CGDH326U | CGDH365U | CGDH472UdE1 |
| QYR | CGDH326U | CGDH365U | CGDH472U |
| LTN | CGDH326U | CGDH365U | CGDH472U |
| RYK | CGDH326U | CGDH365U | CGDH472U |
| KYT | CGDH326RSma | CGDH365U | CGDH472U |
| RRI | CGDH326U | CGDH365U | CGDH472U |
| KYI | CGDH326RSma | CGDH365U | CGDH472U |
| YYS | CGDH326U | CGDH365U | CGDH472U |
| IFK | CGDH326U | CGDH365U | CGDH472UdE1 |
| IFR | CGDH326U | CGDH365U | CGDH472UdE1 |
| FRY | CGDH326U | CGDH365U | CGDH472U |
| FRA | CGDH326U | CGDH365U | CGDH472U |
| EYT | CGDH326RSma | CGDH365U | CGDH472UdE1 |
| FRE | CGDH326U | CGDH365U | CGDH472U |
| RKA | CGDH326RSma | CGDH365U | CGDH472U |
| RRY | CGDH326U | CGDH365U | CGDH472U |
| KYR | CGDH326U | CGDH365U | CGDH472U |
| KYS | CGDH326RSma | CGDH365U | CGDH472UdE1 |
| WFK | CGDH326U | CGDH365U | CGDH472UdE1 |
| IRY | CGDH326U | CGDH365U | CGDH472U |
| IYT | CGDH326U | CGDH365U | CGDH472U |
| IYH | CGDH326U | CGDH365U | CGDH472U |
| EFK | CGDH326RSma | CGDH365U | CGDH472UdE1 |
| FRH | CGDH326U | CGDH365U | CGDH472U |
| NRF | CGDH326U | CGDH365U | CGDH472UdE1 |
| NRT | CGDH326U | CGDH365U | CGDH472UdE1 |
| VRK | CGDH326U | CGDH365U | CGDH472UdE1 |
| KYH | CGDH326RSma | CGDH365U | CGDH472UdE1 |
| LYF | CGDH326RSma | CGDH365U | CGDH472U |
| QRY | GDH326U | CGDH365U | CGDH472U |
| QYY | CGDH326U | CGDH365U | CGDH472U |
| KRH | CGDH326RSma | CGDH365U | CGDH472U |

[Example 3] Analysis of Substrate Specificities of Mutant GDHs

Mutant GDHs were produced by using the plasmids expressing mutant GDHs which were obtained in Example 2, and their substrate specificities were studied.

(1) Culture

The cells of *Escherichia coli* DH5$\alpha$ strain wherein mutations were introduced in various patterns were each cultured under shaking at 37°C overnight in 2 ml of LB medium (containing 50 μg/ml of ampicillin and 30 μg/ml of kanamycin) by using a L-shaped tube. These cultures were inoculated into a 500 ml Sakaguchi flask that contained 150 ml of LB medium (containing 50 μg/ml of ampicillin and 30 μg/ml of kanamycin), and the resultant cultures were cultured under shaking at 37°C. Three hours after the beginning of the culturing, IPTG (isopropyl-β-D-thiogalactopyranoside) was added so as to attain a final concentration of 0.1 mM, and the resultant cultures were cultured for another 2 hours.

(2) Preparing Crude Enzyme Samples

The bacterial cells were collected from the cultures which had been cultured as described above, and the collected cells were washed. Thereafter, the bacterial cells were suspended with 1 ml of 10 mM potassium phosphate buffer (PPB) (pH 7.0) containing 0.2% Triton X100 per 0.3 mg of wet bacterial cells, and sonicated. These suspensions were centrifuged (10,000 r.p.m, 10 minutes, 4°C) to remove the residues. Thereafter, the supernatants were ultracentrifuged (50,000 r.p.m., 60 minutes, 4°C), and the obtained supernatants (water-soluble fractions) were considered as crude enzyme samples. Further, these samples were purified by usual hydrophobic chromatography (column name: Octyl Sepharose (manufactured by Amersham Biosciences)) and ion exchange chromatography (Q-Sepharose (manufactured by Amersham Biosciences) to obtain purified enzyme samples. Which fractions contained the enzymes of interest was decided by using their GDH activity as an index.

Furthermore, purification of His-tag containing proteins was performed as described below.

The bacterial cells were collected from the cultures which had been cultured, and the collected cells were washed. Thereafter, the bacterial cells were suspended in 1 ml of 20 mM sodium phosphate buffer (pH 7.5) containing 0.5 M sodium chloride and 20 mM imidazole per 0.3 mg of wet bacterial cells; and sonicated. These suspensions were centrifuged (10,000, r.p.m, 10 minutes, 4°C) to remove the residues. Thereafter, the supernatants were ultracentrifuged (50,000 r.p.m., 60 minutes, 4°C), and the obtained supernatants (water-soluble fractions) were considered as crude enzyme samples. These samples were added into HisTrap FF columns (manufactured by Amersham Biosciences) equilibrated with 20 mM sodium phosphate buffer (pH 7.5) containing 0.5 M sodium chloride and 20 mM imidazole. The columns were washed with 20 mM sodium phosphate buffer (pH 7.5) containing 0.5 M sodium chloride and 60 mM imidazole, then subjected to elution with 20 mM sodium phosphate buffer (pH 7.5) containing 0.5 M sodium chloride and 150 mM imidazole to obtain purified enzyme samples. Which fractions contained the enzymes of interest was decided by using their GDH activity as an index.

(3) Measurement of GDH Activity

To 8 μl of each crude enzyme sample as mentioned above, 8 μl of a reagent for measuring the activity (a solution that was obtained by adding 10 mM PPB containing 0.2 (w/v)% Triton X-100 to 12 μl of 600 mM methylphenazine methosulfate (PMS) and 120 μl of 6 mM 2,6-dichlorophenolindophenol (DCIP) so as to attain a total volume of 480 μl) was added. The obtained mixture was preincubated at each reaction temperature for I minute by using an aluminum block thermostat, and then 8 μl of each concentration of substrate (glucose or maltose) or distilled water was quickly added. The resultant solution was stirred, and absorbance at 600 nm, which is an absorption wavelength of DCIP, was measured using a spectrophotometer. The final concentrations of the reagents DCIP and PMS were 0.06 mM and 0.6 mM, respectively. The final concentrations of the substrates were 10 mM or 5 mM.

The results are shown in Table 4. At 10 mM and 5 mM substrate concentrations, the reaction ratios of the wild-type GDH were 23.32% and 18.88%, respectively.

[Table 4]

| U/mg crude | 10mM | | | 5mM | | |
|---|---|---|---|---|---|---|
| protein | Glu | Mal | Mal/Glu(%) | Glu | Mal | Mal/Glu(%) |
| WT | 4.69 | 1.09 | 23.32 | 3.99 | 0.75 | 18.88 |
| QYA | 0.68 | 0.0065 | 0.96 | 0.43 | 0.0038 | 0.88 |
| TTS | 1.40 | 0.23 | 16.18 | 1.22 | 0.19 | 15.25 |
| TDF | 0.37 | 0.17 | 45.16 | 0.23 | 0.12 | 52.63 |
| YKY | 0.14 | 0.07 | 51.72 | 0.10 | 0.05 | 50.00 |
| YQE | 0.06 | 0.01 | 9.52 | 0.04 | 0.00 | 8.46 |
| QII | 0.40 | 0.06 | 15.00 | 0.24 | 0.03 | 13.89 |
| QDN | 0.17 | 0.08 | 47.42 | 0.11 | 0.05 | 48.44 |
| ERY | 0.29 | 0.002 | 0.79 | 0.17 | 0.001 | 0.78 |
| EFE | 0.05 | 0.001 | 2.74 | 0.03 | 0.001 | 2.75 |
| IKD | 0.01 | 0.029 | 236.36 | 0.01 | 0.019 | 188.89 |
| QYR | 1.90 | 0.0180 | 0.97 | 1.21 | 0.0112 | 0.93 |
| LTN | 0.07 | 0.0140 | 18.95 | 0.05 | 0.0105 | 19.44 |
| RYK | 1.08 | 0.2400 | 22.09 | 0.75 | 0.1348 | 17.90 |
| KYT | 1.135 | 0.0082 | 0.721 | 0.669 | 0.0048 | 0.714 |
| RRI | 0.08 | 0.0048 | 6.32 | 0.06 | 0.0037 | 5.70 |
| KYI | 0.12 | 0.0022 | 1.80 | 0.07 | 0.0018 | 2.53 |
| YYS | nd | nd | nd | nd | nd | nd |
| IFK | 1.27 | 0.0380 | 2.99 | 0.81 | 0.0243 | 3.00 |
| IFR | 0.28 | 0.0094 | 3.38 | 0.18 | 0.0060 | 3.36 |
| FRY | 0.34 | 0.0060 | 1.76 | 0.26 | 0.0037 | 1.42 |
| FRA | 0.14 | 0.0086 | 6.09 | 0.13 | 0.0065 | 5.08 |
| YET | 0.13 | 0.0026 | 1.94 | 0.09 | 0.0024 | 2.79 |
| FRE | nd | nd | nd | nd | nd | nd |
| RKA | nd | nd | nd | nd | nd | nd |
| RRY | 0.49 | 0.0050 | 1.01 | 0.38 | 0.0034 | 0.88 |
| KYR | 1.67 | 1.61E-02 | 0.97 | 1.09 | 8.68E-03 | 0.80 |
| KYS | 0.47 | 3.64E-03 | 0.78 | 0.29 | 1.82E-03 | 0.63 |
| WFK | 0.61 | 2.47E-02 | 4.03 | 0.43 | 1.38E-02 | 3.22 |
| IRY | 0.24 | 5.01E-02 | 21.2 | 0.18 | 4.75E-02 | 26.54 |
| IYT | 0.21 | - | - | 0.13 | - | - |
| IYH | 0.16 | - | - | 0.09 | - | - |
| EFK | 0.18 | 5.74E-03 | 3.20 | 0.10 | 3.23E-03 | 3.21 |
| FRH | 0.05 | - | - | 0.47 | - | - |
| NRF | 0.19 | - | - | 0.13 | - | - |
| NRT | 0.26 | 1.13E-02 | 4.31 | 0.23 | 5.85E-03 | 2.55 |
| VRK | 0.09 | 4.74E-03 | 5.42 | 0.08 | 2.55E-03 | 3.18 |
| KYH | 0.04 | - | - | 0.02 | - | - |
| LYF | 0.15 | - | - | 0.09 | - | - |
| QRY | 0.35 | 7.64E-02 | 21.8 | 0.26 | 0.0531 | 20.5 |
| QYY | 0.55 | 1.91E-03 | 0.35 | 0.31 | 0.00143 | 0.46 |
| KRH | nd | nd | nd | nd | nd | nd |

In this Table, QYA represents a mutant glucose dehydrogenase whose amino acid residues corresponding to positions 326 and 365 are replaced with glutamine and tyrosine, respectively, but whose amino acid residue corresponding to position 472 is not replaced (that is to say, the amino acid residue corresponding to position 472 remains an alanine residue), i.e., a GDH (QY).

As a result, it was found that 326Glu + 365Arg + 472Tyr, 326Gln + 365Tyr + 472Arg, 326Lys + 365Tyr + 472Thr, 326Ile + 365Phe + 472Lys, 326Arg + 365Arg + 472Tyr, 326Lys + 365Tyr + 472Arg, 326Lys + 365Tyr + 472Ser, and 326Gln + 365Tyr + 472Tyr were very effective mutations wherein the reactivities to glucose were maintained and the reactivities to maltose were reduced.

In other words, a synergistic effect equal to or more than that of the mutant GDH (QY) was confirmed in some of the combinations of the three mutations at positions 326, 365 and 472.

[Example 4] Preparing Purified Enzymes (Complexes of α-Subunit and γ-Subunit)

The 8 mutant GDHs which showed improved substrate specificities in Example 3 were purified. The same method as described in Example 3 was used. Specific activities to glucose and maltose (U/mg), reaction ratios (specific activity to maltose / specific activity to glucose), and Km values and Vmax values for glucose, of each purified enzyme, are shown in Table 5.

As a result, it was found that the reaction ratios of QYA with 10 mM and 5 mM of maltose were both at the level of 1%, whereas the reaction ratios of ERY and KYT were reduced to 1% or less and the reaction ratios of QYY with maltose were reduced to the level of 0.3%.

[Table 5]

| | 10mM | | | 5mM | | | Glucose | | | Mol/Glu (%) |
|---|---|---|---|---|---|---|---|---|---|---|
| | Glu(U/mg) | Mol(U/mg) | Mol/Glu(%) | Glu(U/mg) | Mol(U/mg) | Mol/Glu(%) | Km (mM) | Vmax (U/mg) | Vmax/Km | |
| WT his | 90.2 | 23.7 | 26.3 | 77.6 | 15.8 | 20.4 | 1.35 | 100 | 74.1 | 10.9 |
| QYA his | 55.3 | 0.56 | 1.02 | 32.8 | 0.41 | 1.25 | 4.32 | 62.9 | 14.6 | 2.08 |
| RYT his | 5.18 | 0.05 | 1.03 | 2.89 | 0.03 | 1.05 | 27.5 | 19.1 | 0.69 | 1.32 |
| KYK his | 5.27 | 0.09 | 1.67 | 3.51 | 0.05 | 1.54 | 10.5 | 10.9 | 1.04 | 1.37 |
| ERY his | 9.53 | 0.08 | 0.89 | 6.07 | 0.05 | 0.75 | 22.9 | 33.4 | 1.46 | 2.26 |
| QYR his | 41.3 | 0.44 | 1.07 | 25.1 | 0.37 | 1.47 | 5.34 | 55.6 | 10.4 | 1.90 |
| KYT his | 33.3 | 0.21 | 0.63 | 19.6 | 0.16 | 0.81 | 8.13 | 52.1 | 6.41 | 3.26 |
| IFK his | 5.08 | 0.23 | 4.49 | 3.39 | 0.13 | 3.85 | 6.52 | 7.70 | 1.18 | 2.90 |
| RRY his | 2.64 | 0.04 | 1.67 | 2.15 | 0.03 | 1.36 | 3.82 | 3.82 | 1.00 | 2.40 |
| QYY his | 32.2 | 0.09 | 0.29 | 20.2 | 0.07 | 0.34 | 13.8 | 76.3 | 5.53 | unmeasurable |
| **DCIP 0.6 mM; PMS 6 mM, room temperature** | | | | | | | | | | |

[Example 5] Preparing Purified Enzymes (Complexes of α-Subunit, β-Subunit and γ-Subunit)

The mutant CyGDH (QYY) which showed an improved substrate specificity in Example 3 was purified. The same method as described in Example 3 was used. Specific activities to glucose (U/mg) of each purified enzyme are shown in Table 6.

As a result, it was found that, in the form of a complex of an α-subunit, a β-subunit and a γ-subunit, the reactivity with maltose of QYY (326Gln + 365Tyr + 472Tyr) were reduced to about 1/2 to 1/3 as compared to that of QYA (326Gln + 365Tyr).

[Table 6]

| Glucose (mM) | 0.1 | 0.5 | 1 | 2 | 5 | 7 | 10 | 15 | 20 |
|---|---|---|---|---|---|---|---|---|---|
| **QYA** (U/mg protein) | 1.37 | 24.7 | 60.4 | 131 | 250 | 350 | 420 | 513 | 551 |
| **QYY** (U/mg protein) | 0.80 | 9.88 | 35.4 | 62.7 | 139 | 202 | 233 | 324 | 409 |
| **WT** (U/mg protein) | 5.28 | 45.2 | 108 | 263 | 421 | 485 | 584 | 631 | 651 |
| | | | | | | | | | |
| **QYA** (U/mg protein) | 0.009 | 0.47 | 0.17 | 0.14 | 0.66 | 0.66 | 1.38 | 3.13 | 3.44 |
| **QYY** (U/mg protein) | 0.005 | 0.015 | 0.03 | 0.07 | 0.11 | 0.31 | 0.45 | 0.59 | 0.99 |
| **WT** (U/mg protein) | 1.41 | 5.41 | 13.9 | 28.5 | 42.7 | 48.7 | 65.6 | 80.7 | 83.4 |
| Mal/Glu | 0.1 | 0.5 | 1 | 2 | 5 | 7 | 10 | 15 | 20 |
| **QYA** | 0.69% | 0.19% | 0.28% | 0.11% | 0.27% | 0.19% | 0.33% | 0.61% | 0.62% |
| **QYY** | 0.62% | 0.16% | 0.08% | 0.12% | 0.08% | 0.16% | 0.19% | 0.18% | 0.24% |
| **WT** | 26.7% | 12.0% | 12.9% | 8.95% | 10.1% | 10.0% | 11.2% | 12.8% | 12.8% |

[Example 6] Analysis of Substrate Specificities of Mutant Enzymes (QYX Mutation)

Mutations were introduced in the same manner as in Example 2 into the GDH α-subunit gene contained in the pTrc99A/γ+α so that serine residue at position 326, serine residue at position 365 and alanine residue at position 472 of the α-subunit that were coded for by that gene were replaced with glutamine, tyrosine and an amino acid residue other than alanine, respectively. Using the obtained plasmids expressing mutant enzymes, in the same manner as in Example 3, mutant enzymes were produced, and their substrate specificities were studied. The results are shown in Table 7 below.

[Table 7]

| U/mL sample | Glucose | | | Maltose | | | 5mM Mal /5mM Glc | 50mM Mal /50mM Glc | 50mM Mal /5mM Glc |
|---|---|---|---|---|---|---|---|---|---|
| | 5mM | 50mM | 5mM /50mM | 5mM | 50mM | 5mM /50mM | | | |
| **QYA(Wild)** | **5.68** | **14.60** | **38.9%** | **0.07** | **0.36** | **18.2%** | **1.1%** | **2.5%** | 6.3% |
| QYC | 0.46 | 0.90 | 51.7% | 0.00 | 0.05 | 0.0% | 0.0% | 5.6% | 10.9% |
| **QYD** | **3.31** | **12.69** | **26.1%** | **0.08** | **0.28** | **27.2%** | **2.3%** | **2.2%** | **8.4%** |
| QYE | 2.44 | 11.96 | 20.4% | 0.00 | 0.16 | 0.0% | 0.0% | 1.4% | 6.7% |
| QYF | 0.46 | 8.48 | 5.5% | 0.01 | 0.05 | 16.1% | 1.8% | 0.6% | 11.0% |
| **QYG** | **3.27** | **9.58** | **34.2%** | **0.05** | **0.12** | **44.7%** | **1.7%** | **1.3%** | **3.7%** |
| QYH | 0.85 | 6.30 | 13.5% | 0.00 | 0.07 | 0.0% | 0.0% | 1.1% | 8.3% |
| QYI | 2.64 | 10.72 | 24.7% | 0.00 | 0.18 | 0.0% | 0.0% | 1.7% | 6.8% |
| **QYK** | **3.06** | **9.08** | **33.8%** | **0.03** | **0.34** | **7.7%** | **0.9%** | **3.8%** | **11.2%** |
| **QYL** | **7.48** | **20.86** | **35.8%** | **0.04** | **0.27** | **16.5%** | **0.6%** | **1.3%** | **3.6%** |
| QYM | 2.10 | 11.42 | 18.4% | 0.01 | 0.29 | 1.8% | 0.3%. | 2.5% | 13.8% |
| QYN | 1.88 | 14.77 | 12.7% | 0.05 | 0.23 | 22.3% | 2.7% | 1.6% | 12.3% |

(continued)

| U/mL sample | Glucose | | | Maltose | | | 5mM Mal /5mM Glc | 50mM Mal /50mM Glc | 50mM Mal /5mM Glc |
|---|---|---|---|---|---|---|---|---|---|
| | 5mM | 50mM | 5mM /50mM | 5mM | 50mM | 5mM /50mM | | | |
| **QYP** | **1.88** | **7.47** | **25.2%** | **0.01** | **0.06** | **24.7%** | **0.7%** | **0.7%** | **3.0%** |
| **QYQ** | **4.75** | **15.41** | **30.8%** | **0.06** | **0.34** | **17.6%** | **1.3%** | **2.2%** | **7.2%** |
| **QYR** | **4.30** | **12.58** | **34.2%** | **0.06** | **0.33** | **19.8%** | **1.5%** | **2.6%** | **7.6%** |
| QYS | 2.19 | 12.00 | 18.2% | 0.00 | 0.18 | 0.0% | 0.0% | 1.5% | 8.1% |
| **QYT** | **3.23** | **12.54** | **25.8%** | **0.06** | **0.20** | **32.5%** | **2.0%** | **1.6%** | **6.1%** |
| **QYV** | **5.11** | **15.91** | **32.1%** | **0.05** | **0.31** | **15.0%** | **0.9%** | **1.9%** | **6.0%** |
| QYW | 0.80 | 9.35 | 8.6% | 0.00 | 0.11 | 0.0% | 0.0% | 1.2% | 13.5% |
| **QYY** | **2.38** | **14.49** | **16.4%** | **0.01** | **0.09** | **8.9%** | **0.3%** | **0.6%** | **3.6%** |

As a result, it was found that QYD, QYG2 QYK, QYL, QYP, QYQ, QYR, QYT, QYV and QYY were mutations wherein the reactivities to glucose were maintained and the reactivities with maltose were reduced.

[Example 7] Preparing Purified Enzymes (Complexes of α-Subunit and γ-Subunit)

The 10 mutant GDHs which showed improved substrate specificities in Example 6 (QYD, QYG, QYK, QYL, QYP, QYQ, QYR, QYT, QYV and QYY) were purified. The same method as described in Example 3 was used. Specific activities to glucose and maltose (U/mg), reaction ratios (specific activity to maltose / specific activity to glucose), and Km values and Vmax values for glucose, of each purified enzyme, are shown in Table 8.

As a result, it was found that the reaction ratios of the mutant GDHs with maltose were all low, but, except for QYY, the enzyme activities of these mutant GDHs were reduced, suggesting that these mutant GDHs except for QYY are not suitable for measuring the glucose concentration. In other words, it is suggested that, among the 10 mutant GDHs represented by QYX, QYY is most suitable for measuring the glucose concentration.

[Table 8]

**QYY**
Km (Glc.) = 30.1 mM
Vmax (Glc.) = 131.6 U/mg protein

| U/mg | SmM | 10mM | 20mM |
|---|---|---|---|
| Glucose | 21.89 (100.0%) | 28.69 (131.0%) | 52.39 (239.3%) |
| Maltose | 0.06 (0.3%) | 0.13 (0.6%) | 0.29 (1.3%) |

**QYA**
Km (Glc.) = 16.1 mM
Vmax (Glc.) = 149.3 U/mg protein

| U/mg | 5mM | 10mM | 20mM |
|---|---|---|---|
| Glucose | 34.91 (100.0%) | 56.61 (162.1%) | 83.94 (240.5%) |
| Maltose | 0.40 (1.1%) | 0.64 (1.8%) | 1.45 (4.1%) |

**QYR**
Km (Glc.) = 11.2 mM
Vmax (Glc.) = 93.5 U/mg protein

| U/mg | 5mM | 10mM | 20mM |
|---|---|---|---|
| Glucose | 30.00 (100.0%) | 42.68 (142.3%) | 59.73 (199.1%) |
| Maltose | 0.32 (1.1%) | 0.59 (2.0%) | 1.09 (3.6%) |

(continued)

**QYD**
Km (Glc.) = 24.9 mM
Vmax (Glc.) = 64.1 U/mg protein

| U/mg | 5mM | 10mM | 20mM |
|---|---|---|---|
| Glucose | 10.26 (100.0%) | 18.78 (183.1%) | 29.32 (285.8%) |
| Maltose | 0.02 (0.2%) | 0.18 (1.7%) | 0.31 (3.0%) |

**QYP**
Km (Glc.) = 35.5 mM
Vmax (Glc.) = 74.1 U/mg protein

| U/mg | 5mM | 10mM | 20mM |
|---|---|---|---|
| Glucose | 9.53 (100.0%) | 16.78 (176.0%) | 25.23 (264.8%) |
| Maltose | 0.09 (0.9%) | 0.13 (1.4%) | 0.15 (1.6%) |

**QYG**
Km (Glc.) = 15.7 mM
Vmax (Glc.) = 59.2 U/mg protein

| U/mg | 5mM | 10mM | 20mM |
|---|---|---|---|
| Glucose | 12.92 (100.0%) | 23.54 (182.2%) | 31.56 (244.3%) |
| Maltose | 0.18 (1.4%) | 0.25 (1.9%) | 0.38 (3.0%) |

**QYQ**
Km (Glc.) = 15.7 mM
Vmax (Glc.) = 65.4 U/mg protein

| U/mg | 5mM | 10mM | 20mM |
|---|---|---|---|
| Glucose | 15.36 (100.0%) | 24.94 (162.4%) | 38.55 (251.0%) |
| Maltose | 0.21 (1.4%) | 0.42 (2.7%) | 1.03 (6.7%) |

**QYK**
Km (Glc.) = 10.8 mM
Vmax (Glc.) = 78.7 U/mg protein

| U/mg | 5mM | 10mM | 20mM |
|---|---|---|---|
| Glucose | 28.02 (100.0%) | 33.68 (120.2%) | 52.64 (187.8%) |
| Maltose | 0.48 (1.7%) | 0.65 (2.3%) | 1.23 (4.4%) |

**QYT**
Km (Glc.) = 36.3 mM
Vmax (Glc.) = 88.5 U/mg protein

| U/mg | 5mM | 10mM | 20mM |
|---|---|---|---|
| Glucose | 9.68 (100.0%) | 20.44 (211.3%) | 32.87 (339.7%) |
| Maltose | 0.14 (1.5%) | 0.21 (2.2%) | 0.33 (3.4%) |

**QYL**
Km (Glc.) = 13.4 mM
Vmax (Glc.) = 94.3 U/mg protein

| U/mg | 5mM | 10mM | 20mM |
|---|---|---|---|
| Glucose | 23.95 (100.0%) | 37.28 (155.7%) | 57.03 (238.1%) |
| Maltose | 0.23 (1.0%) | 0.34 (1.4%) | 0.66 (2.8%) |

**QYV**
Km (Glc.) = 18.3 mM
Vmax (Glc.) = 108.7 U/mg protein

| U/mg | 5mM | 10mM | 20mM |
|---|---|---|---|
| Glucose | 27.25 (100.0%) | 35.53 (130.4%) | 54.04 (198.3%) |
| Maltose | 0.15 (0.6%) | 0.32 (1.2%) | 0.80 (2.9%) |

(DCIP f.c. 0.06 mM; PMS f.c 6 mM, 10 mM PPB pH 7.0)

[Example 8] Production of Colorimetric Sensor for Measuring Blood Sugar Level Using Mutant CyGDH

With respect to the QYY, which had the best specific activity as described above, a glucose sensor was produced using the purified mutant enzyme obtained in the Example 5.

A glucose sensor having the basic structure as shown in Fig. 1 was produced. That is to say, this glucose sensor has a configuration wherein the transparent cover 4 (material: PET) is laminated on the transparent base plate 2 via the spacer 3, and the capillary 5 is defined by these elements 2 to 4. The dimension of the capillary 5 is 1.3 mm x 9 mm x 50 μm (Fig. 1). The transparent base plate 2 and the transparent cover 4 were formed with PET having a thickness of 250 μm, and the spacer 3 was formed with black double-sided tape.

The glucose sensor has first to third reagent parts as shown in Fig. 2. Ingredients and coating amounts for each reagent part are shown in Table 9. In this Table, "Ru" represents a ruthenium hexaammine complex ($Ru(NH_3)_6Cl_3$); ACES represents *N*-(2-acetamido)-2-aminoethanesulfonic acid; WST-4 represents 2-**Benzothiazolyl-3-(4-carboxy-2-methoxyphenyl)-5-[4-(2-**sulfoethylcarbamoyl)phenyl]-2*H*-tetrazolium; and SWN represents Lithium Magnesium Sodium Silicate.

[Table 9]

**First reagent part**

**Material solution for reagent part containing electron transfer substance (solvent is water)**

| Ru | Coating amount |
|---|---|
| 60 mM | 0.5 µl |

**Second reagent part**

**Material solution for reagent part containing enzyme (solvent is water)**

| Enzyme concentration | Raffinose | Sucrose monolaurate | ACES (pH 7.5) | Coating amount |
|---|---|---|---|---|
| 12 KU/ml | 1.0% | 0.1% | 100 mM | 0.2µl |

**Third reagent part**

**Material solution for reagent part containing color developer (solvent is water)**

| WST-4 | SWN | Coating amount |
|---|---|---|
| About 50 mM | 0.16% | 0.4 µl |

Each sample to be measured was supplied to the capillary of the glucose sensor as mentioned above, and, at 5 seconds after that, absorbance of the end point was plotted. In these absorbance measurements, the third reagent part was irradiated with light along the height direction of the capillary, and, at that time, the light transmitted through the glucose sensor was received. The light irradiation was performed by irradiating with 630 nm light using a light emitting diode. The transmitted light was received with a photodiode.

As for the influence of maltose, when maltose was added to 50 mg/dl of glucose, the absorbance in the case of the wild-type CyGDH was largely increased depending on the maltose concentration, which suggests that the wild-type CyGDH reacts strongly with maltose. On the other hand, with respect to the sensor that uses the mutant CyGDH (QY), it was found that the absorbance increase depending on the maltose concentration is suppressed, and thus the influence of maltose is reduced. With respect to the sensor that uses the mutant CyGDH (QYY), it was found that the absorbance increase which is dependent on the maltose concentration is further suppressed, and thus the influence of maltose is further reduced. These data were converted into apparent blood sugar elevation values, and the results are shown in Fig. 3.

In the sensor that uses the wild-type enzyme, a low blood sugar level (50 mg/dl of glucose) is apparently indicated as a level above the normal range (174.8 mg/dl of glucose) due to the presence of maltose (ratio of change: 300%). Even in the sensor that uses the modified CyGDH (QY), the low blood sugar level is also apparently indicated as 64.8 mg/dl of glucose due to the presence of maltose (ratio of change: 54%). On the other hand, in the sensor that uses the modified CyGDH (QYY), even if maltose is present in an amount up to 300 mg/dl, the apparent blood sugar level is elevated only up to at most 46.1 mg/dl, and therefore it can be said that the influence is significantly suppressed (ratio of change: 14%). In conclusion, it is suggested that the QYY mutant is most suitable from the point of view of the reactivity to glucose (linearity) and influence of maltose.

As is clear from the above results, in the glucose sensor that uses the mutant CyGDH (QYY), the reactivity with maltose is largely reduced compared to the glucose sensor that uses the mutant CyGDH (QY). If this glucose sensor that uses the mutant CyGDH (QYY) is used, then it can be said that there will be no difference between right and wrong at 200 mg/dl of maltose, which is the upper limit of blood maltose level that can be administered in a hospital or the like; and, even at 300 mg/dl of maltose, which is above the upper limit, low blood sugar levels (50 mg/dl or less) are not judged as normal levels or high blood sugar levels, thereby allowing for safe therapeutic treatments.

[Example 9] Production of Electrode Sensor for Measuring Blood Sugar Level Using Mutant CyGDH

With respect to the QYY, which had the best specific activity as described above, an electrode glucose sensor was produced using the purified mutant enzyme obtained in the Example 5.

(Procedure for Producing)

The method of producing an electrode glucose sensor is shown below with reference to Fig. 5. A base plate made of PET (28 mm in length, 7 mm in width, and 250 μm in thickness) was prepared as the insulating base plate 1, and, on one surface thereof, a carbon electrode system consisting of the working electrode 4 and the counter electrode 5, which had the lead parts 2 and 3, respectively, was formed by screen printing of carbon ink. Then, an insulating paste was prepared and screenprinted on the above-mentioned electrodes to form the insulating layer 6. By this screen printing, the parts wherein the insulating layer was not formed were obtained as the detecting part and the lead part, respectively. Then, 0.6 g of "Lucentite SWN" which is a trade name of a synthetic smectite (manufactured by Co-op Chemical Co., Ltd.) was suspended in 100 mL of purified water, and the resultant suspension was stirred for about 8 to 24 hours. To 10 mL of this suspension of the synthetic smectite, 0.1 mL of 10% (w/v) aqueous solution of CHAPS (manufactured by DOJINDO LABORATORIES), 5.0 mL of 1.0 M ACES buffer solution (pH 7.4, manufactured by DOJINDO LABORATORIES) and 4.0 mL of purified water were added in the order mentioned. Further, the resultant solution was mixed with 1.0 g of $[Ru(NH_3)_6]Cl_3$ (manufactured by Aldrich) as a mediator. To the detecting part, 1.0 μL of this mixture was dispensed, and the dispensed mixture was dried under the conditions of 30°C and 10% of relative humidity for 10 minutes to form the reagent layer 7. Further, the enzyme reagent layer 8 was formed on this reagent layer 7. This enzyme reagent layer 8 was formed by dispensing 1.0 μL of 2700 U/mL aqueous solution of the glucose dehydrogenase of the present invention onto the reagent layer of the detecting part, and drying the dispensed solution under the conditions of 30°C and 10% of relative humidity for 10 minutes. Finally, the spacer 11 having the slit 15 was placed on the insulating layer, and the cover 12 having a penetrating pore, i.e., the air hole 14, was further placed on this spacer to produce a biosensor.

As for the influence of maltose, when maltose is added to 50 mg/dl of glucose, the electric current value in the case of the wild-type CyGDH was largely increased depending on the maltose concentration, which suggests that the wild-type CyGDH reacts strongly with maltose. On the other hand, with respect to the sensor that uses the mutant CyGDH (QY), it was found that the electric current value increase which is dependent on the maltose concentration is suppressed, and thus the influence of maltose is reduced. With respect to the sensor that uses the mutant CyGDH (QYY), it was found that the electric current value increase dependent on the maltose concentration is further suppressed, and thus the influence of maltose is further reduced. These data were converted into apparent blood sugar elevation values, and the results are shown in Fig. 4.

In the sensor that uses the wild-type enzyme, a low blood sugar level (50 mg/dl of glucose) is apparently indicated as a level above the normal range (288.2 mg/dl of glucose) due to the presence of maltose (ratio of change: 549%). Even in the sensor that uses the modified CyGDH (QY), the low blood sugar level is also apparently indicated as 93.9 mg/dl of glucose due to the presence of maltose (ratio of change: 114%). On the other hand, in the sensor that uses the modified CyGDH (QYY), even if maltose is present in an amount up to 300 mg/dl, the apparent blood sugar level is elevated only up to at most 60.0 mg/dl, and therefore it can be said that the influence is significantly suppressed (ratio of change: 37%). In conclusion, it is suggested that the QYY mutant is most suitable from the point of view of the reactivity to glucose (linearity) and influence of maltose.

As is clear from the above results, in the glucose sensor that uses the mutant CyGDH (QYY), the reactivity with maltose is largely reduced compared to the glucose sensor that uses the mutant CyGDH (QY). If this glucose sensor that uses the mutant CyGDH (QYY) is used, then it can be said that there will be no difference between right and wrong at 200 mg/dl of maltose, which is the upper limit of blood maltose level that can be administered in a hospital or the like; and, even at 300 mg/dl of maltose, which is above the upper limit, low blood sugar levels (50 mg/dl or less) are not judged as normal levels or high blood sugar levels, thereby allowing for safe therapeutic treatments.

[Example 10] Introducing Mutations into GDH Homologues

Plasmids expressing each α- and γ-subunits of a putative oxidoreductase of *Burkholderia cenocepacia* J2315 strain, a hypothetical protein BthaT_07876 of *Burkholderia thailandensis* TXDOH strain, a FAD dependent oxidoreductase of *Ralstonia pickettii* 12D strain, a transmembrane dehydrogenase of *Ralstonia solanacearum* IPO1609 strain and a glucose-methanol-choline oxidoreductase of *Burkholderia phytofirmans* PsJN strain were prepared in the same manner as in Example 1.

These expression plasmids are plasmids wherein a DNA fragment contiguously containing a γ-subunit structural gene and an α-subunit structural gene is inserted into the NdeI/HindIII site, which is a cloning site of the vector pET30c(+) (Novagen). The γα gene in this plasmid is regulated by the T7 promoter. This plasmid has a kanamycin resistance gene.

Using a commercially available site-specific mutagenesis kit (Stratagene, QuikChangeII Site-Directed Mutagenesis Kit), mutations were introduced into the α-subunit gene contained in the above-mentioned plasmids so that residues corresponding to positions 326, 365 and 472 of the wild-type GDH α-subunit of *Burkholderia cepacia* KS1 strain were replaced with glutamine, tyrosine and tyrosine, respectively.

The sequences of forward primers used for these amino acid residue substitutions are shown below. The sequences of reverse primers were strands fully complementary to these forward primers.

In the notations that represent the mutations, the numerals represent positions in the amino acid sequences, the letters before the numerals represent amino acid residues before the amino acid substitutions, and the letters after the numerals represent amino acid residues after the amino acid substitutions. For example, R53F indicates that arginine at position 53 is replaced with phenylalanine.

PCR was carried out with the following reaction composition by performing a reaction of 95°C for 30 seconds; then repeating 15 times a cycle of 95°C for 30 seconds, 55°C for 1 minute and 68°C for 8 minutes; performing a reaction of 68°C for 30 minutes; and then maintaining at 4°C.

[Composition of the Reaction Solution]

| | |
|---|---|
| Template DNA (5 ng/μl) | 2 μl |
| (pTrc99A/γ+α or pTrc99Aγαβ with 3 mutations introduced) | |
| 10 x Reaction Buffer Solution | 5 μl |
| Forward Primer (100 ng/μl) | 1.25 μl |
| Reverse Primer (100 ng/μl) | 1.25 μl |
| dNTPs | 1 μl |
| Distilled Water | 38.5 μl |
| DNA Polymerase | 1 μl |
| Total | 50 μl |

After the PCR, 0.5 μl of DpnI was added to the reaction solution, and the resultant mixture was incubated at 37°C for 1 hour to degrade the template plasmid.

Competent cells of *Escherichia coli* DH5α (supE44, ΔlacU 169 (φ80lacZΔM15), hsdR17, recAi, endA1, gyrA96, thi-1, relA1) were transformed using the obtained reaction solution. Each plasmid DNA was prepared from several colonies that had been grown on LB agar medium (bacto tryptone 1%, yeast extract 0.5%, sodium chloride 1% and agar 1.5%) containing ampicillin (50 μg/ml) and kanamycin (30 μg/ml), and the sequence thereof was analyzed to confirm that mutations of interest were introduced into each α-subunit gene.

[Table 10]

Forward Primers for Introducing Mutations into Putative Oxidoreductase of *Burkholderia cenocepacia* J2315 Strain

| Amino acid substituion | Sequence | SEQ ID NOs. |
|---|---|---|
| S326Q | 5'GGGCACCGGCGTGCAGTTCTATGCGAACGAG 3' | SEQ ID NO:58 |
| S365Y | 5'GAAGAAGATCCACCTGTACAACATGTCGCGCATCAAC3' | SEQ ID NO:59 |
| A472Y | 5'GTCGTGTTCAACGACGAATTCTACCCGAACAATCACATC ACGGG 3' | SEQ ID NO:60 |

Reverse Primers for Introducing Mutations into Putative Oxidoreductase of *Burkholderia cenocepacia* J2315 Strain

| Amino acid substituion | Sequence | SEQ ID NOs. |
|---|---|---|
| S326Q | 5'CTCGTTCGCATAGAACTGCACGCCGGTGCCC 3' | SEQ ID NO:61 |
| S365Y | 5'GTTGATGCGCGACATGTTGTACAGGTGGATCTTCTTC 3' | SEQ ID NO:62 |
| A472Y | 5'CCCGTGATGTGATTGTTCGGGTAGAATTCGTCGTTGAAC ACGAC 3' | SEQ ID NO:63 |

(continued)

Forward Primers for Introducing Mutations into Hypothetical Protein BthaT_07876 of *Burkholderia thailandensis* TXDOH Strain

| Amino acid substituion | Sequence | SEQ ID NOs. |
|---|---|---|
| S324Q | 5'GGGCACCGGCGTGCAGTTCTATGCGAGCGAG 3' | SEQ ID NO:64 |
| S363Y | 5'GAAGAAGATCCACCTGTACAACCTGTCGCGCATCGAC 3' | SEQ ID NO:65 |
| A470Y | 5'GTCGTGTTCAACGACGAATTCTACCCGAACAATCACATC ACGGG 3' | SEQ ID NO:60 |

Reverse Primers for Introducing Mutations into Hypothetical Protein BthaT_07876 of *Burkholderia thailandensis* TXDOH Strain

| Amino acid substituion | Sequence | SEQ ID NOs. |
|---|---|---|
| S324Q | 5'CTCGCTCGCATAGAACTGCACGCCGGTGCCC 3' | SEQ ID NO:66 |
| S363Y | 5'GTCGATGCGCGACAGGTTGTACAGGTGGATCTTCTTC 3' | SEQ ID NO:67 |
| A470Y | 5'CCCGTGATGTGATTGTTCGGGTAGAATTCGTCGTTGAAC ACGAC 3' | SEQ ID NO:63 |

Forward Primers for Introducing Mutations into FAD Dependent Oxidoreductase of *Ralstonia pickettii* 12D Strain

| Amino acid substituion | Sequence | SEQ ID NOs. |
|---|---|---|
| S327Q | 5'GGGCACCGGCGTGCAGTTCTATGCGGACCGC 3' | SEQ ID NO:68 |
| S366Y | 5'CAAGAAGCTGCACCTGTACAACATCTCGCGCATCGAG 3' | SEQ ID NO:69 |
| A473Y | 5'GTCGAGTTCCACGACGACTTCTACCCGAACAATCACATC ACGGG 3' | SEQ ID NO:70 |

Reverse Primers for Introducing Mutations into FAD Dependent Oxidoreductase of *Ralstonia pickettii* 12D Strain

| Amino acid substituion | Sequence | SEQ ID NOs. |
|---|---|---|
| S327Q | 5'GCGGTCCGCATAGAACTGCACGCCGGTGCCC 3' | SEQ ID NO:71 |
| S366Y | 5'CTCGATGCGCGAGATGTTGTACAGGTGCAGCTTCTTG 3' | SEQ ID NO:72 |
| A473Y | 5'CCCGTGATGTGATTGTTCGGGTAGAAGTCGTCGTGGAAC TCGAC 3' | SEQ ID NO:73 |

Forward Primers for Introducing Mutations into Transmembrane Dehydrogenase of *Ralstonia solanacearum* IPO1609 Strain

| Amino acid substituion | Sequence | SEQ ID NOs. |
|---|---|---|
| S327Q | 5'GGGCACCGGCGTGCAGTTCTATGCGGACCGC 3' | SEQ ID NO:68 |
| S366Y | 5'CAAGAAGCTGCACCTGTACAACATCTCGCGCATCGAG 3' | SEQ ID NO:69 |
| A473Y | 5'GTCCAGTTCCACGACGACTTCTACCCGAACAATCACATC ACGGG 3' | SEQ ID NO:74 |

(continued)

Reverse Primers for Introducing Mutations into Transmembrane Dehydrogenase of *Ralsionia solanacearum* IPO1609 Strain

| Amino acid substituion | Sequence | SEQ ID NOs. |
|---|---|---|
| S327Q | 5'GCGGTCCGCATAGAACTGCACGCCGGTGCCC 3' | SEQ ID NO:71 |
| S366Y | 5'CTCGATGCGCGAGATGTTGTACAGGTGCAGCTTCTTG 3' | SEQ ID NO:72 |
| A473Y | 5'CCCGTGATGTGATTGTTCGGGTAGAAGTCGTCGTGGAAC TGGAC 3' | SEQ ID NO:75 |

Forward Primers for Introducing Mutations into Glucose-Methanol-Choline Oxidoreductase of *Burkholderia phytofirmans* PsJN Strain

| Amino acid substituion | Sequence | SEQ ID NOs. |
|---|---|---|
| S322Q | 5'GGGCACCGGCGTGCAGTTCCTGGCGAACGAG 3' | SEQ ID NO:76 |
| S361Y | 5'GAAGAAGCTGCACCTGTACAACGGCGTCCCGACGATG 3' | SEQ ID NO:77 |
| A466Y | 5'GTCACGTTCGACGACACGTTCTACCCGAACAATCACATC ATGGG 3' | SEQ ID NO:78 |

Reverse Primers for Introducing Mutations into Glucose-Methanol-Choline Oxidoreductase of *Burkholderia phytofirmans* PsJN Strain

| Amino acid substituion | Sequence | SEQ ID NOs. |
|---|---|---|
| S322Q | 5'CTCGTTCGCCAGGAACTGCACGCCGGTGCCC 3' | SEQ ID NO:79 |
| S361Y | 5'CATCGTCGGGACGCCGTTGTACAGGTGCAGCTTCTTC 3' | SEQ ID NO:80 |
| A466Y | 5'CCCATGATGTGATTGTTCGGGTAGAACGTGTCGTCGAAC GTGAC 3' | SEQ ID NO:81 |

[Example 11] Analysis of Substrate Specificities of Mutant Enzymes

Mutant enzymes were produced by using the plasmids expressing mutant enzymes which were obtained in the Example 10, and their substrate specificities were studied.

(1) Culture

Competent cells of *Escherichia coli* BL21 (DE3) (F-, dcm, ompT, hsdS (rB-mB-), gal, λ (DE3)) were transformed with each of the plasmids expressing mutant enzymes. The obtained transformants were each cultured under shaking at 37°C overnight in 3 ml of LB medium (containing 25 μg/ml of kanamycin) by using a L-shaped tube. These cultures were inoculated into a 500 ml Sakaguchi flask that contained 100 ml of LB medium (containing 0.5% glycerol, 0.05% glucose, 0.2% lactose, 100 mM $PO_4$, 25 mM $SO_4$, 50 mM $NH_4$, 100 mM Na, 50 mM K, 1 mM $MgSO_4$ and 25 μg/ml of kanamycin), and the resultant cultures were cultured under shaking at 20°C for 24 hours.

(2) Preparing Crude Enzyme Samples

The bacterial cells were collected from the cultures which had been cultured as described above, and the collected cells were washed. Thereafter, the bacterial cells were suspended with 1 ml of 10 mM potassium phosphate buffer (PPB) (pH 7.0) per 0.1 g of wet bacterial cells, and sonicated. These suspensions were centrifuged (10000 r.p.m, 10 minutes, 4°C) to remove the residues. Thereafter, the supernatants were ultracentrifuged (50,000 r.p.m., 60 minutes, 4°C), and the obtained supernatants (water-soluble fractions) were considered as crude enzyme samples.

(3) Measurement of GDH Activity

To 10 μl of each crude enzyme sample as mentioned above, 170 μl of a reagent for measuring the activity (a solution that was obtained by adding 10 mM PPB to 94 μl of 600 mM methylphenazine methosulfate (PMS) and 9.4 μl of 6 mM 2,6-dichlorophenolindophenol (DCIP) so as to attain a total volume of 8 ml) was added. To the obtained mixture, 20 μl of each concentration of substrate (glucose or maltose) or distilled water was added. The resultant solution was stirred, and absorbance at 600 nm, which is an absorption wavelength of DCIP, was measured by using a spectrophotometer. The final concentrations of the reagents DCIP and PMS were 0.06 mM and 0.6 mM, respectively.

The results are shown in Table 11. In all of these mutant enzymes, it was confirmed that the reactivities to maltose were reduced and the substrate specificities to glucose were improved compared to the wild-type enzymes before introduction of the mutation.

[Table 11]

| | | 10mM Glc (U/mL) | 10mM Mal (U/mL) | 10mM Mal/Glc | 5mM Glc (U/mL) | 5mM Mal (U/mL) | 5mM Mal/Glc | Glc Km (mM) | Glc Vmax (U/mL) | Glc Vmax/Km | Mal Km (mM) | Mal Vmax (U/mL) | Mal Vmax/Km |
|---|---|---|---|---|---|---|---|---|---|---|---|---|---|
| *B.cenocepacia* J2315 putative oxidoreductase | WT | 2.02 | 1.64 | 80.8% | 2.02 | 1.48 | 72.9% | 0.11 | 2.05 | 18.6 | 1.3 | 1.87 | 1.44 |
| | QYY | 11.4 | 0.31 | 2.7% | 7.59 | 0.15 | 2.0% | 20.3 | 39.2 | 1.93 | >100 | - | - |
| *B.thailandensis* TXDOH hypothetical protein BthaT_07876 | WT | 20.1 | 12.0 | 59.8% | 19.6 | 9.58 | 49.0% | 0.31 | 20.7 | 66.8 | 2.67 | 15.5 | 5.81 |
| | QYY | 20.7 | 0.22 | 1.1% | 13.6 | 0.13 | 1.0% | 13.0 | 48.8 | 3.75 | >100 | - | - |
| *R.pickettii* 12D FAD dependent oxidoreductase | WT | 0.94 | 0.78 | 82.5% | 0.89 | 0.68 | 76.6% | 0.36 | 1.03 | 2.86 | 2.02 | 0.94 | 0.47 |
| | QYY | 0.73 | 0.03 | 3.7% | 0.50 | 0.02 | 3.0% | 11.0 | 1.62 | 0.15 | >100 | - | - |
| *R.solanacearum* IPO1609 transmembrane dehydrogenase | QT | 0.13 | 0.11 | 83.6% | 0.12 | 0.09 | 74.7% | 0.59 | 0.14 | 0.24 | 2.64 | 0.14 | 0.05 |
| | QYY | 0.58 | 0.01 | 2.4% | 0.40 | 0.01 | 2.2% | 13.9 | 1.55 | 0.11 | >100 | - | - |

From the results as described above, it is suggested that the QYY mutation is also useful for GDH homologues of strains other than *Burkholderia cepacia*.

Industrial Applicability

The mutant GDHs of the present invention show improved substrate specificities to glucose, and can be suitably used for measuring glucose using a glucose sensor or the like.

## Claims

1. A mutant glucose dehydrogenase having an amino acid sequence at least 80% identical to SEQ ID NO:3 and having glucose dehydrogenase activity,
wherein amino acid residues in said mutant glucose dehydrogenase corresponding to positions 326, 365 and 472 of SEQ ID NO:3 are replaced with glutamine, tyrosine and tyrosine, respectively, and
wherein said mutant glucose dehydrogenase shows an improved substrate specificity to glucose and a reduced reactivity to maltose relative to the glucose dehydrogenase with an amino acid sequence as set forth in SEQ ID NO:3, wherein said substrate specificity to glucose is improved by at least 20% and reactivity to maltose is 1% or less of the reactivity to glucose.

2. The mutant glucose dehydrogenase according to claim 1, which has an amino acid sequence at least 90% identical to SEQ ID NO:3.

3. The mutant glucose dehydrogenase according to claim 1 or 2, which has the amino acid sequence of SEQ ID NO:3 except for the positions corresponding to positions 326, 365 and 472.

4. The mutant glucose dehydrogenase according to claim 1 or 2, which has the amino acid sequence of SEQ ID NO:7 except for the positions corresponding to positions 326, 365 and 472 of SEQ ID NO:3.

5. The mutant glucose dehydrogenase according to claim 1 or 2, which has the amino acid sequence of SEQ ID NO:8 except for the positions corresponding to positions 326, 365 and 472 of SEQ ID NO:3.

6. The mutant glucose dehydrogenase according to claim 1 or 2, which has the amino acid sequence of SEQ ID NO:9 except for the positions corresponding to positions 326, 365 and 472 of SEQ ID NO:3.

7. The mutant glucose dehydrogenase according to claim 1 or 2, which has the amino acid sequence of SEQ ID NO:10 except for the positions corresponding to positions 326, 365 and 472 of SEQ ID NO:3.

8. The mutant glucose dehydrogenase according to any one of claims 1 to 7, wherein said mutant glucose dehydrogenase shows substrate specificity to glucose improved by at least 20% and a reduced reactivity to maltose as compared to the glucose dehydrogenase with an amino acid sequence as set forth in SEQ ID NO:3 except that the amino acid residues in said glucose dehydrogenase corresponding to positions 326 and 365 of SEQ ID NO:3 are replaced with glutamine and tyrosine, respectively.

9. A mutant glucose dehydrogenase complex comprising at least the mutant glucose dehydrogenase according to any one of claims 1 to 8 and an electron transfer subunit.

10. The glucose dehydrogenase complex according to claim 9, wherein the electron transfer subunit is cytochrome C.

11. A DNA molecule coding for the mutant glucose dehydrogenase according to any one of claims 1 to 8.

12. A microorganism comprising the DNA molecule according to claim 11 and producing the mutant glucose dehydrogenase according to any one of claims 1 to 8 or the mutant glucose dehydrogenase complex according to claim 9 or 10.

13. A glucose assay kit comprising the mutant glucose dehydrogenase according to any one of claims 1 to 8, the mutant glucose dehydrogenase complex according to claim 9 or 10, or the microorganism according to claim 12.

14. A glucose sensor comprising the mutant glucose dehydrogenase according to any one of claims 1 to 8, the mutant glucose dehydrogenase complex according to claim 9 or 10, or the microorganism according to claim 12.

**Patentansprüche**

1. Eine mutante Glucosedehydrogenase mit einer Aminosäuresequenz, die zumindest zu 80% identisch mit SEQ ID NR.: 3 ist, und mit Glucosedehydrogenaseaktivität,
wobei Aminosäurereste in der besagten mutanten Glucosedehydrogenase entsprechend den Positionen 326, 365 und 472 von SEQ ID NR.: 3 mit Glutamin, Tyrosin beziehungsweise Tyrosin ersetzt werden und
wobei die besagte mutante Glucosedehydrogenase eine verbesserte Substratspezifität zu Glucose und eine reduzierte Reaktivität auf Maltose bezüglich der Glucosedehydrogenase mit einer Aminosäuresequenz, wie in SEQ ID NR.: 3 dargelegt, zeigt, wobei die besagte Substratspezifität zu Glucose um zumindest 20% verbessert ist und die Reaktivität auf Maltose 1 % oder weniger der Reaktivität auf Glucose ist.

2. Die mutante Glucosedehydrogenase gemäß Anspruch 1, welche eine Aminosäuresequenz hat, die zumindest zu 90% identisch mit SEQ ID NR.: 3 ist.

3. Die mutante Glucosedehydrogenase gemäß Anspruch 1 oder 2, welche bis auf die Positionen entsprechend den Positionen 326, 365 und 472 die Aminosäuresequenz von SEQ ID NR.: 3 hat.

4. Die mutante Glucosedehydrogenase gemäß Anspruch 1 oder 2, welche bis auf die Positionen entsprechend den Positionen 326, 365 und 472 von SEQ ID NR.: 3 die Aminosäuresequenz von SEQ ID NR.: 7 hat.

5. Die mutante Glucosedehydrogenase gemäß Anspruch 1 oder 2, welche bis auf die Positionen entsprechend den Positionen 326, 365 und 472 von SEQ ID NR.: 3 die Aminosäuresequenz von SEQ ID NR.: 8 hat.

6. Die mutante Glucosedehydrogenase gemäß Anspruch 1 oder 2, welche bis auf die Positionen entsprechend den Positionen 326, 365 und 472 von SEQ ID NR.: 3 die Aminosäuresequenz von SEQ ID NR.: 9 hat.

7. Die mutante Glucosedehydrogenase gemäß Anspruch 1 oder 2, welche bis auf die Positionen entsprechend den Positionen 326, 365 und 472 von SEQ ID NR.: 3 die Aminosäuresequenz von SEQ ID NR.: 10 hat.

8. Die mutante Glucosedehydrogenase gemäß einem der Ansprüche 1 bis 7, wobei die besagte mutante Glucosedehydrogenase eine um zumindest 20% verbesserte Substratspezifität zu Glucose und eine reduzierte Reaktivität auf Maltose bezüglich der Glucosedehydrogenase mit einer Aminosäuresequenz, wie in SEQ IS NR.: 3 dargelegt, zeigt, außer dass die Aminosäurereste in der besagten Glucosedehydrogenase entsprechend den Positionen 326 und 365 von SEQ ID NR.: 3 mit Glutamin beziehungsweise Tyrosin ersetzt werden.

9. Ein mutante Glucosedehydrogenase-Komplex, der zumindest die mutante Glucosedehydrogenase gemäß einem der Ansprüche 1 bis 8 und eine Elektronentransferuntereinheit aufweist.

10. Der mutante Glucosedehydrogenase-Komplex gemäß Anspruch 9, wobei die Elektronentransferuntereinheit Cytochrom C ist.

11. Eine DNA-Molekülkodierung für die mutante Glucosedehydrogenase gemäß einem der Ansprüche 1 bis 8.

12. Ein Mikroorganismus, der das DNA-Molekül gemäß Anspruch 11 aufweist und der die mutante Glucosedehydrogenase gemäß einem der Ansprüche 1 bis 8 oder den mutante Glucosedehydrogenase-Komplex gemäß Anspruch 9 oder 10 produziert.

13. Ein Glucosetestkit, das die mutante Glucosedehydrogenase gemäß einem der Ansprüche 1 bis 8, den mutante Glucosedehydrogenase-Komplex gemäß Anspruch 9 oder 10 oder den Mikroorganismus gemäß Anspruch 12 aufweist.

14. Ein Glucosesensor, der die mutante Glucosedehydrogenase gemäß einem der Ansprüche 1 bis 8, den mutante Glucosedehydrogenase-Komplex gemäß Anspruch 9 oder 10 oder den Mikroorganismus gemäß Anspruch 12 aufweist.

**Revendications**

1. Glucose déshydrogénase mutante ayant une séquence d'acides aminés identique à au moins 80 % à SEQ ID n° 3 et ayant une activité de glucose déshydrogénase,
dans laquelle des résidus d'acides aminés dans ladite glucose déshydrogénase mutante correspondant aux positions 326, 365 et 472 de SEQ ID n° 3 sont respectivement remplacés par de la glutamine, de la tyrosine et de la tyrosine, et dans laquelle ladite glucose déshydrogénase mutante présente une spécificité de substrat améliorée au glucose et une réactivité réduite au maltose quant à la glucose déshydrogénase avec une séquence d'acides aminés telle qu'exposée dans SEQ ID n° 3, dans laquelle ladite spécificité de substrat au glucose est améliorée d'au moins 20 % et la réactivité au maltose est de 1 % ou moins de la réactivité au glucose.

2. Glucose déshydrogénase mutante selon la revendication 1, qui a une séquence d'acides aminés identique à au moins 90 % à SEQ ID n° 3.

3. Glucose déshydrogénase mutante selon la revendication 1 ou 2, qui a une séquence d'acides aminés de SEQ ID n° 3 sauf pour les positions correspondant aux positions 326, 365 et 472.

4. Glucose déshydrogénase mutante selon la revendication 1 ou 2, qui a la séquence d'acides aminés de SEQ ID n° 7 sauf pour les positions correspondant aux positions 326, 365 et 472 de SEQ ID n° 3.

5. Glucose déshydrogénase mutante selon la revendication 1 ou 2, qui a la séquence d'acides aminés de SEQ ID n° 8 sauf pour les positions correspondant aux positions 326, 365 et 472 de SEQ ID n° 3.

6. Glucose déshydrogénase mutante selon la revendication 1 ou 2, qui a la séquence d'acides aminés de SEQ ID n° 9 sauf pour les positions correspondant aux positions 326, 365 et 472 de SEQ ID n° 3.

7. Glucose déshydrogénase mutante selon la revendication 1 ou 2, qui a la séquence d'acides aminés de SEQ ID n° 10 sauf pour les positions correspondant aux positions 326, 365 et 472 de SEQ ID n° 3.

8. Glucose déshydrogénase mutante selon l'une quelconque des revendications 1 à 7, dans laquelle ladite glucose déshydrogénase mutante présente une spécificité de substrat au glucose améliorée d'au moins 20 % et une réactivité réduite au maltose en comparaison à la glucose déshydrogénase avec une séquence d'acides aminés telle qu'exposée dans SEQ ID n° 3 sauf que les résidus d'acides aminés dans ladite glucose déshydrogénase correspondant aux positions 326 et 365 de SEQ ID n° 3 sont respectivement remplacés par de la glutamine et de la tyrosine.

9. Complexe de glucose déshydrogénase mutante comprenant au moins la glucose déshydrogénase mutante selon l'une quelconque des revendications 1 à 8 et une sous-unité de transfert d'électrons.

10. Complexe de glucose déshydrogénase selon la revendication 9, dans lequel la sous-unité de transfert d'électrons est un cytochrome C.

11. Molécule d'ADN codant pour la glucose déshydrogénase mutante selon l'une quelconque des revendications 1 à 8.

12. Micro-organisme comprenant la molécule d'ADN selon la revendication 11 et produisant la glucose déshydrogénase mutante selon l'une quelconque des revendications 1 à 8 ou le complexe de glucose déshydrogénase mutante selon la revendication 9 ou 10.

13. Kit de dosage de glucose comprenant la glucose déshydrogénase mutante selon l'une quelconque des revendications 1 à 8, le complexe de glucose déshydrogénase mutante selon la revendication 9 ou 10, ou le micro-organisme selon la revendication 12.

14. Capteur de glucose comprenant la glucose déshydrogénase mutante selon l'une quelconque des revendications 1 à 8, le complexe de glucose déshydrogénase mutante selon la revendication 9 ou 10, ou le micro-organisme selon

la revendication 12.

[Fig. 1]

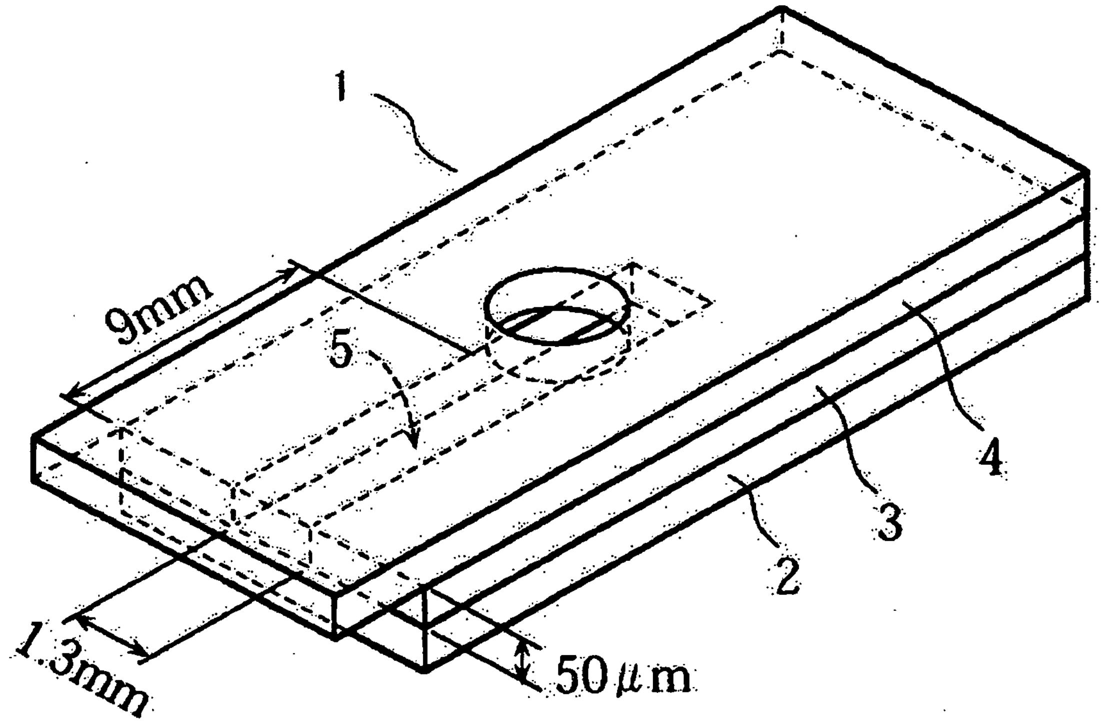


[Fig. 2]

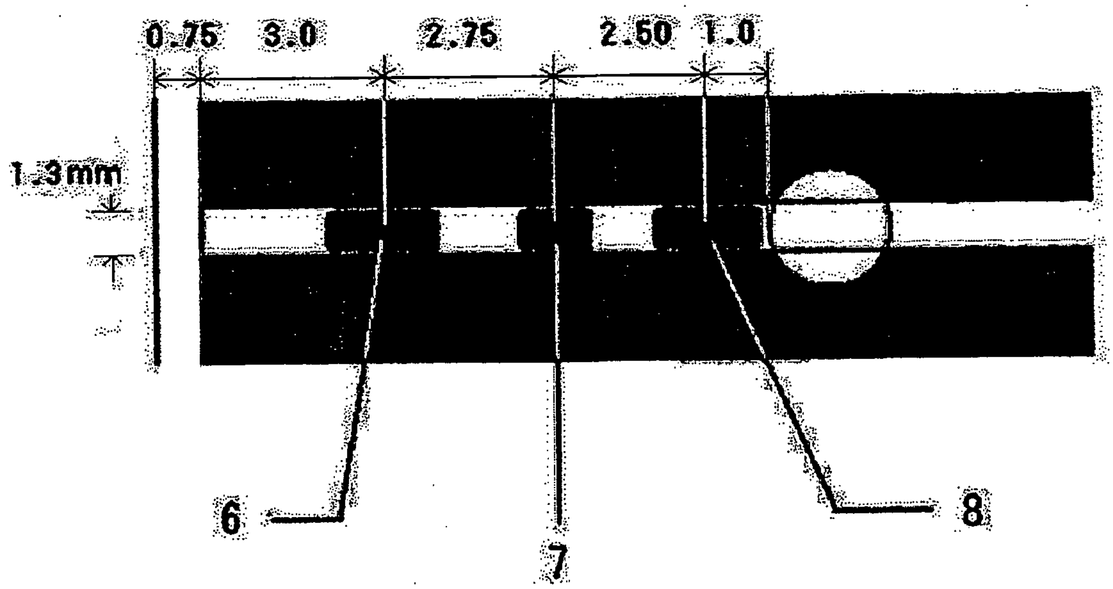

[Fig. 3]

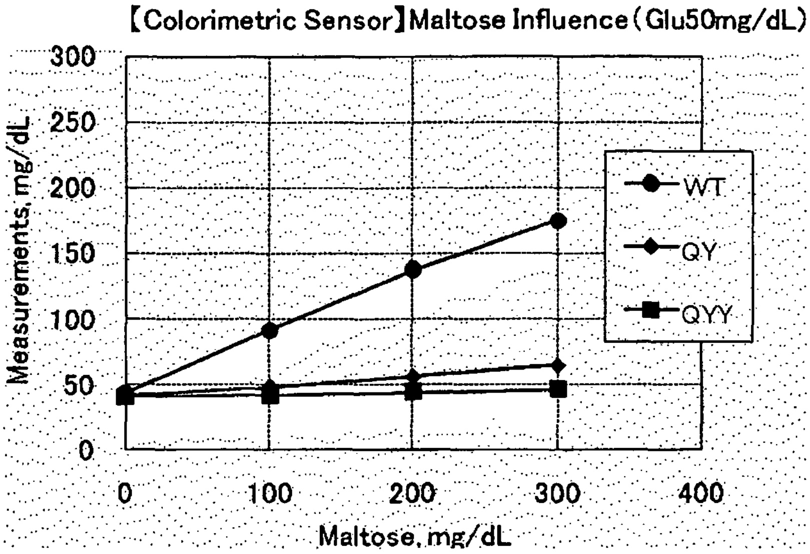
【Colorimetric Sensor】Maltose Influence(Glu50mg/dL)
300
250
200
150
100
50
0
Measurements, mg/dL
0
100
200
300
400
Maltose, mg/dL
WT
QY
QYY

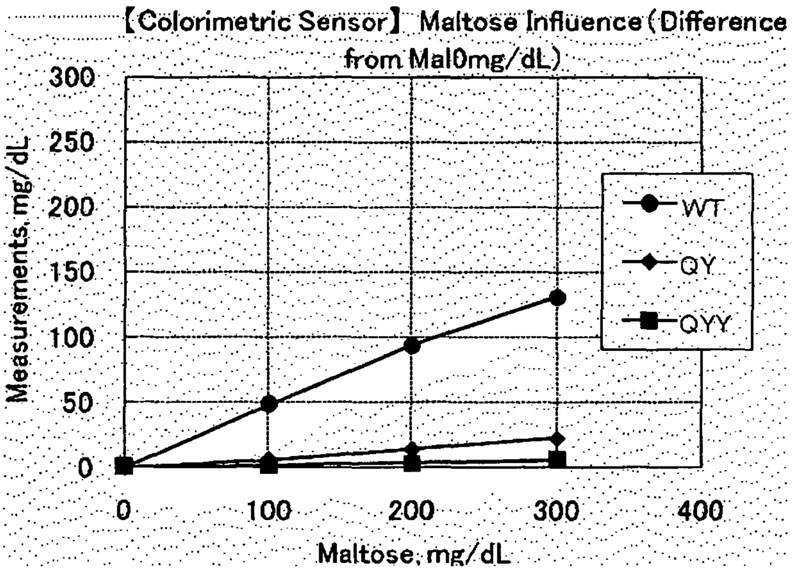
【Colorimetric Sensor】 Maltose Influence(Difference from Mal0mg/dL)
300
250
200
150
100
50
0
Measurements, mg/dL
0
100
200
300
400
Maltose, mg/dL
WT
QY
QYY

[Fig. 4]

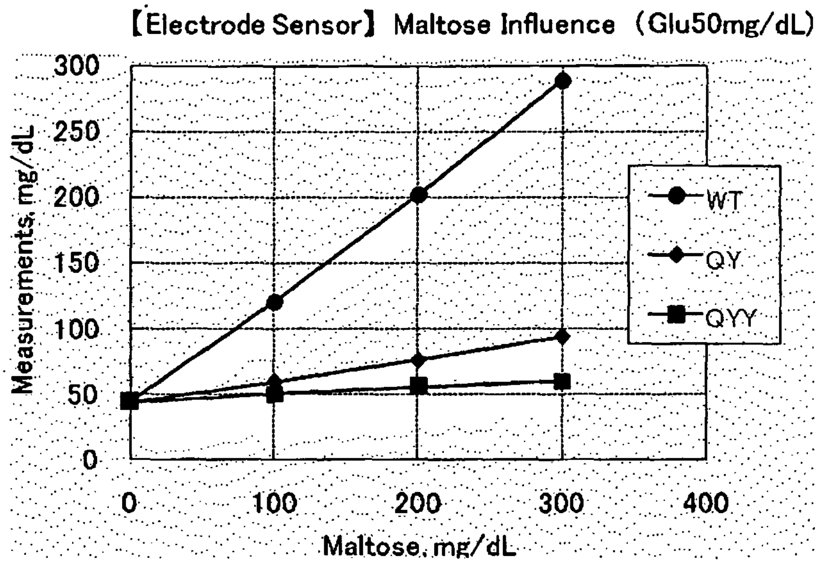
【Electrode Sensor】 Maltose Influence (Glu50mg/dL)
Measurements, mg/dL
300
250
200
150
100
50
0
0
100
200
300
400
Maltose, mg/dL
WT
QY
QYY

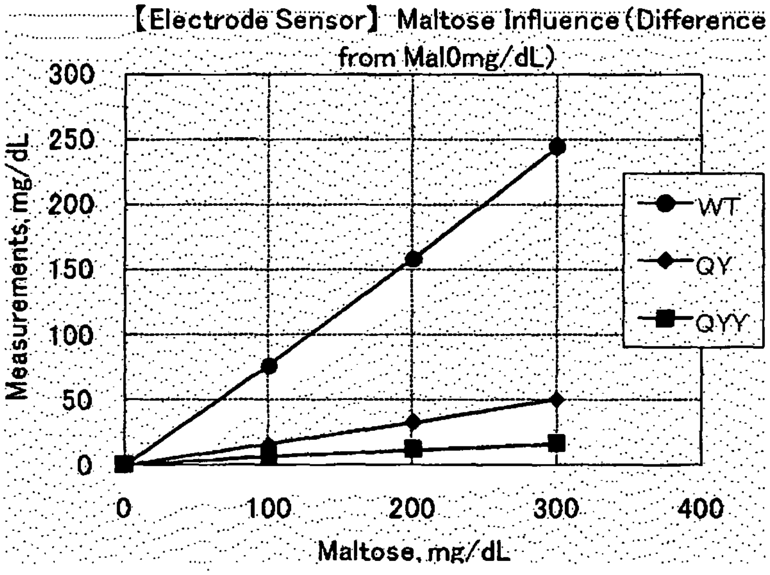
【Electrode Sensor】 Maltose Influence (Difference from Mal0mg/dL)
Measurements, mg/dL
300
250
200
150
100
50
0
0
100
200
300
400
Maltose, mg/dL
WT
QY
QYY

[Fig. 5]

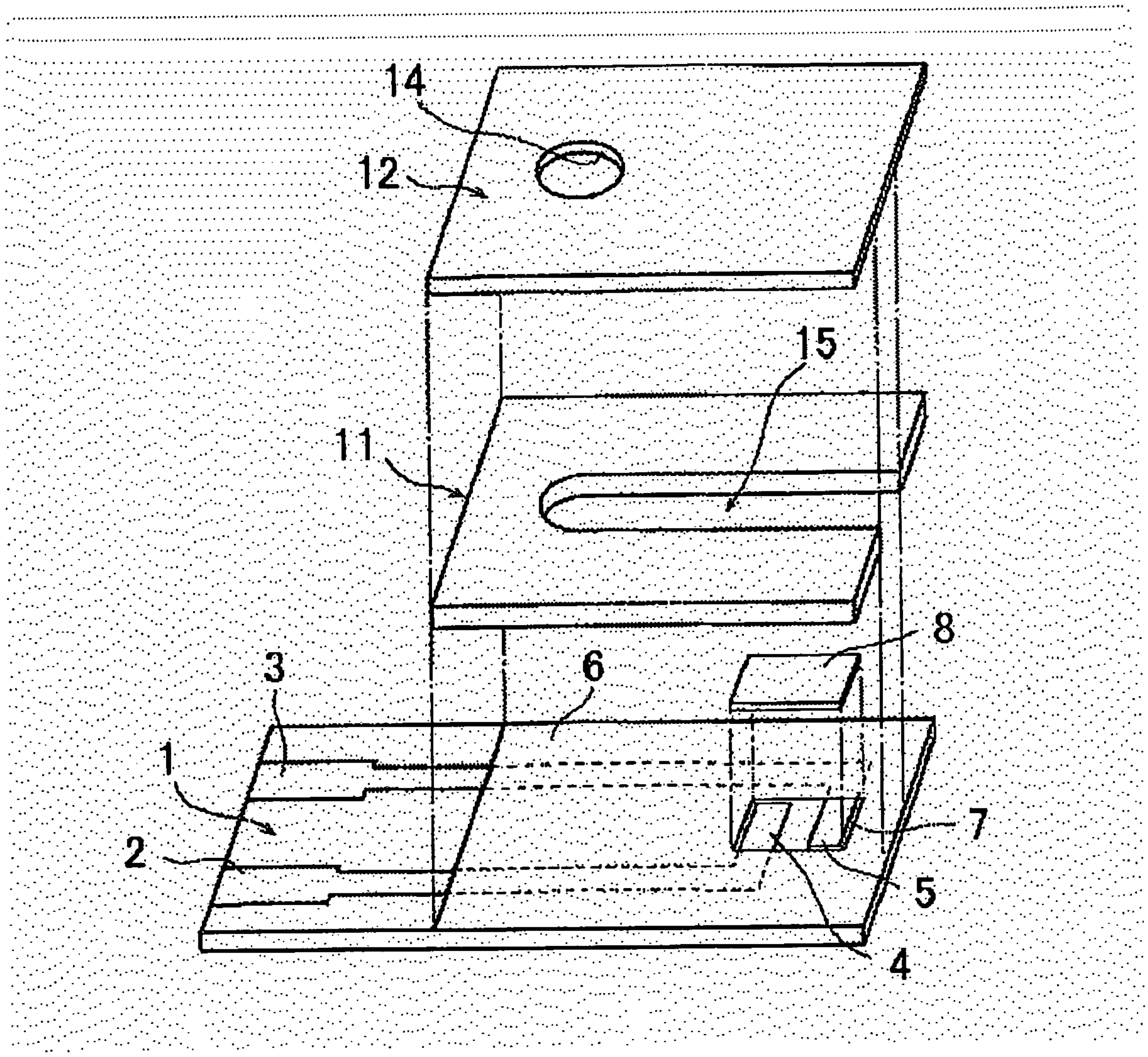

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006137283 A
- WO 2005103248 A
- US 20040023330 A
- EP 1498484 A
- US 20040023330 A1
- WO 02036779 A
- EP 1331272 A1
- US 2004023330 A
- CN 1484703 A
- JP 2010240426 A